(19) Europäisches Patentamt
European Patent Office
Office européen des brevets

(11) EP 4 635 951 A1

# (12) EUROPEAN PATENT APPLICATION
published in accordance with Art. 153(4) EPC

(43) Date of publication:
**22.10.2025 Bulletin 2025/43**

(21) Application number: **23902642.0**

(22) Date of filing: **11.12.2023**

(51) International Patent Classification (IPC):
***C07D 405/14*** (2006.01) ***A61K 31/4545*** (2006.01)
***A61P 3/10*** (2006.01) ***A61P 3/06*** (2006.01)
***A61P 3/04*** (2006.01)

(52) Cooperative Patent Classification (CPC):
**A61K 31/4545; A61P 1/16; A61P 3/04; A61P 3/06; A61P 3/10; A61P 5/50; A61P 13/12; A61P 25/00; A61P 27/02; C07D 405/14**

(86) International application number:
**PCT/CN2023/137851**

(87) International publication number:
**WO 2024/125446 (20.06.2024 Gazette 2024/25)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **13.12.2022 CN 202211600156**

(71) Applicant: **Hangzhou Zhongmeihuadong Pharmaceutical Co., Ltd.**
**GongShu District**
**HangZhou, Zhejiang 310011 (CN)**

(72) Inventors:
- **ZHAI, Wenqiang**
  **Hangzhou, Zhejiang 310011 (CN)**
- **TAN, Fang**
  **Hangzhou, Zhejiang 310011 (CN)**
- **HAN, Min**
  **Hangzhou, Zhejiang 310011 (CN)**
- **PAN, Hao**
  **Hangzhou, Zhejiang 310011 (CN)**
- **LIU, Dongzhou**
  **Hangzhou, Zhejiang 310011 (CN)**

(74) Representative: **Nederlandsch Octrooibureau**
**P.O. Box 29720**
**2502 LS The Hague (NL)**

## (54) SALT-TYPE GLP-1 RECEPTOR AGONIST, CRYSTAL FORM THEREOF, PREPARATION METHOD THEREFOR, PHARMACEUTICAL COMPOSITION THEREOF, AND USE THEREOF

(57) Disclosed are a salt-type GLP-1 receptor agonist, a crystal form thereof, a preparation method therefor, a pharmaceutical composition thereof, and the use thereof. Specifically, the GLP-1 receptor agonist is (S)-2-((4-(6-((2-fluoro-4-(oxetane-3-yl)benzyl)oxy)pyridin-2-yl)piperidin-1-yl)methyl)-1-(oxetane-2-ylmethyl)-$^1$H-benzo[d]imidazole-6-carboxylic acid.



Processed by Luminess, 75001 PARIS (FR)

## Description

### Technical Field

This application relates to salts of a GLP-1 receptor agonist, crystal forms thereof and preparation methods therefor, and also relates to pharmaceutical compositions containing the salts and crystal forms, as well as the use of the salts, crystal forms and pharmaceutical compositions for the treatment and/or prevention of GLP-1 receptor mediated diseases or related disorders.

### Background art

Diabetes is a chronic comprehensive disease caused by absolute or relative deficiency of insulin, or reduced sensitivity of target cells to insulin, which is mainly characterized by glucose metabolic disorder. It is divided into type I diabetes and type II diabetes. Among them, type II diabetes is an adult-onset diabetes, which is an endocrine disease caused by insulin resistance and/or defects in insulin secretion, and mainly manifested by chronic elevation of blood glucose. Type II diabetes patients account for more than 90% of diabetes patients.

Insulin and GLP-1 receptor agonists are among the most effective drugs for the treatment of type II diabetes. Insulin preparations remain the most widely used drugs for diabetes globally, and about 30-40% of type II diabetes patients ultimately need to use insulin. GLP-1 preparations mainly include exenatide, liraglutide, semaglutide, etc. However, insulin preparations and GLP-1 preparations are substantially polypeptide drugs and injection preparations at present. Even for oral semaglutide, there are still many restrictions in medication. Therefore, it is still necessary to further develop small molecule GLP-1 receptor agonist drugs.

Other disorders related to type II diabetes include diabetic nephropathy, diabetic ocular complications (diabetic retinopathy, uveitis related to diabetes, diabetic cataract), diabetic foot, diabetic cardiovascular complications, diabetic cerebrovascular disease, diabetic neuropathy, obesity, and hypertension.

As highly potential drugs, most of GLP-1 receptor agonists currently on the market are administered in the form of injection. Developing oral small molecule GLP-1 receptor agonists can improve patient compliance and is a development trend of GLP-1 receptor agonists.

### Summary of the Invention

(S)-2-((4-(6-((2-fluoro-4-(oxetan-3-yl)benzyl)oxy)pyridin-2-yl)piperidin-1-yl)methyl)-1-(oxetan-2-ylmethyl)-1H-benzo[d]imidazole-6-carboxylic acid ($C_{33}H_{35}FN_4O_5$), also referred to as Compound I in the specification and claims of the present application, is a small molecule GLP-1 receptor agonist and has the following structure:

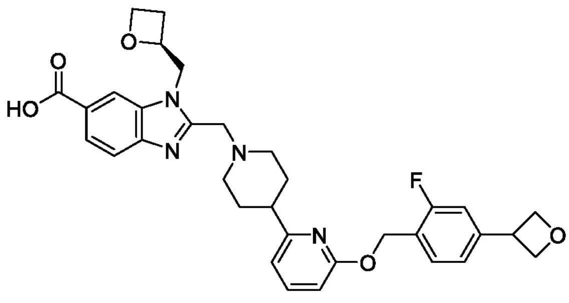
(I).

In one aspect, the present invention provides pharmaceutically acceptable salts of Compound I, wherein the salts include a mesylate salt, a meglumine salt, and a tromethamine salt. The tromethamine salt is preferably (S)-2-((4-(6-((2-fluoro-4-(oxetan-3-yl)benzyl)oxy)pyridin-2-yl)piperidin-1-yl)methyl)-1-(oxetan-2-ylmethyl)-1H-benzo[d]imidazole-6-carboxylic acid • tromethamine salt ($C_{33}H_{35}FN_4O_5$ • $C_4H_{11}NO_3$), also referred to as Compound Z in the specification and claims of the present application, with the following structure:

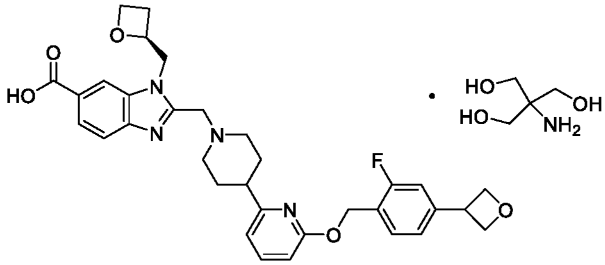

(Z).

In another aspect, the present invention provides the pharmaceutically acceptable salt of Compound I of the present invention which is in a crystalline form. In some preferred embodiments, the salt is a mesylate salt in a crystalline form, or a meglumine salt in a crystalline form (e.g., Crystal Form A), or Compound Z in a crystalline form.

In some embodiments, the present invention provides the crystalline forms of Compound Z, including Crystal Form A, Crystal Form B, Crystal Form C, Crystal Form D, Crystal Form E, Crystal Form F, Crystal Form G, Crystal Form H, Crystal Form Ix, Crystal Form J, Crystal Form K, Crystal Form L, Crystal Form M, Crystal Form N, Crystal Form O, Crystal Form P, Crystal Form Q, Crystal Form R, Crystal Form S, Crystal Form T, Crystal Form U, Crystal Form V, and Crystal Form W, with Crystal Form A, Crystal Form P, Crystal Form C, Crystal Form V, and Crystal Form W being preferred and Crystal Form A and Crystal Form V being more preferred, as described below.

In another aspect, the present invention provides methods for preparing the pharmaceutically acceptable salts of Compound I of the present invention, particularly Compound Z of the present invention, wherein the Compound Z is preferably in a crystalline form, more preferably Crystal Form A, Crystal Form P, Crystal Form C, Crystal Form V, and Crystal Form W, and even more preferably Crystal Form A and Crystal Form V.

In another aspect, the present invention provides a pharmaceutical composition comprising the pharmaceutically acceptable salt of Compound I of the present invention, particularly the tromethamine salt of the present invention, preferably a crystalline form of, more preferably Crystal Form A, Crystal Form P, Crystal Form C, Crystal Form V, or Crystal Form W, and even more preferably Crystal Form A and Crystal Form V of Compound Z.

In another aspect, the present invention provides the pharmaceutically acceptable salt of Compound I of the present invention, particularly the tromethamine salt of the present invention, preferably a crystalline form of, more preferably Crystal Form A, Crystal Form P, Crystal Form C, Crystal Form V, or Crystal Form W, and even more preferably Crystal Form A and Crystal Form V of Compound Z, or the pharmaceutical composition of the present invention, for use in the treatment and/or prevention of GLP-1 receptor mediated diseases and related disorders.

In another aspect, the present invention provides a use of the pharmaceutically acceptable salt of Compound I of the present invention, particularly the tromethamine salt of the present invention, preferably a crystalline form of, more preferably Crystal Form A, Crystal Form P, Crystal Form C, Crystal Form V, or Crystal Form W, and even more preferably Crystal Form A and Crystal Form V of Compound Z, or the pharmaceutical composition of the present invention in the manufacture of a medicament for the treatment and/or prevention of GLP-1 receptor mediated diseases or related disorders.

In another aspect, the present invention provides a method for treating and/or preventing GLP-1 receptor mediated diseases or related disorders, which comprises administering to an individual in need thereof an effective amount of the pharmaceutically acceptable salt of Compound I of the present invention, particularly the tromethamine salt of the present invention, preferably a crystalline form of, more preferably Crystal Form A, Crystal Form P, Crystal Form C, Crystal Form V, or Crystal Form W, and even more preferably Crystal Form A and Crystal Form V of Compound Z, or the pharmaceutical composition of the present invention.

In some embodiments, the GLP-1 receptor mediated diseases or related disorders are selected from diabetes, hyperglycemia, insulin resistance, impaired glucose tolerance, diabetic nephropathy, diabetic neuropathy, diabetic retinopathy, adipocyte dysfunction, obesity, non-alcoholic fatty liver disease, dyslipidemia, and hyperinsulinemia. In some embodiments, the diabetes is selected from type I diabetes (T1D) and/or type II diabetes (T2DM), idiopathic T1D, early-onset T2DM, latent autoimmune diabetes, atypical diabetes in adolescents, and gestational diabetes.

## Description of Drawings

Figure 1 shows the XRPD pattern of Crystal Form A of Compound Z; Figure 2 shows the DSC pattern of the Crystal Form A; Figure 3 shows the TGA pattern of the Crystal Form A; Figure 4-1 shows the $^1H$ NMR spectrum of the Crystal Form A; Figure 4-2 shows the DVS pattern of the Crystal Form A; and Figure 4-3 shows the DVS pattern of Compound I in an amorphous form.

Figure 5 shows the XRPD pattern of Crystal Form P of Compound Z; Figure 6 shows the DSC pattern of the Crystal Form P; Figure 7-1 shows the TGA pattern of the Crystal Form P; and Figure 7-2 shows the $^1$H NMR spectrum of the Crystal Form P.

Figure 8 shows the XRPD pattern of Crystal Form C of Compound Z; Figure 9 shows the DSC pattern of the Crystal Form C; Figure 10-1 shows the TGA pattern of the Crystal Form C; and Figure 10-2 shows the $^1$H NMR spectrum of the Crystal Form C.

Figure 11 shows the XRPD pattern of Crystal Form F of Compound Z; Figure 12 shows the DSC pattern of the Crystal Form F; Figure 13-1 shows the TGA pattern of the Crystal Form F; and Figure 13-2 shows the $^1$H NMR spectrum of the Crystal Form F.

Figure 14 shows the XRPD pattern of Crystal Form G of Compound Z; Figure 15 shows the DSC pattern of the Crystal Form G; Figure 16-1 shows the TGA pattern of the Crystal Form G; Figure 16-2 shows the $^1$H NMR spectrum of the Crystal Form G; and the XRPD pattern in Figure 16-3 shows that during the DSC test, when heated to 110°C for 5 minutes, the Crystal Form G transforms into a mixture of the Crystal Form G and Crystal Form A of Compound Z.

Figure 17 shows the XRPD pattern of Crystal Form H of Compound Z; Figure 18 shows the DSC pattern of the Crystal Form H; Figure 19-1 shows the TGA pattern of the Crystal Form H; and Figure 19-2 shows the $^1$H NMR spectrum of the Crystal Form H.

Figure 20 shows the XRPD pattern of Crystal Form Ix of Compound Z; Figure 21 shows the DSC pattern of the Crystal Form Ix; Figure 22-1 shows the TGA pattern of the Crystal Form Ix; and Figure 22-2 shows the $^1$H NMR spectrum of the Crystal Form Ix.

Figure 23 shows the XRPD pattern of Crystal Form N of Compound Z; Figure 24 shows the DSC pattern of the Crystal Form N; Figure 25-1 shows the TGA pattern of the Crystal Form N; and Figure 25-2 shows the $^1$H NMR spectrum of the Crystal Form N.

Figure 26 shows the XRPD pattern of Crystal Form O of Compound Z; Figure 27 shows the DSC pattern of the Crystal Form O; Figure 28-1 shows the TGA pattern of the Crystal Form O; and Figure 28-2 shows the $^1$H NMR spectrum of the Crystal Form O.

Figure 29 shows the XRPD pattern of Crystal Form B of Compound Z.

Figure 30 shows the XRPD pattern of Crystal Form D of Compound Z.

Figure 31 shows the XRPD pattern of Crystal Form E of Compound Z.

Figure 32 shows the XRPD pattern of Crystal Form J of Compound Z.

Figure 33 shows the XRPD pattern of Crystal Form K of Compound Z.

Figure 34 shows the XRPD pattern of Crystal Form L of Compound Z.

Figure 35 shows the XRPD pattern of Crystal Form M of Compound Z.

Figure 36 shows the XRPD pattern of Crystal Form Q of Compound Z.

Figure 37 shows the XRPD pattern of Crystal Form R of Compound Z.

Figure 38-1 shows the XRPD pattern of Crystal Form S of Compound Z; Figure 38-2 shows the DSC pattern of Crystal Form S of Compound Z; and Figure 38-3 shows the TGA pattern of Crystal Form S of Compound Z.

Figure 39 shows the XRPD pattern of Crystal Form T of Compound Z.

Figure 40 shows the XRPD pattern of Crystal Form U of Compound Z.

Figure 41 shows the XRPD pattern of Compound I in an amorphous form.

Figure 42 shows the XRPD pattern of Crystal Form A of the meglumine salt of Compound I.

Figure 43 shows the polymorphs of the mesylate salt of Compound I, wherein curve A is the XRPD pattern of Crystal Form A of the mesylate salt prepared in Example 4; curve B is the XRPD pattern of mesylate salt in an amorphous form prepared in Test 1 of Example 5; curve C is the XRPD pattern of mesylate salt in an amorphous form prepared in Test 2 of Example 5; and curve D is the XRPD pattern of mesylate salt in an amorphous form prepared in Test 3 of Example 5.

Figure 44 shows the XRPD pattern of Crystal Form A of the hydrochloride salt of Compound I.

Figure 45-1 shows the XRPD pattern of Crystal Form V of Compound Z; Figure 45-2 shows the DSC pattern of the Crystal Form V; Figure 45-3 shows the TGA pattern of the Crystal Form V; and Figure 45-4 shows the $^1$H NMR spectrum of the Crystal Form V.

Figure 46 shows the XRPD pattern of Crystal Form W of Compound Z; and Figure 47 shows the TGA pattern of the Crystal Form W.

The XRPD pattern in Figure 48 shows that in Example 8, a solid-state mixture of Crystal Form A and Crystal Form P of Compound Z in an EtOH system (at room temperature and 50°C) yields Crystal Form A and Crystal Form B of Compound Z.

The XRPD pattern in Figure 49 shows that in Example 8, a solid-state mixture of Crystal Form A and Crystal Form P of Compound Z in an EtOAc system (at room temperature and 50°C) yields Crystal Form A of Compound Z.

Figure 50 shows a comparison of the XRPD patterns of the solid before and after testing Compound I (in the free state) in a crystalline form in the long-term (25°C/60%RH) and accelerated (40°C/75%RH) solid-state stability tests in Example 12.

Figure 51 shows a comparison of the XRPD patterns of the solid before and after testing Crystal Form A of Compound Z in the long-term (25°C/60%RH) and accelerated (40°C/75%RH) solid-state stability tests in Example 12.

## Detailed Description of Invention

The present invention is further explained and illustrated below. It should be understood that the terms are for descriptive purpose, instead of imposing any limitation on the present invention.

### Definitions

Unless otherwise specified, all technical and scientific terms used herein have the same meaning as commonly understood by those skilled in the art to which the present invention pertains. In case of any discrepancy, the definitions herein shall prevail. When an amount, concentration, or other values or parameters are expressed in the form of a range, a preferred range, or a preferred upper limit numerical value and a preferred lower limit numerical value, it should be understood to be equivalent to specifically disclosing any ranges obtained by combining any pair of an upper limit numerical value or a preferred numerical value of a range with any lower limit numerical value or a preferred numerical value of the range. Unless otherwise specified, a numerical range listed herein is intended to include the endpoints of the range and all integers and fractions (decimals) within the range.

The term "about", when used in conjunction with a numerical variable, generally means that the numerical value of the variable and all numerical values of the variable are within the experimental error (for example, within a 95% confidence interval for the mean value) or within ±20%, ±10%, ±5%, or ±2% of the specified numerical value.

As used herein, the term "about", when describing the diffraction angles of XRPD, means being within the acceptable standard error of the value as considered by a person of ordinary skill in the art, such as ±0.05, ±0.10, ±0.20, ±0.30, ±1, ±2, or ±3, etc.

The term "comprise" or similar synonymous expressions such as "include", "contain", and "have" are open-ended and do not exclude additional unrecited elements, steps, or components. The expression "consist of" excludes any unspecified element, step, or component. The term "substantially consist of" means that the scope is limited to the specified elements, steps, or components, with the addition of optionally existing elements, steps, or components that will not essentially affect the basic and novel characteristics of the claimed subject matter. It should be understood that the

terms "comprise", "include", and similar terms encompass the terms "substantially consist of" and "consist of".

As used herein, the term "optional" or "optionally" means that the subsequently described event or situation may or may not occur, and that the description includes instances where the event or situation occurs, and instances where the event or situation does not occur.

Unless otherwise specified, all percentages, parts and the like herein are by weight.

As used herein, the term "crystal form" or "crystal" refers to any solid substance that presents three-dimensional ordering, and as opposed to an amorphous solid substance, produces a characteristic XRPD pattern with well-defined peaks.

As used herein, the term "X-ray powder diffraction pattern" or "XRPD pattern" refers to an experimentally observed diffraction pattern, or parameters, data, or values derived therefrom. An XRPD pattern is usually characterized by peak positions (abscissa) and/or peak intensities (ordinate).

As used herein, the term "diffraction angle" or "2θ" refers to the peak position expressed in degrees (°) based on X-ray diffraction experiment setting, and is usually an abscissa unit in a diffraction pattern. If the reflection is diffracted when the incident beam forms an angle θ with a certain lattice plane, it is necessary to record the reflected beam at an angle of 2θ in the experimental setting. It should be understood that the specific 2θ values mentioned herein for a specific crystal form are intended to denote the 2θ values (expressed in degrees) measured using the X-ray diffraction experimental conditions described herein. For example, as described herein, Cu-Kα (Kα1 (Å): 1.5406) monochromatic radiation is used. The XRPD patterns herein are preferably collected on a Bruker D8 Advance (Bruker, GER) X-ray powder diffraction analyzer.

As used herein, the term "substantially the same" or "substantially as shown in Figure x" for X-ray diffraction peaks means that the variations in representative peak positions and intensities are taken into account. For example, those skilled in the art will understand that the peak positions (2θ) will show some variations, usually up to 0.1 to 0.2 degrees, and the instruments used to measure diffraction will also cause some variations. In addition, those skilled in the art will understand that the relative peak intensities will vary due to inter-instrument differences, as well as the degree of crystallinity, preferred orientation, the surface of the prepared sample, and other factors known to those skilled in the art.

Similarly, as used herein, the expressions "substantially as shown in Figure x" for DSC patterns and TGA patterns are also intended to encompass the variations related to these analytical techniques known to those skilled in the art. For example, there is usually a variation of up to ±0.20 °C for well-defined peaks, and even larger (for example, up to ±1 °C) for broad peaks in a DSC pattern.

The nuclear magnetic resonance spectra in this application are preferably collected on a Bruker AVANCE-III or Bruker AVANCE NEO (Bruker, GER) nuclear magnetic resonance spectrometer. Unless otherwise specified, MeOD-d4 is used as the solvent.

As used herein, the term "solvent" or "good solvent" means a solvent in which Compound I can dissolve, or has a relatively higher solubility. As used herein, the term "anti-solvent" means a solvent in which Compound I is insoluble or substantially insoluble, or has a relatively lower solubility. In the present application, the terms "solvent" or "good solvent" and "anti-solvent" can also be relative and do not represent the absolute solubility of Compound I therein. The same solvent can act as a good solvent in some cases and as an anti-solvent in other cases.

As used herein, the numerical ranges (such as "1 to 10") and their sub-ranges (such as "2 to 10", "2 to 6", "3 to 10") contemplate any number within the numerical range (for example, 1, 2, 3, 4, 5, 6, 7, 8, 9, or 10).

As used herein, the term "room temperature" refers to 20 °C ± 5 °C.

## I. Pharmaceutically Acceptable Salts of Compound I

In one aspect, the present invention provides a pharmaceutically acceptable salt of Compound I,

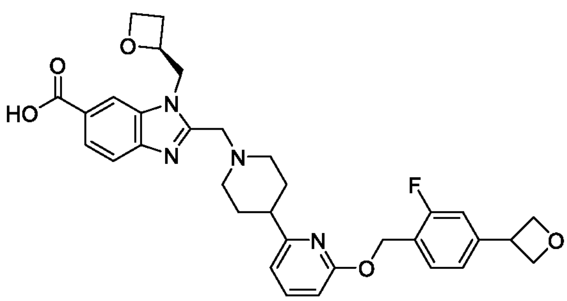

(I),

wherein the salt includes a mesylate salt, a meglumine salt, and a tromethamine salt.

In a preferred embodiment, the pharmaceutically acceptable salt is a tromethamine salt, preferably Compound Z:

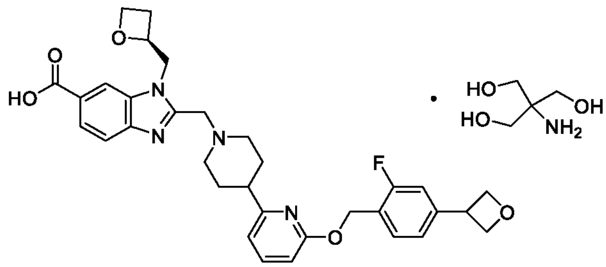

(Z).

In another aspect, the present invention provides a method for preparing the pharmaceutically acceptable salt of Compound I of the present invention, wherein the method comprises reacting the Compound I with a reagent selected from methanesulfonic acid, meglumine and tromethamine in a solvent to obtain the corresponding mesylate salt, meglumine salt, or tromethamine salt of Compound I.

In some embodiments, the present invention provides a method for preparing Compound Z of the present invention, wherein the method comprises reacting the Compound I with tromethamine in a solvent (preferably at room temperature).

In some embodiments, the solvent is one or a mixture of two or more selected from isopropyl alcohol, methyl isobutyl ketone, acetonitrile, methyl tert-butyl ether, tetrahydrofuran, ethanol, methanol, and water. A preferred example of the solvent is isopropyl alcohol.

In some embodiments, the molar ratio of the Compound I to the tromethamine is about 2:1 to about 1:2.

In the salt formation screening tests on Compound I, the present inventors prepared four salts of Compound I: a hydrochloride salt, a mesylate salt, meglumine salt, and a tromethamine salt. The inventors found that the purity of the hydrochloride salt of Compound I decreased significantly as compared to Compound I in the free state, while the mesylate salt, meglumine salt, and tromethamine salt of Compound I did not exhibit any significant decrease in purity. The inventors repeated the preparation of the mesylate salt, meglumine salt, and tromethamine salt of Compound I at a scale of 200 mg and found that: for the mesylate salt, a crystalline state was not obtained in multiple solvents and the salt formation was poor (it was prone to form a gel or an oily substance); for the meglumine salt, the salt formation was poor (it was prone to form a gel), and dissociation occurred during the drying process; and for the tromethamine salt, the salt formation was good and Crystal Form A of the tromethamine salt was obtained. The tromethamine salt of Compound I, preferably Compound Z, has better preparability than the mesylate salt and the meglumine salt. By providing the pharmaceutically acceptable salt of Compound I, preferably the tromethamine salt, the present invention improves the physical and chemical properties of Compound I, such as providing increased water solubility, improved chemical stability and/or reduced hygroscopicity, thereby increasing the bioavailability of Compound I.

## II. Crystalline Forms of the Pharmaceutically Acceptable Salts of Compound I

In another aspect, in order to optimize the drug absorption rate and the variation of the absorption rate among individuals, the present invention further provides crystalline forms of the pharmaceutically acceptable salts of Compound I, so as to improve the solubility and bioavailability of the salts and the crystalline forms thereof.

Therefore, the present invention provides the pharmaceutically acceptable salt of Compound I of the present invention which is in a crystalline form.

In some embodiments, the pharmaceutically acceptable salt of Compound I is a crystalline form of the mesylate salt, such as Crystal Form A of the mesylate salt. In some embodiments, the pharmaceutically acceptable salt of Compound I is a crystalline form of the meglumine salt, such as Crystal Form A of the meglumine salt.

In some preferred embodiments, the pharmaceutically acceptable salt of Compound I is a crystalline form of Compound Z.

Meanwhile, the inventors have noticed that there may be mixed crystals of Compound Z, indicating that polymorphs may exist. Therefore, the polymorphs of Compound Z were further studied to determine the solid form suitable for drug development.

## III. Crystalline Forms of Compound Z

Therefore, in another aspect, the present invention provides the crystalline forms of Compound Z, including Crystal Form A, Crystal Form B, Crystal Form C, Crystal Form D, Crystal Form E, Crystal Form F, Crystal Form G, Crystal Form H, Crystal Form Ix, Crystal Form J, Crystal Form K, Crystal Form L, Crystal Form M, Crystal Form N, Crystal Form O, Crystal Form P, Crystal Form Q, Crystal Form R, Crystal Form S, Crystal Form T, Crystal Form U, Crystal Form V, and Crystal Form W, as described below. The preferred crystalline forms are Crystal Form A, Crystal Form P, Crystal Form C,

Crystal Form V, and Crystal Form W. The more preferred crystalline forms are Crystal Form A and Crystal Form V.

The present invention also provides methods for preparing the crystal forms, including but not limited to slow evaporation, suspending with stirring at a low-temperature (for example, 4-8 °C), suspending with stirring at room-temperature, suspending with stirring at a high-temperature (for example, 50 °C), addition of an anti-solvent, addition of an anti-anti-solvent, cooling crystallization, gas-liquid diffusion, gas-solid diffusion, water vapor stress, polymer induction, grinding, rotary evaporation, and cyclic temperature rising and falling.

i. Crystal Form A

The present invention provides Crystal Form A of Compound Z, characterized in that the X-ray powder diffraction (XRPD) pattern of the Crystal Form A includes diffraction peaks at the following diffraction angles (2θ): about 3.62±0.20°, 7.28±0.20°, 17.21±0.20°, and 20.60±0.20°.

Alternatively or additionally, the Crystal Form A has any one, two, or all of the following characteristics:

(1) the differential scanning calorimetry (DSC) pattern of the Crystal Form A has one endothermic peak with a peak value at about 159.9°C±3.0°C;

(2) the Crystal Form A has a weight loss of about 0.815% during the process of heating to about 120°C±3°C, as measured by thermogravimetric analysis (TGA); and

(3) the $^{1}$H NMR spectrum of the Crystal Form A is substantially as shown in Figure 4-1.

In some preferred embodiments, the XRPD pattern of the Crystal Form A further includes diffraction peaks at any one, more, or all of the following diffraction angles (2θ): about 14.66±0.20°, 15.55±0.20°, 16.66±0.20°, 21.94±0.20°, and 26.04±0.20°. In some preferred embodiments, the XRPD pattern of the Crystal Form A further includes diffraction peaks at any one, more, or all of the following diffraction angles (2θ): about 7.74±0.20°, 8.54±0.20°, 10.92±0.20°, 15.28±0.20°, 17.73±0.20°, 18.05±0.20°, 18.44±0.20°, 19.47±0.20°, 19.90±0.20°, 22.38±0.20°, 23.18±0.20°, 25.04±0.20°, 27.28 ±0.20°, 27.82±0.20°, 28.10±0.20°, 29.52±0.20°, 31.06±0.20°, 32.97±0.20°, 33.36±0.20°, and 40.57±0.20°.

In some preferred embodiments, the X-ray powder diffraction pattern of the Crystal Form A includes diffraction peaks at the following diffraction angles (2θ): 3.564°, 7.245°, 7.696°, 8.531°, 14.612°, 15.253°, 15.526°, 16.637°, 17.163°, 17.675°, 17.998°, 18.409°, 19.425°, 20.570°, 21.933°, 22.384°, 23.124°, 25.013°, 26.026°, 29.555°, and 33.388°.

In some preferred embodiments, the DSC pattern of the Crystal Form A is substantially as shown in Figure 2.

In some preferred embodiments, the TGA pattern of the Crystal Form A is substantially as shown in Figure 3.

In some more preferred embodiments, the XRPD pattern of the Crystal Form A includes diffraction peaks at the diffraction angles (2θ) that are substantially the same as those shown in Figure 1, and further more preferably, the XRPD pattern of the Crystal Form A is as shown in Figure 1.

The TGA weight loss of the Crystal Form A is small, and the $^{1}$H NMR spectrum in Figure 4-1 shows no obvious solvent residue. In some embodiments, the Crystal Form A is not a solvate. More preferably, the Crystal Form A is an anhydride.

The Crystal Form A of the tromethamine salt is an anhydride that is thermodynamically more stable at room temperature and 50°C, and is stable at a water activity aw≤0.388.

Meanwhile, as compared to Compound I in the free state, Crystal Form A has increased water solubility, improved stability (including solid-state stability, high-temperature stability, high-humidity stability, and pressure stability), and lower hygroscopicity. The Crystal Form A was not subject to any crystal form change in the determination of solid-state stability, high-temperature stability, high-humidity stability, pressure stability, and hygroscopicity.

In another aspect, the present invention also provides a method for preparing the Crystal Form A of Compound Z, comprising stirring the Compound I and tromethamine in a solvent at room temperature to obtain the Crystal Form A as a solid precipitate.

In some embodiments, the solvent is one or a mixture of two or more selected from isopropyl alcohol, methyl isobutyl ketone, acetonitrile, methyl tert-butyl ether, tetrahydrofuran, ethanol, methanol, and water. A preferred example of the solvent is isopropyl alcohol. In some embodiments, the molar ratio of the Compound I to the tromethamine is about 2:1 to about 1:2. In some embodiments, the stirring at room temperature can be carried out for an appropriate period of time, for example, until the salt formation is complete, or for example, for about 3 days.

ii. Crystal Form P

The present invention also provides Crystal Form P of Compound Z, characterized in that the XRPD pattern of the Crystal Form P includes diffraction peaks at the following diffraction angles (2θ): about 3.55±0.20°, 7.31±0.20°, 14.77±0.20°, 17.01±0.20°, and 20.44±0.20°.

Alternatively or additionally, the Crystal Form P has any one, two, or all of the following characteristics:

(1) the DSC pattern of the Crystal Form P has two endothermic peaks with peak values at about 152.5°C±3.0°C and about 158.1°C±3.0°C, respectively;

(2) the Crystal Form P has a weight loss of about 0.367% during the process of heating to about 140°C±3°C, as measured by TGA; and

(3) the $^1$H NMR spectrum of the Crystal Form P is substantially as shown in Figure 7-2.

In some preferred embodiments, the XRPD pattern of the Crystal Form P further includes diffraction peaks at any one, more, or all of the following diffraction angles (2θ): about 15.50±0.20°, 18.01±0.20°, 19.06±0.20°, 19.57±0.20°, 22.07±0.20°, and 25.23±0.20°.

In some preferred embodiments, the XRPD pattern of the Crystal Form P includes diffraction peaks at the following diffraction angles (2θ): 3.547°, 7.306°, 14.765°, 15.501°, 17.009°, 18.005°, 19.058°, 19.571°, 20.436°, 22.071°, 22.441°, and 25.229°.

In some preferred embodiments, the DSC pattern of the Crystal Form P is substantially as shown in Figure 6.

In some preferred embodiments, the TGA pattern of the Crystal Form P is substantially as shown in Figure 7-1.

In some more preferred embodiments, the XRPD pattern of the Crystal Form P includes diffraction peaks at the diffraction angles (2θ) that are substantially the same as those shown in Figure 5, and further more preferably, the XRPD pattern of the Crystal Form P is as shown in Figure 5.

The TGA weight loss of the Crystal Form P is small, and the $^1$H NMR spectrum in Figure 7-2 shows no obvious solvent residue. In some embodiments, the Crystal Form P is not a solvate. More preferably, the Crystal Form P is an anhydride.

The Crystal Form P of the tromethamine salt is an anhydride that is stable at room temperature. For example, during the process of air-drying the Crystal Form P at room temperature for 3 days, no crystal form change is observed.

In another aspect, the present invention also provides a method for preparing the Crystal Form P of Compound Z, comprising suspending the Compound Z in a mixture of $CHCl_3$:EtOAc (about 1:1, v/v), and stirring the resulting suspension at a suitable temperature to obtain the Crystal Form P as a solid precipitate. In some such embodiments, the Compound Z is the Crystal Form A of Compound Z. In some embodiments, the suitable temperature is, for example, about 1-8°C, such as about 3-8°C, about 4-8°C, or about 5-8°C, preferably about 5°C. In some embodiments, the stirring can be carried out for an appropriate period of time, for example, about 3 days.

iii. Crystal Form C

The present invention also provides Crystal Form C of Compound Z, characterized in that the XRPD pattern of the Crystal Form C includes diffraction peaks at the following diffraction angles (2θ): about 3.88±0.20°, 7.81±0.20°, 15.60 ±0.20°, 17.49±0.20°, and 19.64±0.20°.

Alternatively or additionally, the Crystal Form C has any one, two, or all of the following characteristics:

(1) the DSC pattern of the Crystal Form C has three endothermic peaks with peak values at about 81.8°C±3.0°C, about 93.6°C±3.0°C, and about 155.2°C±3.0°C, respectively;

(2) the Crystal Form C has a weight loss of about 4.560% during the process of heating to about 100°C±3°C, as measured by TGA; and

(3) the $^1$H NMR spectrum of the Crystal Form C is substantially as shown in Figure 10-2.

In some preferred embodiments, the XRPD pattern of the Crystal Form C further includes diffraction peaks at any one, more, or all of the following diffraction angles (2θ): about 14.86±0.20°, 19.99±0.20°, 20.24±0.20°, 22.37±0.20°, 24.21±0.20°, and 24.51±0.20°.

In some preferred embodiments, the XRPD pattern of the Crystal Form C further includes diffraction peaks at any one, more, or all of the following diffraction angles (2θ): about 10.44±0.20°, 13.13±0.20°, 16.09±0.20°, 19.39±0.20°, 23.57±0.20°, 26.82±0.20°, 27.18±0.20°, and 33.37±0.20°.

In some preferred embodiments, the XRPD pattern of the Crystal Form C further includes diffraction peaks at any one, more, or all of the following diffraction angles (2θ): about 6.50±0.20°, 7.42±0.20°, 9.90±0.20°, 11.74±0.20°, 13.75 ±0.20°, 18.15±0.20°, 18.49±0.20°, 20.65±0.20°, 21.02±0.20°, 21.25±0.20°, 21.58±0.20°, 21.82±0.20°, 22.86±0.20°, 23.15±0.20°, 24.97±0.20°, 25.35±0.20°, 26.05±0.20°, 26.40±0.20°, 27.41±0.20°, 28.17±0.20°, 28.79 ±0.20°, 30.19±0.20°, 30.63±0.20°, 31.614±0.20°, 31.87±0.20°, 35.09±0.20°, and 35.30±0.20°.

In some preferred embodiments, the XRPD pattern of the Crystal Form C includes diffraction peaks at the following diffraction angles (2θ): 3.877°, 6.503°, 7.415°, 7.807°, 9.896°, 10.442°, 11.742°, 13.126°, 13.75°, 14.861°, 15.603°, 16.091°, 17.494°, 18.154°, 18.487°, 19.386°, 19.635°, 19.985°, 20.242°, 20.654°, 21.021°, 21.253°, 21.583°, 21.819°, 22.365°, 22.856°, 23.153°, 23.570°, 24.214°, 24.506°, 24.970°, 25.345°, 26.048°, 26.396°, 26.824°, 27.178°, 27.413°, 28.172°, 28.793°, 30.193°, 30.627°, 31.614°, 31.868°, 33.370°, 35.086°, and 35.303°.

In some preferred embodiments, the DSC pattern of the Crystal Form C has one exothermic peak with a peak value at about 125.0±3.0°C.

In some more preferred embodiments, the DSC pattern of the Crystal Form C is substantially as shown in Figure 9.

In some preferred embodiments, the TGA pattern of the Crystal Form C is substantially as shown in Figure 10-1.

In some more preferred embodiments, the XRPD pattern of the Crystal Form C includes diffraction peaks at the diffraction angles (2θ) that are substantially the same as those shown in Figure 8, and further more preferably, the XRPD pattern of the Crystal Form C is as shown in Figure 8.

In some embodiments, the Crystal Form C is a hydrate.

The Crystal Form C of the tromethamine salt is a hydrate that is stable at room temperature. For example, during the process of leaving the Crystal Form C open at room temperature for 3 days, no crystal form change is observed. In the DSC test, when heated to 100°C for 5 minutes, the Crystal Form C transforms into a mixture of the Crystal Form C and Crystal Form A of Compound Z as described above; and when heated to 100°C and continuously heated for 10 minutes, it transforms into Crystal Form A of the tromethamine salt as described above.

In another aspect, the present invention also provides a method for preparing the Crystal Form C of Compound Z, comprising suspending the Compound Z in a mixed solvent of 1,4-dioxane and $H_2O$, and stirring the resulting suspension at room temperature to obtain the Crystal Form C as a solid precipitate. In some of such embodiments, the Compound Z is the Crystal Form A of Compound Z. In some embodiments, in the mixed solvent, the volume ratio of 1,4-dioxane:$H_2O$ is about (3-10):1, such as about (4-8):1, about (5-7):1, about (5.5-6.5):1, or about 6:1. In some embodiments, the stirring can be carried out for an appropriate period of time, for example, about 3 days.

iv. Crystal Form F

The present invention also provides Crystal Form F of Compound Z, characterized in that the XRPD pattern of the Crystal Form F includes diffraction peaks at the following diffraction angles (2θ): about 3.33±0.20°, 6.70±0.20°, 10.09 ±0.20°, and 13.48±0.20°.

Alternatively or additionally, the Crystal Form F has any one, two, or all of the following characteristics:

(1) the DSC pattern of the Crystal Form F has four endothermic peaks with peak values at about 42.5°C±3.0°C, about 107.8±3.0°C, about 118.3±3.0°C, and about 154.3°C±3.0°C, respectively;

(2) the Crystal Form F has a weight loss of about 16.719% during the process of heating to about 180°C±3°C, as measured by TGA; and

(3) the $^1$H NMR spectrum of the Crystal Form F is substantially as shown in Figure 13-2.

In some preferred embodiments, the XRPD pattern of the Crystal Form F further includes diffraction peaks at any one, more, or all of the following diffraction angles (2θ): about 16.89±0.20°, 19.99±0.20°, 20.32±0.20°, 23.75±0.20°, 30.71±0.20°, 34.23±0.20°.

In some preferred embodiments, the XRPD pattern of the Crystal Form F includes diffraction peaks at the following diffraction angles (2θ): 3.325°, 6.700°, 10.089°, 13.481°, 16.890°, 19.991°, 20.318°, 23.751°, 30.705°, and 34.228°.

In some preferred embodiments, the DSC pattern of the Crystal Form F is substantially as shown in Figure 12.

In some preferred embodiments, the TGA pattern of the Crystal Form F is substantially as shown in Figure 13-1.

In some more preferred embodiments, the XRPD pattern of the Crystal Form F includes diffraction peaks at the diffraction angles (2θ) that are substantially the same as those shown in Figure 11, and further more preferably, the XRPD pattern of the Crystal Form F is as shown in Figure 11.

In some embodiments, the Crystal Form F is a solvate, more specifically a solvate with N-methyl pyrrolidone (NMP), wherein the stoichiometric ratio of the Compound Z to the NMP is preferably about 1:1.5.

The Crystal Form F of the tromethamine salt is stable at room temperature. For example, during the process of leaving the Crystal Form F open at room temperature for 3 days, no crystal form change is observed. In the DSC test, when

heated to 120°C for 5 minutes, the Crystal Form F transforms into Crystal Form A of Compound Z as described above.

In another aspect, the present invention also provides a method for preparing the Crystal Form F of Compound Z, comprising:

(1) providing a clear solution of the Compound Z in a mixture of NMP:toluene (e.g., at about 1:1, v:v); and

(2) adding the solution to a container containing isopropyl acetate, sealing the container and leaving it at room temperature to obtain the Crystal Form F as a solid precipitate.

In some embodiments, the sealed container can be placed at room temperature for an appropriate period of time, for example, about 3 days.

v. Crystal Form G

The present invention also provides Crystal Form G of Compound Z, characterized in that the XRPD pattern of the Crystal Form G includes diffraction peaks at the following diffraction angles (2θ): about 3.18±0.20°, 6.43±0.20°, 12.94 ±0.20°, 17.99±0.20°, and 19.95±0.20°.

Alternatively or additionally, the Crystal Form G has any one, two, or all of the following characteristics:

(1) the DSC pattern of the Crystal Form G has three endothermic peaks with peak values at about 108.7°C±3.0°C, about 143.7±3.0°C, and about 156.5°C±3.0°C, respectively;

(2) the Crystal Form G has a weight loss of about 4.9911% during the process of heating to about 160°C±3°C, as measured by TGA; and

(3) the $^1$H NMR spectrum of the Crystal Form G is substantially as shown in Figure 16-2.

In some preferred embodiments, the XRPD pattern of the Crystal Form G further includes diffraction peaks at any one, more, or all of the following diffraction angles (2θ): about 9.67±0.20°, 15.73±0.20°, 16.95±0.20°, 18.47±0.20°, 18.70±0.20°, 22.43±0.20°, 24.94±0.20°, and 29.46±0.20°.

In some preferred embodiments, the XRPD pattern of the Crystal Form G further includes diffraction peaks at any one, more, or all of the following diffraction angles (2θ): about 14.05±0.20°, 14.81±0.20°, 16.69±0.20°, 19.50±0.20°, 21.79±0.20°, 22.17±0.20°, 23.06±0.20°, 23.44±0.20°, and 32.794±0.20°.

In some preferred embodiments, the XRPD pattern of the Crystal Form G includes diffraction peaks at the following diffraction angles (2θ): 3.176°, 6.427°, 9.668°, 12.936°, 14.046°, 14.807°, 15.726°, 16.692°, 16.947°, 17.982°, 18.465°, 18.701°, 19.498°, 19.953°, 21.792°, 22.170°, 22.426°, 23.062°, 23.442°, 24.937°, 29.455°, and 32.794°.

In some preferred embodiments, the DSC pattern of the Crystal Form G is substantially as shown in Figure 15.

In some preferred embodiments, the TGA pattern of the Crystal Form G is substantially as shown in Figure 16-1.

In some more preferred embodiments, the XRPD pattern of the Crystal Form G includes diffraction peaks at the diffraction angles (2θ) that are substantially the same as those shown in Figure 14, and further more preferably, the XRPD pattern of the Crystal Form G is as shown in Figure 14.

In some embodiments, the Crystal Form G is a solvate, more specifically a solvate with 1,4-dioxane, wherein the stoichiometric ratio of the Compound Z to the 1,4-dioxane is preferably about 1:0.4.

The Crystal Form G of the tromethamine salt is stable at room temperature. For example, during the process of leaving the Crystal Form G open at room temperature for 3 days, no crystal form change is observed. In the DSC test, when heated to 110°C for 5 minutes, the Crystal Form G transforms into a mixture of the Crystal Form G and Crystal Form A of Compound Z as described above, as shown in Figure 16-3.

In another aspect, the present invention also provides a method for preparing the Crystal Form G of Compound Z, comprising suspending the Compound Z in a mixture of 1,4-dioxane:EtOAc (e.g., at about 1:1, v:v), and stirring the resulting suspension at a suitable temperature to obtain the Crystal Form G as a solid precipitate. In some of such embodiments, the Compound Z is the Crystal Form A of Compound Z. In some embodiments, the suitable temperature is, for example, about 1-8°C, such as about 3-8°C, about 4-8°C, or about 5-8°C, preferably about 5°C. In some embodiments, the stirring can be carried out for an appropriate period of time, for example, about 3 days.

vi. Crystal Form H

The present invention also provides Crystal Form H of Compound Z, characterized in that the XRPD pattern of the Crystal Form H includes diffraction peaks at the following diffraction angles (2θ): about 3.25±0.20°, 6.56±0.20°, 15.97±0.20°, 18.19±0.20°, and 19.91±0.20°.

Alternatively or additionally, the Crystal Form H has any one, two, or all of the following characteristics:

(1) the DSC pattern of the Crystal Form H has two endothermic peaks with peak values at about 144.47°C±3.0°C and about 160.98°C±3.0°C, respectively;

(2) the Crystal Form H has a weight loss of about 8.668% during the process of heating to about 170°C±3°C, as measured by TGA; and

(3) the $^1$H NMR spectrum of the Crystal Form H is substantially as shown in Figure 19-2.

In some preferred embodiments, the XRPD pattern of the Crystal Form H further includes diffraction peaks at any one, more, or all of the following diffraction angles (2θ): about 16.93±0.20°, 17.26±0.20°, 19.42±0.20°, 19.60±0.20°, and 23.20±0.20°.

In some preferred embodiments, the XRPD pattern of the Crystal Form H further includes diffraction peaks at any one, more, or all of the following diffraction angles (2θ): about 11.28±0.20°, 13.21±0.20°, 14.72±0.20°, 15.25±0.20°, 15.41±0.20°, 16.38±0.20°, 17.86±0.20°, 18.78±0.20°, 19.08±0.20°, 20.24±0.20°, 20.60±0.20°, 21.00±0.20°, 21.49 ±0.20°, 22.19±0.20°, 22.595±0.20°, 24.43±0.20°, 25.83±0.20°, 26.28±0.20°, 28.56±0.20°, 29.17±0.20°, 29.73 ±0.20°, and 30.03±0.20°.

In some preferred embodiments, the XRPD pattern of the Crystal Form H includes diffraction peaks at the following diffraction angles (2θ): 3.253°, 6.563°, 11.275°, 13.207°, 14.715°, 15.248°, 15.407°, 15.974°, 16.384°, 16.928°, 17.261°, 17.859°, 18.194°, 18.776°, 19.082°, 19.422°, 19.598°, 19.911°, 20.238°, 20.601°, 21.001°, 21.491°, 22.190°, 22.595°, 23.202°, 24.432°, 25.826°, 26.282°, 28.559°, 29.165°, 29.727°, 30.030°.

In some preferred embodiments, the DSC pattern of the Crystal Form H is substantially as shown in Figure 18.

In some preferred embodiments, the TGA pattern of the Crystal Form H is substantially as shown in Figure 19-1.

In some preferred embodiments, the XRPD pattern of the Crystal Form H includes diffraction peaks at the diffraction angles (2θ) that are substantially the same as those shown in Figure 17, and further more preferably, the XRPD pattern of the Crystal Form H is as shown in Figure 17.

In some embodiments, the Crystal Form H is a solvate, more specifically a solvate with tetrafluoroethylene (TFE), wherein the stoichiometric ratio of the Compound Z to the TFE is preferably about 1:0.65.

In the DSC test, when heated to 140°C for 5 minutes, the Crystal Form H transforms into Crystal Form A of Compound Z as described above.

In another aspect, the present invention also provides a method for preparing the Crystal Form H of Compound Z, comprising:

(1) providing a clear solution of the Compound Z in TFE; and

(2) heating and evaporating the solution to obtain the Crystal Form H as a solid product.

In some embodiments, the solution is heated to a temperature of about 40-70°C, such as a temperature of about 45-65°C or about 50-60°C, preferably about 50°C. In some embodiments, the solution is rotary evaporated.

vii. Crystal Form Ix

The present invention also provides Crystal Form Ix of Compound Z, characterized in that the XRPD pattern of the Crystal Form Ix includes diffraction peaks at the following diffraction angles (2θ): about 3.25±0.20°, 6.58±0.20°, 13.21 ±0.20°, 17.54±0.20°, and 20.63±0.20°.

Alternatively or additionally, the Crystal Form Ix has any one, two, or all of the following characteristics:

(1) the DSC pattern of the Crystal Form Ix has five endothermic peaks with peak values at about 44.32°C±3.0°C, about 58.81°C±3.0°C, about 77.47°C±3.0°C, about 97.14°C±3.0°C, and about 155.71°C±3.0°C, respectively;

(2) the Crystal Form Ix has a weight loss of about 5.416% during the process of heating to about 100°C±3°C, as measured by TGA; and

(3) the $^1$H NMR spectrum of the Crystal Form Ix is substantially as shown in Figure 22-2.

In some preferred embodiments, the XRPD pattern of the Crystal Form Ix further includes diffraction peaks at any

one, more, or all of the following diffraction angles (2θ): about 15.51±0.20°, 21.24±0.20°, 23.88±0.20°, and 26.59±0.20°.

In some preferred embodiments, the XRPD pattern of the Crystal Form Ix further includes diffraction peaks at any one, more, or all of the following diffraction angles (2θ): about 8.21±0.20°, 9.41±0.20°, 14.63±0.20°, 16.18±0.20°, 16.99 ±0.20°, 18.04±0.20°, 18.39±0.20°, 18.93±0.20°, 19.36±0.20°, 22.05±0.20°, 24.97±0.20°, 25.79±0.20°, 28.58 ±0.20°, 29.46±0.20°, and 30.08±0.20°.

In some preferred embodiments, the XRPD pattern of the Crystal Form Ix includes diffraction peaks at the following diffraction angles (2θ): 3.252°, 6.582°, 8.206°, 9.407°, 13.211°, 14.629°, 15.509°, 16.183°, 16.992°, 17.535°, 18.041°, 18.388°, 18.934°, 19.364°, 20.633°, 21.236°, 22.047°, 23.883°, 24.973°, 25.792°, 26.590°, 28.576°, 29.457°, and 30.079°.

In some preferred embodiments, the DSC pattern of the Crystal Form Ix is substantially as shown in Figure 21.

In some preferred embodiments, the TGA pattern of the Crystal Form Ix is substantially as shown in Figure 22-1.

In some preferred embodiments, the XRPD pattern of the Crystal Form Ix includes diffraction peaks at the diffraction angles (2θ) that are substantially the same as those shown in Figure 20, and further more preferably, the XRPD pattern of the Crystal Form Ix is as shown in Figure 20.

In some embodiments, the Crystal Form Ix is a solvate, more specifically a solvate with anisole, wherein the stoichiometric ratio of the Compound Z to the anisole is preferably about 1:0.4.

The Crystal Form Ix of the tromethamine salt is stable at room temperature. For example, during the process of leaving the Crystal Form Ix open at room temperature for 3 days, no crystal form change is observed. In the DSC test, when heated to 100°C for 5 minutes, the Crystal Form Ix transforms into Crystal Form A of Compound Z as described above.

In another aspect, the present invention also provides a method for preparing the Crystal Form Ix of Compound Z, comprising suspending the Compound Z in anisole, and stirring the resulting suspension at a suitable temperature to obtain the Crystal Form Ix as a solid precipitate. In some of such embodiments, the Compound Z is the Crystal Form A of Compound Z. In some embodiments, the suitable temperature is, for example, a temperature of about 40-70°C, such as a temperature of about 45-65°C or about 50-60°C, preferably about 50°C. In some embodiments, the stirring can be carried out for an appropriate period of time, for example, about 3 days.

<u>viii. Crystal Form N</u>

The present invention also provides Crystal Form N of Compound Z, characterized in that the XRPD pattern of the Crystal Form N includes diffraction peaks at the following diffraction angles (2θ): about 3.41±0.20°, 6.93±0.20°, 17.20 ±0.20°, and 20.51±0.20°.

Alternatively or additionally, the Crystal Form N has any one, two, or all of the following characteristics:

(1) the DSC pattern of the Crystal Form N has two endothermic peaks with peak values at about 120.5°C±3.0°C and about 160.1°C±3.0°C, respectively;

(2) the Crystal Form N has a weight loss of about 2.292% during the process of heating to about 150°C±3°C, as measured by TGA; and

(3) the $^{1}H$ NMR spectrum of the Crystal Form N is substantially as shown in Figure 25-2.

In some preferred embodiments, the XRPD pattern of the Crystal Form N further includes diffraction peaks at any one, more, or all of the following diffraction angles (2θ): about 13.95±0.20°, 14.61±0.20°, 15.76±0.20°, 17.93±0.20°, 19.42±0.20°, 22.02±0.20°, 25.03±0.20°, and 25.78±0.20°.

In some preferred embodiments, the XRPD pattern of the Crystal Form N includes diffraction peaks at the following diffraction angles (2θ): 3.409°, 6.933°, 13.948°, 14.611°, 15.762°, 17.201°, 17.934°, 19.419°, 20.513°, 22.016°, 25.032°, and 25.776°.

In some preferred embodiments, the DSC pattern of the Crystal Form N is substantially as shown in Figure 24.

In some preferred embodiments, the TGA pattern of the Crystal Form N is substantially as shown in Figure 25-1.

In some more preferred embodiments, the XRPD pattern of the Crystal Form N includes diffraction peaks at the diffraction angles (2θ) that are substantially the same as those shown in Figure 23, and further more preferably, the XRPD pattern of the Crystal Form N is as shown in Figure 23.

In some embodiments, the Crystal Form N is a solvate, more specifically a solvate with 1,4-dioxane. In some embodiments, the solvate is an incompletely solvated crystal. In some embodiments, in the Crystal Form N, the stoichiometric ratio of the Compound Z to the 1,4-dioxane is about 1:0.16.

The Crystal Form N of the tromethamine salt is stable at room temperature. For example, during the process of leaving the Crystal Form N open at room temperature for 3 days, no crystal form change is observed. In the DSC test, when heated to 130°C for 5 minutes, the Crystal Form N transforms into Crystal Form A of Compound Z as described above.

In another aspect, the present invention also provides a method for preparing the Crystal Form N of Compound Z, comprising suspending the Compound Z in a mixture of acetonitrile (ACN):1,4-dioxane (e.g., at about 1:1, v:v), and stirring the resulting suspension to obtain the Crystal Form N as a solid precipitate. In some such embodiments, the Compound Z is the Crystal Form A of Compound Z. In some embodiments, the stirring is carried out under the condition of cyclic temperature rising and falling. In some embodiments, the cyclic temperature rising and falling includes:

(1) maintaining at a temperature $T_1$ of about 40-70°C for a time period t1 of about 90-150 minutes (min);

(2) decreasing from the temperature $T_1$ to a temperature $T_2$ of about 1-8°C at a rate of about 0.1°C/min;

(3) maintaining at the temperature $T_2$ for a time period t2 of about 90-150 min;

(4) increasing the temperature from the temperature $T_2$ to the temperature $T_1$; and

(5) repeating the sequence from (1) to (4) above once or more times;

provided that in the last repetition, only the sequence from (1) to (3) is repeated.

The temperature $T_1$ in each sequence is independent and can be the same or different. The temperature $T_2$ in each sequence is independent and can be the same or different. In some embodiments, the temperature $T_1$ is a temperature of about 45-65°C or about 50-60°C, preferably about 50°C. In some embodiments, the temperature $T_2$ is, for example, about 3-8°C, about 4-8°C, or about 5-8°C, preferably about 5°C.

The time period t1 in each sequence is independent and can be the same or different. The time period t2 in each sequence is independent and can be the same or different. In some embodiments, the time periods t1 and t2 are each about 100-140 min, about 110-130 min, or about 120 min.

In some embodiments, the stirring can be carried out for an appropriate period of time, for example, about 3 days.

In some embodiments, the stirring is carried out for about 3 days under the condition of cyclic temperature rising and falling as described below:

(1) maintaining at a temperature of about 50°C for about 120 minutes (min);

(2) decreasing from about 50°C to about 5°C at a rate of about 0.1°C/min;

(3) maintaining at about 5°C for about 120 min;

(4) increasing the temperature from about 5°C to about 50°C; and

(5) repeating the sequence from (1) to (3) above once.

<u>ix. Crystal Form O</u>

The present invention also provides Crystal Form O of Compound Z, characterized in that the XRPD pattern of the Crystal Form O includes diffraction peaks at the following diffraction angles (2θ): about 3.43±0.20°, 7.01±0.20°, 18.00 ±0.20°, and 20.34±0.20°.

Alternatively or additionally, the Crystal Form O has any one, two, or all of the following characteristics:

(1) the DSC pattern of the Crystal Form O has two endothermic peaks with peak values at about 120.32°C±3.0°C and about 156.54°C±3.0°C, respectively;

(2) the Crystal Form O has a weight loss of about 1.601% during the process of heating to about 140°C±3°C, as measured by TGA; and

(3) the $^1$H NMR spectrum of the Crystal Form O is substantially as shown in Figure 28-2.

In some preferred embodiments, the XRPD pattern of the Crystal Form O further includes diffraction peaks at any one, more, or all of the following diffraction angles (2θ): about 6.54±0.20°, 13.12±0.20°, 14.12±0.20°, 17.07±0.20°, 23.05±0.20°, and 25.91±0.20°.

In some preferred embodiments, the XRPD pattern of the Crystal Form O includes diffraction peaks at the following diffraction angles (2θ): 3.429°, 6.544°, 7.012°, 13.115°, 14.116°, 17.074°, 18.000°, 20.339°, 23.045°, and

25.905°.

In some preferred embodiments, the DSC pattern of the Crystal Form O is substantially as shown in Figure 27.

In some preferred embodiments, the TGA pattern of the Crystal Form O is substantially as shown in Figure 28-1.

In some preferred embodiments, the XRPD pattern of the Crystal Form O includes diffraction peaks at the diffraction angles (2θ) that are substantially the same as those shown in Figure 26, and further more preferably, the XRPD pattern of the Crystal Form O is as shown in Figure 26.

In some embodiments, the Crystal Form O is a solvate, more specifically a solvate with cyclopentyl methyl ether (CPME). In some embodiments, the solvate is an incompletely solvated crystal. In some embodiments, in the Crystal Form O, the stoichiometric ratio of the Compound Z to the CPME is about 1:0.11.

The Crystal Form O of the tromethamine salt is stable at room temperature. For example, during the process of leaving the Crystal Form O open at room temperature for 3 days, no crystal form change is observed. In the DSC test, when heated to 130°C for 5 minutes, the Crystal Form O transforms into Crystal Form A of Compound Z as described above.

In another aspect, the present invention also provides a method for preparing the Crystal Form O of Compound Z, comprising suspending the Compound Z in a mixture of acetone:CPME (e.g., at about 1:1, v:v), and stirring the resulting suspension at a suitable temperature to obtain the Crystal Form O as a solid precipitate. In some of such embodiments, the Compound Z is the Crystal Form A of Compound Z. In some embodiments, the suitable temperature is, for example, a temperature of about 40-70°C, such as a temperature of about 45-65°C or about 50-60°C, preferably about 50°C. In some embodiments, the stirring can be carried out for an appropriate period of time, for example, about 3 days.

x. Crystal Form S

The present invention also provides Crystal Form S of Compound Z with an XRPD pattern which includes diffraction peaks at the diffraction angles (2θ) that are substantially the same as those shown in Figure 38-1. More preferably, the XRPD pattern of the Crystal Form S is as shown in Figure 38-1.

In some embodiments, the DSC pattern of the Crystal Form S is substantially as shown in Figure 38-2.

In some embodiments, the Crystal Form S has a weight loss of about 5.345% during the process of heating to about 160°C±3°C, as measured by TGA. In some preferred embodiments, the TGA pattern of the Crystal Form S is substantially as shown in Figure 38-3.

In some embodiments, the Crystal Form S is a solvate, more specifically a solvate with TFE. In some embodiments, in the Crystal Form S, the stoichiometric ratio of the Compound Z to the TFE is about 1:0.45.

The Crystal Form S of the tromethamine salt is stable at room temperature. For example, during the process of leaving the Crystal Form S open at room temperature for 1 day, no crystal form change is observed. In the DSC test, when heated to 140°C for 10 minutes, the Crystal Form S transforms into Crystal Form A of Compound Z as described above.

In another aspect, the present invention also provides a method for preparing the Crystal Form S of Compound Z, comprising:

(1) providing a clear solution of the Compound Z in a mixture of TFE:ACN (e.g., at about 1:1, v:v); and

(2) adding an anti-solvent (e.g., methyl tert-butyl ether) to the solution with stirring to obtain the Crystal Form S as a solid precipitate.

xi. Crystal Form V

The present invention also provides Crystal Form V of Compound Z, characterized in that the XRPD pattern of the Crystal Form V includes diffraction peaks at the following diffraction angles (2θ): about 4.46±0.20°, 8.98±0.20°, 12.88 ±0.20°, 18.04±0.20°, and 19.26±0.20°.

Alternatively or additionally, the Crystal Form V has any one, two, or all of the following characteristics:

(1) the DSC pattern of the Crystal Form V has two endothermic peaks with peak values at about 64.5°C±3.0°C and about 164.8°C±3.0°C, respectively;

(2) the Crystal Form V has a weight loss of about 2.987% during the process of heating to about 90°C±3°C, as measured by TGA; and

(3) the $^{1}H$ NMR spectrum of the Crystal Form V is substantially as shown in Figure 45-4.

In some preferred embodiments, the XRPD pattern of the Crystal Form V further includes diffraction peaks at any one, more, or all of the following diffraction angles (2θ): about 16.68±0.20°, 18.39±0.20°, 20.75±0.20°, 23.84±0.20°, and

25.42±0.20°.

In some preferred embodiments, the XRPD pattern of the Crystal Form V further includes diffraction peaks at any one, more, or all of the following diffraction angles (2θ): about 9.16±0.20°, 9.39±0.20°, 10.31±0.20°, 12.70±0.20°, 12.99 ±0.20°, 14.36±0.20°, 14.70±0.20°, 19.46±0.20°, 19.62±0.20°, 21.29±0.20°, 22.03±0.20°, 22.69±0.20°, 22.93 ±0.20°, 24.23±0.20°, 24.66±0.20°, and 27.00±0.20°.

In some preferred embodiments, the XRPD pattern of the Crystal Form V further includes diffraction peaks at any one, more, or all of the following diffraction angles (2θ): about 11.89±0.20°, 13.50±0.20°, 16.17±0.20°, 17.71±0.20°, 18.88±0.20°, 19.93±0.20°, 20.20±0.20°, 23.20±0.20°, 25.98±0.20°, 27.23±0.20°, 28.02±0.20°, 28.40±0.20°, 28.68 ±0.20°, 29.01±0.20°, 29.81±0.20°, 30.25±0.20°, 31.52±0.20°, 31.87±0.20°, 32.63±0.20°, 33.74±0.20°, 36.47 ±0.20°, 37.48±0.20°, and 7.96±0.20°.

In some preferred embodiments, the DSC pattern of the Crystal Form V is substantially as shown in Figure 45-2.

In some preferred embodiments, the TGA pattern of the Crystal Form V is substantially as shown in Figure 45-3.

In some more preferred embodiments, the XRPD pattern of the Crystal Form V includes diffraction peaks at the diffraction angles (2θ) that are substantially the same as those shown in Figure 45-1, and further more preferably, the XRPD pattern of the Crystal Form V is as shown in Figure 45-1.

In some embodiments, the Crystal Form V is a hydrate.

The inventors found that in the DSC test, when heated to 100°C for 10 minutes, the Crystal Form V transforms into Crystal Form W of Compound Z described below.

In another aspect, the present invention also provides a method for preparing the Crystal Form V of Compound Z, comprising:

(1) providing a solution of the Compound Z in acetonitrile:water (e.g., at about 6:1, v:v) at an elevated temperature;

(2) transferring the solution to a lower temperature and stirring; and

(3) filtering, and drying the resulting product in a hot air stream to obtain the Crystal Form V.

In some embodiments, the temperature of the solution in step (1) is about 45-70°C, such as about 50-65°C or about 50-60°C, preferably about 55°C. In some embodiments, the lower temperature in step (2) is about 20-30°C, such as about 25°C. In some embodiments, the stirring in step (2) is carried out for an appropriate period of time, for example, about 3h. In some embodiments, the temperature of the hot air stream in step (3) is about 40-70°C, such as about 45-65°C or about 50-60°C, preferably about 50°C.

In some other embodiments, the present invention provides another method for preparing the Crystal Form V of Compound Z, comprising:

(1) providing a clear solution of the Compound Z in methanol at an elevated temperature;

(2) cooling the solution to a first lower temperature and adding a crystal seed of the Crystal Form V;

(3) cooling the solution to a second lower temperature at a first rate;

(4) further cooling the solution to a third lower temperature at a second rate; and

(5) stirring at the third lower temperature and then filtering, and oven drying the resulting product in a vacuum to obtain the Crystal Form V.

In some of such embodiments, the temperature of the solution in step (1) is about 50-75°C, such as about 55-70°C or about 55-65°C, preferably about 60°C. In some embodiments, the first lower temperature in step (2) is about 30-55°C, such as about 35-50°C or about 35-45°C, preferably about 40°C. In some embodiments, the second lower temperature in step (3) is about 10-20°C, such as about 15°C. In some embodiments, the third lower temperature in step (4) is about -15 to -25°C, for example, about -18°C. In some embodiments, the first rate in step (3) is different from, preferably smaller than the second rate in step (4). Preferably, the first rate is about 5-10°C/h, for example, about 8°C/h. Preferably, the second rate is about 10-15°C/h, for example, about 12°C/h. In some embodiments, the stirring in step (4) is carried out for an appropriate period of time, for example, about 2h. In some embodiments, the oven drying in step (5) is carried out at a temperature of about 40-50°C, for example, about 40°C for an appropriate period of time such as about 15-20h, for example, about 17h.

In some other embodiments, the present invention provides another method for preparing the Crystal Form V of Compound Z, comprising:

(1) providing a saturated solution of the Compound Z in a mixture of ethanol and water;

(2) suspending a solid mixture of the Crystal Form V, Crystal Form A, and Crystal Form C of Compound Z in the saturated solution; and

(3) stirring the resulting suspension at room temperature to obtain the Crystal Form V as a solid precipitate.

In some of such embodiments, the saturated solution in step (1) has a water activity of not less than 0.20. In some embodiments, the saturated solution in step (1) has a water activity of not more than 0.5. In some embodiments, the saturated solution in step (1) has a water activity of not less than 0.20 and not more than 0.5. In some embodiments, the stirring in step (3) is carried out for an appropriate period of time, for example, about 4 days.

xii. Crystal Form W

The present invention also provides Crystal Form W of Compound Z, characterized in that the XRPD pattern of the Crystal Form W includes diffraction peaks at the following diffraction angles (2θ): about 4.62±0.20°, 12.90±0.20°, 18.06±0.20°, 19.40±0.20°, and 19.60±0.20°.

In some preferred embodiments, the XRPD pattern of the Crystal Form W further includes diffraction peaks at any one, more, or all of the following diffraction angles (2θ): about 14.86 ±0.20°, 16.74 ±0.20°, 18.41 ±0.20°, 18.68 ±0.20°, 21.63 ±0.20°, 22.03 ±0.20°, 22.60 ±0.20°, 22.99 ±0.20°, and 25.34±0.20°.

In some preferred embodiments, the XRPD pattern of the Crystal Form W further includes diffraction peaks at any one, more, or all of the following diffraction angles (2θ): about 9.14 ±0.20°, 9.37 ±0.20°, 10.21 ±0.20°, 15.35 ±0.20°, 20.52 ±0.20°, 20.77 ±0.20°, 23.88 ±0.20°, 24.27 ±0.20°, 24.66 ±0.20°, 27.04 ±0.20°, and 28.01±0.20°.

In some preferred embodiments, the XRPD pattern of the Crystal Form W further includes diffraction peaks at any one, more, or all of the following diffraction angles (2θ): about 11.50 ±0.20°, 12.02 ±0.20°, 13.93 ±0.20°, 14.40 ±0.20°, 23.44 ±0.20°, 25.81 ±0.20°, 28.46 ±0.20°, 30.33 ±0.20°, 31.60 ±0.20°, and 37.34±0.20°.

In some more preferred embodiments, the XRPD pattern of the Crystal Form W includes diffraction peaks at the diffraction angles (2θ) that are substantially the same as those shown in Figure 46, and further more preferably, the XRPD pattern of the Crystal Form W is as shown in Figure 46.

In some embodiments, the Crystal Form W is not a solvate, and more preferably is an anhydride.

The Crystal Form W has a weight loss of about 1.111% during the process of heating to about 90°C±3°C, as measured by TGA. In some embodiments, the TGA pattern of the Crystal Form W is substantially as shown in Figure 47.

The inventors found that the Crystal Form W transforms into the Crystal Form V after being placed at room temperature for 45 minutes.

In another aspect, the present invention also provides a method for preparing the Crystal Form W of Compound Z, comprising heating the Crystal Form V of Compound Z at about 100°C for an appropriate period of time (e.g., about 10 minutes) under the protection of an inert gas (e.g., nitrogen) to obtain the Crystal Form W.

In another aspect, the present invention provides another method for preparing the Crystal Form W of Compound Z, comprising:

(1) providing a saturated solution of Compound Z in a mixture of ethanol and water;

(2) suspending a solid mixture of the Crystal Form V, Crystal Form A, and Crystal Form C of Compound Z in the saturated solution; and

(3) stirring the resulting suspension at room temperature to obtain the Crystal Form W as a solid precipitate.

In some of such embodiments, the saturated solution in step (1) has a water activity of not more than 0.12. In some embodiments, the stirring in step (3) is carried out for an appropriate period of time, for example, about 1 day.

xiii. Other Crystal Forms

The present invention also provides metastable crystal forms of Compound Z, including Crystal Forms B, D, E, J, K, L, M, Q, R, T, and U. These crystal forms, when placed open at room temperature for, for example, 10min, 20min, 30min, 1 hour, 5 hours, 10 hours, 15 hours, 20 hours, 1 day, 2 days, 3 days, or 4 days, transform into a mixed crystal of each with Crystal Form A, or another single crystal form, such as Crystal Form A, Crystal Form F, or Crystal Form U, preferably Crystal Form A.

The XRPD pattern of the Crystal Form B is substantially as shown in Figure 29. The Crystal Form B transforms

into a mixed crystal of the Crystal Forms A and B when air-dried at room temperature for 10 minutes, and transforms into the Crystal Form A when air-dried at room temperature for 25 minutes. The XRPD pattern of the Crystal Form D is substantially as shown in Figure 30. The Crystal Form D transforms into a mixed crystal of the Crystal Forms A and D when placed open at room temperature for 3 days, and transforms into the Crystal Form A when placed open at room temperature for 4 days. The XRPD pattern of the Crystal Form E is substantially as shown in Figure 31. The Crystal Form E transforms into the Crystal Form A when placed open at room temperature for 3 days. The XRPD pattern of the Crystal Form J is substantially as shown in Figure 32. The Crystal Form J transforms into the Crystal Form A when placed open at room temperature for 3 days. The XRPD pattern of the Crystal Form K is substantially as shown in Figure 33. The Crystal Form K transforms into the Crystal Form A when placed open at room temperature for 3 days. The XRPD pattern of the Crystal Form L is substantially as shown in Figure 34. The Crystal Form L transforms into the Crystal Form A when placed open at room temperature for 3 days. The XRPD pattern of the Crystal Form M is substantially as shown in Figure 35. The Crystal Form M transforms into the Crystal Form E when placed open at room temperature for 3 days. The XRPD pattern of the Crystal Form Q is substantially as shown in Figure 36. The Crystal Form Q transforms into the Crystal Form U when placed open at room temperature for 1 day. The XRPD pattern of the Crystal Form R is substantially as shown in Figure 37. The Crystal Form R transforms into a mixed crystal of the Crystal Forms A and R when placed open at room temperature for 1 day. The XRPD pattern of the Crystal Form T is substantially as shown in Figure 39. The Crystal Form T transforms into a mixed crystal of the Crystal Forms A and T when placed open at room temperature for 3 days. The XRPD pattern of the Crystal Form U is substantially as shown in Figure 40. The Crystal Form U transforms into a mixed crystal of the Crystal Forms A and U when placed open at room temperature for 1 day, and remains a mixed crystal of the Crystal Forms A and U when placed open at room temperature for 10 days.

## IV. Pharmaceutical Compositions

In another aspect, the present invention provides a pharmaceutical composition comprising the pharmaceutically acceptable salt of Compound I of the present invention, wherein the pharmaceutically acceptable salt includes a tromethamine salt, a mesylate salt, and a meglumine salt.

In some embodiments, the pharmaceutically acceptable salt of Compound I is a mesylate salt, preferably a crystalline form of the mesylate salt, and more preferably Crystal Form A of the mesylate salt. The XRPD pattern of the Crystal Form A of the mesylate salt is substantially as shown by curve A in Figure 43.

In some embodiments, the pharmaceutically acceptable salt of Compound I is a meglumine salt, preferably a crystalline form of the meglumine salt, and more preferably Crystal Form A of the meglumine salt. The XRPD pattern of the Crystal Form A of the meglumine salt is substantially as shown in Figure 42.

In some preferred embodiments, the pharmaceutically acceptable salt of Compound I is a tromethamine salt of the Compound I, preferably Compound Z, and more preferably a crystalline form of Compound Z. In some preferred embodiments, the crystalline form of the tromethamine salt is selected from the Crystal Forms A, P, C, F, G, H, Ix, N, O, B, D, E, J, K, L, M, Q, R, S, T, U, V, and W of Compound Z of the present invention. In some more preferred embodiments, the crystalline form of the tromethamine salt is selected from the Crystal Forms A, P, C, V, and W of Compound Z of the present invention. More preferably, the crystalline form of the tromethamine salt is the Crystal Forms A and V.

The present invention also provides the pharmaceutical composition as described above, which further comprises one, two, or more of other therapeutically active ingredients.

The pharmaceutical composition of the present invention can also comprise a pharmaceutically acceptable carrier.

In the present invention, a "pharmaceutically acceptable carrier" refers to a diluent, adjuvant, excipient, or vehicle with which a therapeutic agent is administered, and which is, within the scope of sound medical judgment, suitable for contact with the tissues of humans and/or other animals without excessive toxicity, irritation, allergic response, or other problems or complications commensurate with a reasonable benefit/risk ratio.

Pharmaceutically acceptable carriers that can be used in the pharmaceutical composition of the present invention include, but are not limited to, sterile liquids, such as water and oils, including those of petroleum, animal, vegetable, or synthetic origin, such as peanut oil, soybean oil, mineral oil, sesame oil, etc. When the pharmaceutical composition is administered intravenously, water is an exemplary carrier. Saline solutions and aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, skim milk powder, glycerol, propylene glycol, water, ethanol, etc. The composition may also, if desired, contain minor amounts of wetting agents, emulsifying agents, or pH buffering agents. Oral formulations may include standard carriers such as drug grade mannitol, lactose, starch, magnesium stearate, sodium saccharin, cellulose, magnesium carbonate, etc. Examples of suitable pharmaceutically acceptable carriers are described in Remington's Pharmaceutical Sciences (1990).

The pharmaceutical composition of the present invention can act systemically and/or locally. For this purpose,

they can be administered by suitable routes, such as by injection, intravenously, intra-arterially, subcutaneously, intraperitoneally, intramuscularly, or transdermally; or orally, buccally, nasally, transmucosally, topically, in the form of ophthalmic formulation, or by inhalation.

The pharmaceutical composition of the present invention can be administered in suitable dosage forms for these administration routes.

The dosage forms include, but are not limited to, liquid preparations, semi-solid preparations, and solid preparations. Solid or semi-solid preparations include, but are not limited to, capsules, tablets, pills, lozenges, dragees, granules, powders, ointments, and creams. Liquid preparations include, but are not limited to, elixirs, syrups, emulsions, dispersions, suspensions, solutions, sprays, and drops.

As used herein, the term "therapeutically effective amount" refers to an amount of a compound that, when administered, will to some extent alleviate one or more symptoms of the disease or disorder being treated.

The dosing regimen may be adjusted to provide the optimal desired response. For example, a single bolus may be administered, several divided doses may be administered over time, or the dose may be reduced or increased proportionally as indicated by the exigencies of the situation of therapy. Of note is that the amount of the dosage may vary with the type and severity of the condition to be alleviated and may include single or multiple doses. It should be further understood that for any particular subject, the specific dosing regimen should be adjusted over time according to the subject's need and the professional judgment of the person administering or supervising the administration of the composition.

The amount of the compound of the present invention administered will depend on the subject being treated, the severity of the disorder or condition, the rate of administration, the disposition of the compound, and the judgment of the prescribing physician. Generally, an effective dose is about 0.0001 to about 50 mg per kg body weight per day, for example, about 0.01 to about 10 mg/kg/day (as a single or divided dose). For a 70 kg person, this will amount to about 0.007 mg/day to about 3500 mg/day, such as about 0.7 mg/day to about 700 mg/day. In some cases, a dose level not more than the lower limit of the aforementioned range may be sufficient, while in other cases, a larger dose may still be employed without causing any harmful side effects, provided that the larger dose is first divided into several smaller doses for administration throughout the day.

The content or amount of the compound of the present invention in the pharmaceutical composition can be about 0.01 mg to about 1000 mg.

As used herein, the term "treatment" means reversing, alleviating, suppressing a disease or disorder to which the term applies, or the progression of one or more symptoms of the disease or disorder. As used herein, the term "prevention" means preventing or deterring the development of, or the appearance of one or more symptoms of a disease or disorder to which the term applies.

As used herein, "subject" includes humans or non-human animals. Exemplary human subjects include human subjects suffering from a disease, e.g., the diseases described herein (referred to as patients) or normal subjects. "Non-human animals" in the present invention include all vertebrates, such as non-mammals (e.g., birds, amphibians, reptiles) and mammals, such as non-human primates, livestocks and/or domestic animals (e.g., sheep, dogs, cats, cows, pigs, etc.).

## V. Uses and Treatment Methods

The pharmaceutically acceptable salts of Compound I of the present invention, preferably Compound Z, more preferably the crystalline forms thereof described above, have excellent GLP-1 receptor agonistic activity and can treat and/or prevent GLP-1 receptor mediated diseases and related disorders.

Therefore, in one aspect, the present invention provides a method for treating and/or preventing GLP-1 receptor mediated diseases or related disorders, comprising administering an effective amount of the pharmaceutically acceptable salt of Compound I of the present invention or the pharmaceutical composition of the present invention to a subject in need thereof.

In another aspect, the present invention provides the pharmaceutically acceptable salt of Compound I of the present invention or the pharmaceutical composition of the present invention for use in treating and/or preventing GLP-1 receptor mediated diseases and related disorders.

In another aspect, the present invention provides the use of the pharmaceutically acceptable salt of Compound I of the present invention or the pharmaceutical composition of the present invention in the manufacture of a medicament for treating and/or preventing GLP-1 receptor mediated diseases or related disorders.

In another aspect, the present invention provides a method for treating and/or preventing metabolism related diseases or disorders, comprising administering an effective amount of the pharmaceutically acceptable salt of Compound I of the present invention or the pharmaceutical composition of the present invention to a subject in need thereof.

In another aspect, the present invention provides the pharmaceutically acceptable salt of Compound I of the present invention or the pharmaceutical composition of the present invention for use in treating and/or preventing

metabolism related diseases or disorders.

In another aspect, the present invention provides the use of the pharmaceutically acceptable salt of Compound I of the present invention or the pharmaceutical composition of the present invention in the manufacture of a medicament for treating and/or preventing metabolism related diseases or disorders.

In some embodiments, the metabolism related diseases or disorders include GLP-1 receptor mediated diseases and related disorders.

In some embodiments, the GLP-1 receptor mediated diseases or related disorders are selected from diabetes, hyperglycemia, insulin resistance, impaired glucose tolerance, diabetic nephropathy, diabetic neuropathy, diabetic retinopathy, adipocyte dysfunction, obesity, non-alcoholic fatty liver disease, dyslipidemia, and hyperinsulinemia.

In some embodiments, the diabetes is selected from T1D and/or T2DM, idiopathic T1D, early-onset T2DM, latent autoimmune diabetes, atypical diabetes in adolescents, and gestational diabetes.

In some embodiments, the GLP-1 receptor mediated disease or related disorder is obesity.

In some embodiments, the GLP-1 receptor mediated disease or related disorder is T2DM.

In some embodiments, the GLP-1 receptor mediated disease or related disorder is non-alcoholic fatty liver disease.

In some embodiments, the pharmaceutically acceptable salt is the tromethamine salt of Compound I, preferably Compound Z of the present invention, more preferably the crystalline forms of Compound Z as described above, further more preferably the Crystal Form A, Crystal Form P, Crystal Form C, Crystal Form V or Crystal Form W of Compound Z, and even more preferably the Crystal Form A or Crystal Form V.

## Beneficial Effects

The Compound Z of the present invention has good salt formation and better preparability; it improves the physical and chemical properties of Compound I, for example, it provides increased water solubility, enhanced chemical stability and/or reduced hygroscopicity, thereby increasing the bioavailability of Compound I. The crystal forms of Compound Z of the present invention (preferably Crystal Forms A, P, C, V, and W) have improved physical and chemical properties (including increased solubility, dissolution rate, and light resistance; low hygroscopicity; enhanced solid-state stability (high-temperature resistance, high-humidity resistance, and high-pressure resistance) and/or chemical stability), and thus may have better drugability and/or bioavailability. For example, the Crystal Form A of Compound Z is a thermodynamically more stable crystal form at room temperature and 50°C, and is stable at a water activity aw≤0.388; there is no significant decrease in purity and no crystal form change is observed under long-term (25°C/60%RH) and accelerated (40°C/75%RH) conditions, high-temperature (60°C) conditions, high-humidity (90% RH) conditions, and high-pressure (1000 Mpa) conditions; as compared to the amorphous form of the free Compound I, it has reduced hygroscopicity. Crystal Form V of Compound Z is also a stable crystal form at room temperature, and the crystal transformation occurs only under very harsh conditions (it transforms into Crystal Form W when heated to 100°C for 10 minutes); and it also has good stability under high-temperature, high-humidity, and high-pressure conditions. In addition, Crystal Form W of Compound Z transforms into the more stable Crystal Form V after being placed at room temperature for 45 minutes.

## Examples

The present invention is further elaborated through the following examples. The examples of the present invention are only used to illustrate the technical solutions of the present invention and are not intended to limit the scope of the present invention. Those skilled in the art can make some non-substantial improvements and adjustments, which still fall within the protection scope of the present invention.

All solvents used in the examples are commercially available and can be used without further purification.

The abbreviations used in this application have the meanings as follows: r.t. represents room temperature; $H_2O$ represents water; $CH_2Cl_2$ represents dichloromethane; THF represents tetrahydrofuran; IPA represents isopropyl alcohol; 2-MeTHF represents 2-methyltetrahydrofuran; NMP represents N-methylpyrrolidone; DME represents dimethoxyethane; DCM represents dichloromethane; Xphos represents 2-dicyclohexylphosphino-2',4',6'-triisopropylbiphenyl; EtOAc represents ethyl acetate; MeOH represents methanol; 2-Me-THF represents 2-methyltetrahydrofuran; DAST represents diethylaminosulfur trifluoride; TFE represents tetrafluoroethylene; ACN represents acetonitrile; CPME represents cyclopentyl methyl ether; DMSO represents dimethyl sulfoxide; EtOH represents ethanol; TFA represents trifluoroacetic acid; TsOH represents p-toluenesulfonic acid; MIBK represents methyl isobutyl ketone; HEP represents n-heptane; IPAc represents isopropyl acetate; EtOAc represents ethyl acetate; DMF represents N,N-dimethylformamide; MTBE represents methyl tert-butyl ether; MEK represents methyl ethyl ketone; $CHCl_3$ represents chloroform; MCH represents methylcyclohexane.

Compounds are named manually or by ChemDraw® software, and commercially available compounds adopt the

names in the supplier catalog.

The instruments and parameters used in this application are as follows:

1. X-ray Powder Diffractometer (XRPD)

Table 1: XRPD Test Parameters

| X-ray Powder Diffractometer (XRPD) | |
|---|---|
| Model | Bruker D8 Advance |
| Serial Number | CA312 |
| Technical Specifications | Cu Kα irradiation at a wavelength of 1.54 Å (40 kV, 40 mA), θ-2θ goniometer, nickel filter, SSD160-2 detector |
| Acquisition Software | DIFFRAC.MEA.CENTER |
| Calibration Substance | Corundum ($Al_2O_3$) |
| c Software | MDI Jade 6 |
| Detection Angle | 3-40° 2θ |
| Step Size | 0.02° 2θ |
| Speed | 0.1 s/step |
| Amount of Test Sample | >1 mg |
| Remarks | Unless otherwise specified, the samples are not ground before detection. |

2. Thermal Gravimetric Analyzer (TGA) and Differential Scanning Calorimeter (DSC)

Table 2: TGA and DSC Test Parameters

| Instruments | | METTLER TOLEDO TGA 2 | METTLER TOLEDO DSC 3 |
|---|---|---|---|
| | Serial Number | CA201 | CA200 |
| | Control Software | STARe Software | STARe Software |
| | Analystic Software | STARe Software | STARe Software |
| | Sample Pan | Alumina Crucible | Aluminum Crucible (with perforated Lid) |
| Parameters | Sample amount for Detection | 1 mg-10 mg | 0.5 mg-5 mg |
| | Protective Gas | Nitrogen | Nitrogen |
| | Gas Flow Rate | 50 mL/min | 50 mL/min |
| | General Detection Method | Segment 1<br>Initial Temperature: 30.0°C<br>Final Temperature: 350.0°C<br>Heating Rate: 10.0 K/min | Segment 1<br>Initial Temperature: 30°C<br>Final Temperature: 300°C<br>Heating Rate: 10.0 K/min |

3. High-Performance Liquid Chromatography (HPLC)

Table 3: HPLC Test Parameters

<table>
<tr><td>Instrument</td><td colspan="3">High Performance Liquid Chromatography</td></tr>
<tr><td>Chromatographic Column</td><td colspan="3">Agilent Poroshell 120 EC-C18, 150mm * 4.6mm, 4μm</td></tr>
<tr><td>Mobile Phase</td><td colspan="3">Mobile Phase A: 10 mM aqueous ammonium acetate Solution, adjusted to pH 5.0 with acetic acid<br>Mobile Phase B: methanol/acetonitrile (3:2, v:v)</td></tr>
<tr><td>Flow Rate</td><td colspan="3">1.2 mL/min</td></tr>
<tr><td>Acquisition Time</td><td colspan="3">45 min</td></tr>
<tr><td>Determination Wavelength</td><td colspan="3">220 nm (purity)/ 270 nm (solubility)</td></tr>
<tr><td>Column Temperature</td><td colspan="3">40°C</td></tr>
<tr><td>Injector Temperature</td><td colspan="3">Room Temperature</td></tr>
<tr><td>Sample Concentration and Injection Volume</td><td colspan="3">Testing the samples from the small-scale trial of salt formation: 1.0 mg/mL, 10 μL<br>Salt type assessment: 1.0 mg/mL, 5 μL, or 0.5 mg/mL, 10 μL<br>Crystal form screening: 0.5 mg/mL, 10 μL</td></tr>
<tr><td>Diluents</td><td colspan="3">Testing the samples from the small-scale trial of salt formation: acetonitrile/water (1:4, v:v)<br>Salt type assessment and Crystal form screening: acetonitrile/water (1:1, v:v)</td></tr>
<tr><td rowspan="8">Gradient Elution Schedule</td><td>Time (min)</td><td>A%</td><td>B%</td></tr>
<tr><td>0</td><td>60</td><td>40</td></tr>
<tr><td>3</td><td>60</td><td>40</td></tr>
<tr><td>25</td><td>30</td><td>70</td></tr>
<tr><td>30</td><td>10</td><td>90</td></tr>
<tr><td>35</td><td>10</td><td>90</td></tr>
<tr><td>36</td><td>60</td><td>40</td></tr>
<tr><td>45</td><td>60</td><td>40</td></tr>
</table>

4. Dynamic Vapor Sorption (DVS) Determination

Table 4: DVS Test Parameters

<table>
<tr><td colspan="2">Dynamic Vapor Sorption Instrument (DVS)</td></tr>
<tr><td>Model</td><td>Intrinsic PLUS</td></tr>
<tr><td>Serial Number</td><td>DVS-001 (CA357)</td></tr>
<tr><td>Method and Parameters</td><td>1. Equilibrate at 25°C<br>2. Humidity 0%<br>3. Isothermal for 360 min<br>4. Abort next iso if weight (%)<0.0200<br>5. Step humidity 0% to 90%<br>6. Abort next iso if weight (%)<0.0200<br>7. Step humidity 90% to 0%</td></tr>
</table>

5. Proton Nuclear Magnetic Resonance ($^1H$ NMR)

The proton nuclear magnetic resonance ($^1H$ NMR) spectra in this application were collected on a Bruker AVANCE-III or Bruker AVANCE NEO (Bruker, Germany) nuclear magnetic resonance spectrometer, using the following parameters: full-frequency excitation, a spectral width of 20 ppm, a single pulse, a 30° angle excitation, scanned 8 times, digital orthogonal detection, the temperature controlled at 298K, and MeOD-d4 as the solvent.

**Example 1: Preparation of Compound 1**

Synthetic Scheme

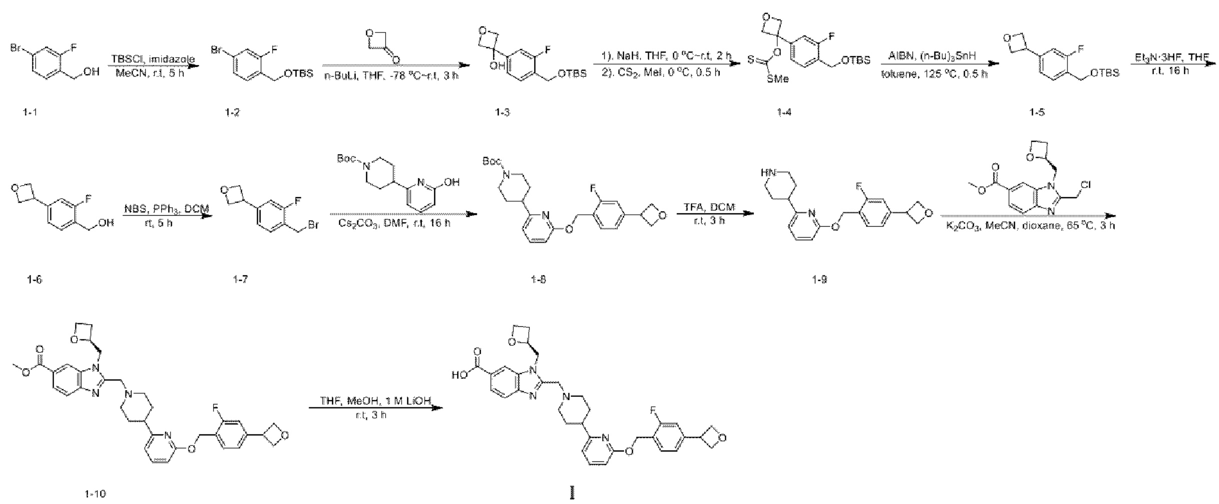


Preparation method

Compound 1-2: To a solution of Compound 1-1 (20.0 g, 98.0 mmol) in MeCN (500 mL) was added imidazole (10.0 g, 147.0 mmol) followed by addition of TBSCl (16.3 g, 107.8 mmol). The mixture was stirred at room temperature for 5 h. $H_2O$ (500 mL) was added. The reaction solution was extracted with EtOAc (3 × 500 mL). The combined organic phases were washed with brine (500 mL), dried ($Na_2SO_4$), filtered, and concentrated, and subjected to flash chromatography ($SiO_2$, hexane) to give 31 g of compound 1-2. Yield: 99.6%. $^1H$ NMR (400 MHz, DMSO-d6) δ 7.35 (m, 3H), 4.62 (s, 2H), 0.81 (s, 9H), 0.00 (s, 6H).

Compound 1-3: To a solution of Compound 1-2 (20.0 g, 62.8 mmol) in anhydrous THF (200 mL) was added dropwise N-BuLi (2.5 M in THF, 27.6 mL, 69.1 mmol) at -78°C under $N_2$. The mixture was stirred at this temperature for 0.5 h, and then added with oxetane-3-one (4.5 g, 62.8 mmol). The mixture was then stirred at room temperature under $N_2$ atmosphere for 2.5 h. The reaction solution was quenched with water (100 mL), and extracted with EtOAc (3 × 100 mL). The combined organic phases were washed with brine (100 mL), dried ($Na_2SO_4$), filtered, and concentrated, and subjected to flash chromatography ($SiO_2$, 25% EtOAc-hexane) to give 14 g of compound 1-3. Yield: 71.0%. $^1H$ NMR (400 MHz, DMSO-d6) δ 7.42 - 7.33 (m, 2H), 7.26 - 7.18 (m, $^1H$), 6.36 (s, $^1H$), 4.69 - 4.53 (m, 6H), 0.81 (s, 9H), - 0.00 (s, 6H).

Compound 1-4: To a solution of Compound 1-3 (14.0 g, 44.8 mmol) in anhydrous THF (200 mL) was added NaH (3.6 g, 89.7 mmol) at 0°C. The mixture was stirred at room temperature for 2 h, and then added with $CS_2$ (3.6 g, 89.7 mmol) and MeI (6.4 g, 44.8 mmol) at 0°C under $N_2$. The mixture was then stirred at 0°C in $N_2$ for 0.5 h. The reaction solution was quenched with saturated $NH_4Cl$ solution (100 mL), and extracted with EtOAc (3 × 200 mL). The combined organic phases were washed with brine (200 mL), dried ($Na_2SO_4$), filtered, and concentrated to give 14 g of compound 1-4. The product was used directly in the next step without further purification.

Compound 1-5: To a solution of Compound 1-4 (14.0 g, 44.8 mmol) in toluene (200 mL) was added $(n\text{-}Bu)_3SnH$ (26.2 g, 89.7 mmol) followed by addition of AIBN (736 mg, 4.4 mmol). The mixture was stirred at 125°C under $N_2$ atmosphere for 0.5 h. The reaction solution was concentrated, and purified by flash chromatography ($SiO_2$, 20% EtOAc-hexane) to give 8 g of compound 1-5. Two-step yield: 60.6%. $^1H$ NMR (400 MHz, DMSO-d6) δ 7.41 (t, *J* = 8.0 Hz, $^1H$), 7.23 - 7.16 (m, 2H), 4.91 (dd, *J* = 8.3, 5.9 Hz, 2H), 4.72 (s, 2H), 4.59 (t, *J* = 6.3 Hz, 2H), 4.30 - 4.18 (m, $^1H$), 0.88 (s, 9H), 0.07 (s, 6H).

Compound 1-6: To a solution of Compound 1-5 (8.0 g, 43.0 mmol) in THF (200 mL) was added $Et_3N{\cdot}HF_3$ (13.9 g, 86.0 mmol). The reaction solution was stirred at room temperature under $N_2$ atmosphere for 16 h. The reaction solution

was concentrated, and purified by flash chromatography ($SiO_2$, EtOAc-hexane) to give 5 g of compound 1-6. Yield: 99.9%. $^1$H NMR (400 MHz, DMSO-d6) δ 7.44 (t, *J* = 7.8 Hz, $^1$H), 7.20 (t, *J* = 9.1 Hz, 2H), 5.22 (t, *J*= 5.7 Hz, $^1$H), 4.92 (dd, *J* = 8.0, 6.1 Hz, 2H), 4.59 (t, *J* = 6.3 Hz, 2H), 4.52 (d, *J*= 5.6 Hz, 2H), 4.30 - 4.18 (m, $^1$H).

Compound 1-7: To a solution of Compound 1-6 (4.8 g, 26.3 mmol) in DCM (100 mL) was added NBS (5.2 g, 29.0 mmol) followed by addition of $PPh_3$ (7.7 g, 29.0 mmol) at 0°C. The mixture was stirred at room temperature under $N_2$ atmosphere for 5 h. $H_2O$ (100 mL) was added. The reaction solution was extracted with DCM (3 × 100 mL). The combined organic phases were washed with brine (100 mL), dried ($Na_2SO_4$), filtered, and concentrated, and purified by flash chromatography ($SiO_2$, EtOAc-hexane) to give 2 g of compound 1-7. Yield: 30.7%. $^1$H NMR (400 MHz, DMSO-d6) δ 7.53 (t, *J* = 8.0 Hz, $^1$H), 7.33 - 7.20 (m, 2H), 4.92 (dd, *J* = 8.3, 6.0 Hz, 2H), 4.70 (s, 2H), 4.60 (t, *J* = 6.3 Hz, 2H), 4.34 - 4.20 (m, $^1$H).

Compound 1-8: Compound 1-7 (600 mg, 2.45 mmol) and tert-butyl 4-(6-hydroxypyridin-2-yl)piperidine-1-carboxylate (684 mg, 2.45 mmol) were added to the solvent DMF (50 mL). Then $Cs_2CO_3$ (2.4 g, 7.37 mmol) was added. The reaction solution was stirred at room temperature for 16 h. $H_2O$ (50 mL) was added. The reaction solution was extracted with EtOAc (3 × 50 mL). The combined organic phases were washed with brine (50 mL), dried ($Na_2SO_4$), filtered, and concentrated, and purified by flash chromatography ($SiO_2$, EtOAc-hexane) to give 500 mg of compound 1-8. Yield: 45.9%. $^1$H NMR (400 MHz, $CDCl_3$) δ 7.60 (t, *J* = 7.7 Hz, $^1$H), 7.46 (t, *J* = 7.6 Hz, $^1$H), 7.17 (s, $^1$H), 7.13 (d, *J* = 11.4 Hz, $^1$H), 6.75 (d, *J* = 7.3 Hz, $^1$H), 6.68 (d, *J* = 8.1 Hz, $^1$H), 5.43 (d, *J* = 7.5 Hz, 3H), 5.33 (s, $^1$H), 4.45 (s, 2H), 4.14 (d, *J* = 14.0 Hz, 2H), 3.03 (t, *J* = 12.8 Hz, $^1$H), 2.80 (t, *J* = 12.9 Hz, 2H), 1.85 (d, *J* = 12.5 Hz, 2H), 1.57-1.61 (m 3H), 1.42 (s, 9H). / LC-MS (ESI) m/z: 443.2 $[M^+H]^+$.

Compound 1-9: To a solution of Compound 1-8 (210 mg, 0.49 mmol) in DCM (10 mL) was added TFA (10 mL). The reaction solution was stirred at room temperature for 3 h. The reaction solution was concentrated to give 250 mg of compound 1-9. LC-MS: MC20-1128-086C (ESI) m/z: 343.1 $[M^+H]^+$.

Compound 1-10: Compound 1-9 (200 mg, 0.58 mmol) and (S)-methyl 2-(chloromethyl)-1-(oxetan-2-ylmethyl)-1H-benzo[d]imidazole-6-carboxylate (172 mg, 0.58 mmol) were added to the solvents dioxane (20 mL) and MeCN (12 mL), followed by $K_2CO_3$ (162 mg, 1.16 mmol). The reaction solution was stirred at 65°C for 3 h. $H_2O$ (20 mL) was added. The reaction solution was extracted with EtOAc (3 × 20 mL). The combined organic phases were washed with brine (20 mL), dried ($Na_2SO_4$), filtered, and concentrated, and purified by flash chromatography ($SiO_2$, EtOAc-hexane) to give 60 mg of compound 1-10. Yield: 22.0%. $^1$H NMR (400 MHz, DMSO-d6) δ 8.30 (d, *J* = 1.1 Hz, $^1$H), 7.82 (dd, *J* = 8.5, 1.6 Hz, $^1$H), 7.70 - 7.59 (m, 2H), 7.53 (t, *J* = 7.8 Hz, $^1$H), 7.27 (d, *J* = 11.3 Hz, $^1$H), 7.21 (d, *J* = 9.5 Hz, $^1$H), 6.86 (d, *J* = 7.4 Hz, $^1$H), 6.65 (d, *J* = 8.0 Hz, $^1$H), 5.38 (s, 2H), 5.35 - 5.30 (m, $^1$H), 5.12 (qd, *J* = 7.0, 2.5 Hz, $^1$H), 4.90 (dd, *J* = 8.3, 6.0 Hz, 2H), 4.80-4.84 (m $^1$H), 4.65-4.71 (m, $^1$H), 4.58 (t, *J* = 6.4 Hz, 2H), 4.47 (dt, *J* = 8.3, 6.5 Hz, $^1$H), 4.37 (dt, *J* = 9.1, 5.9 Hz, $^1$H), 4.21-4.28 (m,$^1$H), 3.94-4.02 (m,$^1$H), 3.87 (s, 3H), 3.78 (d, *J* = 13.6 Hz, $^1$H), 3.01 (d, *J* = 9.4 Hz, $^1$H), 2.85 (d, *J* = 13.5 Hz, $^1$H), 2.73 - 2.59 (m, 2H), 2.27 (d, *J* = 10.0 Hz, $^1$H), 2.17 (d, *J* = 11.6 Hz, $^1$H), 1.76 (m, 4H). / LC-MS (ESI) m/z: 601.4 $[M^+H]^+$.

Compound 1: To a solution of Compound 1-10 (60 mg, 0.1 mmol) in MeOH (1 mL) and THF (5 mL) was added 1 M LiOH (2 mL). The reaction solution was stirred at room temperature for 3 h. The reaction solution was concentrated, and purified by preparative HPLC to give 10.95 mg of compound 1 as a white solid. Yield: 18.6%. $^1$H NMR (400 MHz, DMSO-d6) δ 8.20 (s, $^1$H), 7.79 (dd, $J_1$ = 4.0 Hz, , $J_2$ = 8.0 Hz, $^1$H), 7.62 (t, J = 8.0 Hz, $^1$H), 7.54 (t, J = 8.0 Hz, $^1$H), 7.45 (d, J = 8.0 Hz, $^1$H), 7.27 (d, J = 12.0 Hz, $^1$H), 7.21 (d, J = 8.0 Hz, $^1$H), 6.87 (d, J = 8.0 Hz, $^1$H), 6.65 (d, J = 8.0 Hz, $^1$H), 5.38 (s, 2H), 5.12 (m, $^1$H), 4.90 (dd, J = 8.0 Hz, 2H), 4.77 (dd, $J_1$ = 4.0 Hz, $J_2$ = 16.0 Hz, $^1$H), 4.64 (d, J = 4.0 Hz, $^1$H), 4.58 (m, 2H), 4.50 - 4.44 (m, $^1$H), 4.38 (m, $^1$H), 4.29 - 4.19 (m, $^1$H), 3.94 (d, J = 12.0 Hz, $^1$H), 3.77 (d, J = 12.0 Hz, $^1$H), 3.00 (d, J = 12.0 Hz, $^1$H), 2.86 (d, J = 12.0 Hz, $^1$H), 2.71 (m, $^1$H), 2.64 - 2.56 (m, $^1$H), 2.47 - 2.42 (m, $^1$H), 2.21 (m, 2H), 1.73 (m, 4H).

Compound I was in an amorphous form, as determined by XRPD, with an XRPD pattern as shown in Figure 41.

**Example 2 - Assay of GLP-1R agonistic activity of Compound I**

(1) Test instruments and reagents

Table 5: Instruments and Reagents for Biological Activity Tests

| Instruments/reagents | Supplier | Model |
|---|---|---|
| cAMP-GS DYNAMIC kit | CisBio | 62AM4PEC |
| DMEM | CellMax | CGN101.5 |
| FBS | Gemini | 900-108 |
| 1% Pen-3trep | Sangom biotech | E607011-0100 |
| IBMX | Meilunbio | MB5226 |

(continued)

| Instruments/reagents | Supplier | Model |
|---|---|---|
| 384 well plate | Corning | 3824 |
| Incubator | Thermo | 3111 |
| Microscope | Jiangnan | XD-202 |
| Cell counter | Counter Star | Star IC1000 |
| Plate reader | Tecan | Tecan Spark |

(2) GLP-1R kit

GLP-1R-mediated agonist activity was determined by cell-based assays using a homogeneous time-resolved fluorescence (i.e., HTRF)-based cAMP detection kit, which measures the level of cAMP in cells. The method was a competitive immunoassay. It enabled direct pharmacological characterization of compounds acting on Gs-coupled receptors in adherent or suspending cells.

The standard curve of native cAMP or unlabeled cAMP produced by cells competed with d2-labeled cAMP red receptors to bind monoclonal anti-cAMP Eu3+ cryptate donors, and the specific signal was inversely proportional to the concentration of cAMP in standard or tested samples.

Human GLP-1R encoding sequence (NCBI reference sequence NP_002053.3) was subcloned into pEGFP-N1 (tsingke), and the cell line stably expressing the receptor was isolated. The expression density of GLP-1R was confirmed by the expression of GFP observed under a fluorescence microscope.

(3) GLP-1R-GFP-293A cell culture

293A GFP-GLP-1R cells were incubated in DMEM growth medium, 10% heat-inactivated fetal bovine serum (GEMINI Cat 900-108), 1% Pen-3Trep (Sangom Biotech Cat E607011-0100)] in a moist incubator with 5% $CO_2$ at 37 °C.

(4) cAMP level test method

The tested compound (in DMSO) at different concentrations was 1:5 diluted in distilled water in a stimulating buffer, followed by addition of 500 μm 3-isobutyl-1-methylxanthine (IBMX; Meilunbiocat MB5226) to obtain a working solution of 2X compound, and then 5 μL of the compound was added to a white 384-well assay plate (Corning 3824) using a multichannel pipette. The final DMSO concentration in the buffer mixture was determined to be 1 ‰.

Cells were collected from a T25 tissue culture flask and centrifuged at room temperature at 1000 rpm for 5 minutes. The cell precipitates were then re-suspended in 1 mL of the stimulating buffer. 20 μL sample of cell suspension was counted on a counter STAR IC 1000 to determine the cell viability and the cell count per mL. The remaining cell suspension was then regulated with the stimulating buffer to deliver 2000 living cells per well using a multichannel pipette. 5 μL of the cell suspension was added to each well of the plate which already contained the compound. The plate was sealed and incubated at 37 °C with 5% $CO_2$ for 30 minutes.

After 30 minutes of incubation, 5 μL of d2-labeled cAMP and 5 μL of anti-cAMP cryptate (both 1: 20 diluted in the cell lysis buffer) were added to each well of the plate. The plate was then incubated at room temperature for 60 minutes, and the changes of HTRF signal were read with Tecan Spark reader: absorbance values at 340 nm (excitation)/at 615 nm and 665 nm (emission). Raw data were converted into nM cAMP by interpolation from the cAMP standard curve, and the effect in percentage was determined relative to the saturated concentration of the complete agonist GLP-17~37 (400 nM) contained in each plate. Determination of EC50 was performed based on the agonist dose-response curve, which was analyzed using a four-parameter logical dose-response equation with a curve fitting program.

This test proved that Compound I activated GLP-1R signaling through the cAMP pathway, thus acting as a GLP-1R agonist. The test data presented the results in the form of a geometric mean ($EC_{50}$s) based on the number of repetition times.

Experimental results: Compound I has a strong agonistic effect on GLP-1R.

| Compound | $EC_{50}$ (nM) |
|---|---|
| 1 | 0.03 |

**Example 3: Preparation of Compound Z**

Approximately 200 mg of a sample of Compound I (in free state) was weighed and placed into a 20.0 mL glass vial, added with 49.50 mg of tromethamine, and added with 5 mL of isopropanol. The dissolution of the sample was promoted by vortexing and sonication. After suspending with stirring on a magnetic stirrer at room temperature for 3 days, the resulting solid was separated by vacuum filtration, and then was dried at 50°C in an oven for 4 hours to obtain the tromethamine salt. The $^1H$ NMR (Figure 4-1) showed that the acid-base molar ratio was 1:1, with no obvious solvent residue, indicating that it was an anhydride. The structure was determined to be:

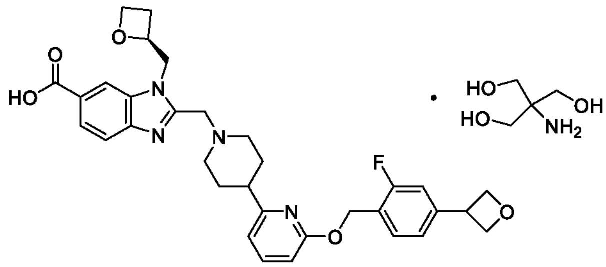

(Z).

It was determined to be Crystal Form A by detection through XRPD, as shown in Figure 1.

**Example 4: Small-scale screening of salt types of Compound I**

Based on the structure of the free Compound I, a screening test was set up using 8 acidic ligands (citric acid, malic acid, succinic acid, tartaric acid, fumaric acid, hydrochloric acid, maleic acid, methanesulfonic acid) and 7 basic ligands (sodium hydroxide, potassium hydroxide, calcium hydroxide, magnesium hydroxide, meglumine, choline, tromethamine) in 4 solvent systems (isopropanol, methyl isobutyl ketone, water/acetonitrile, methyl tert-butyl ether/tetrahydrofuran) by means of suspending with stirring at room temperature.

Approximately 20 mg of Compound I (in free state) was added into a 3.0 mL vial, and 1.2 times (in moles) of the ligand and 0.5 mL of the solvent were added. After magnetic stirring at room temperature for 3 days, the resulting solid was separated, vacuum-dried at 50°C for 2 hours, and then measured for XRPD.

Test results: The results are shown in Table E-1. Obviously, 4 crystal forms of 4 salts (Crystal Form A of the hydrochloride, Crystal Form A of the mesylate salt, Crystal Form A of the meglumine salt, and Crystal Form A of the tromethamine salt (Compound Z)) were obtained from the test.

Table E-1: Results of small-scale salt-type screening

| Solvent / Crystal Form / Ligand | Isopropanol | Methyl Isobutyl Ketone | Water/Acetonitrile (1:1, v:v) | Methyl Tert-Butyl Ether/Tetrahydrofuran (4:1, v:v) |
|---|---|---|---|---|
| Citric acid | Compound I | Compound I + citric acid | Oily | Compound I |
| L-Malic acid | Compound I | Weakly crystalline state | Oily | Compound I |
| Succinic acid | Compound I | Compound I | Oily | Compound I |

| | | | | |
|---|---|---|---|---|
| L-Tartaric acid | Compound I | Amorphous | Weakly crystalline state | Weakly crystalline state |
| Fumaric acid | Compound I | Compound I | Amorphous | Compound I |
| Hydrochloric acid | Oily | Crystal Form A of the hydrochloride (Figure 44) | Oily | Crystal Form A of the Hydrochloride |
| Maleic acid | Oily | Amorphous | Oily | Amorphous |
| Methanesulfonic acid | Crystal Form A of the mesylate salt (curve A in Figure 43) | Compound I | Compound I | Compound I |
| Sodium hydroxide | Oily | Amorphous | Oily | Amorphous |
| Potassium hydroxide | Oily | Oily | Oily | Oily |
| Calcium hydroxide | Compound I + calcium hydroxide | Calcium hydroxide | Calcium hydroxide | Compound I + calcium hydroxide |
| Magnesium hydroxide | Compound I + magnesium hydroxide | Compound I + magnesium hydroxide | Magnesium hydroxide | Compound I + magnesium hydroxide |
| Meglumine | Crystal Form A of the meglumine salt + meglumine | Oily | Oily | Compound I + meglumine |
| Choline | Oily | Oily | Oily | Oily |
| Tromethamine | Crystal Form A of Compound Z | Crystal Form A of Compound Z | Oily | Crystal Form A of Compound Z |
| Blank control | Compound I | Compound I | Compound I | Compound I |

As shown in Table E-1, the crystalline forms of the salts of Compound I were successfully obtained only when the ligands were hydrochloric acid, methanesulfonic acid, meglumine, and tromethamine. Especially in the case of tromethamine, the salt formation was excellent, and Crystal Form A of the tromethamine salt was obtained. Compared with the mesylate and meglumine salts, the tromethamine salt of Compound I was easier to prepare, and the post-treatment (such as separation) was also more convenient.

When other ligands were selected, either the salt of Compound I could not be formed, or only weakly crystalline or amorphous products were obtained, or the salt formation was poor (it was prone to form a gel or an oil).

Since there was a risk of degradation of Compound I under the action of strong acids or strong bases, the four salts were subjected to detection by high-performance liquid chromatography (HPLC), with the results shown in Table E-2.

Table E-2: Purity of hydrochloride, mesylate, meglumine, and tromethamine salts

| Solid Form | Purity by Area (%) |
|---|---|
| Compound I | 90.70 |
| Crystal Form A of the hydrochloride | 62.30 |
| Crystal Form A of the mesylate salt | 94.70 |
| Crystal Form A of the meglumine Salt | 92.18 |
| Crystal Form A of Compound Z | 92.33 |

**Example 5: Preparation of mesylate and meglumine salts of Compound I**

Test 1: Approximately 200 mg of Compound I (in free state) was added into a 20.0 mL glass vial, added with 5 mL

of isopropanol, and added with 39.34 mg of methanesulfonic acid. The dissolution of the sample was promoted by vortexing and sonication. After suspending with stirring on a magnetic stirrer at room temperature for 3 days, a mesylate salt was obtained. XRPD determination showed that it was amorphous (curve B in Figure 43).

Test 2: Approximately 100 mg of Compound I (in free state) was added into a 20.0 mL glass vial, added with 2.5 mL of ethyl acetate, and added with 19.67 mg of methanesulfonic acid. The dissolution of the sample was promoted by vortexing and sonication. After suspending with stirring on a magnetic stirrer at room temperature for 2 days, centrifugation was done and the supernatant was removed. The residue was dried at 50°C in an oven for 3 hours to obtain a mesylate salt. XRPD determination showed that it was amorphous (curve C in Figure 43).

Test 3: A mesylate salt was prepared by replacing the ethyl acetate with sec-butanol in Test 2. XRPD determination showed that it was amorphous (curve D in Figure 43).

Test 4: Approximately 200 mg of Compound I (in free state) was added into a 20.0 mL glass vial, added with 79.91 mg of meglumine, and added with 5 mL of isopropanol. The dissolution of the sample was promoted by vortexing and sonication. After suspending with stirring on a magnetic stirrer at room temperature for 3 days, it was dried at 50°C in an oven for 4 hours to obtain a meglumine salt. XRPD determination showed that it was Crystal Form A.

**Example 6: Preparation of crystal forms of Compound Z**

1. Crystal Form A

Approximately 4.6 g of Compound I and 867.75 mg of tromethamine were placed in a 250 mL eggplant shaped flask, and added with 120 mL of IPA. After stirring at room temperature for 3 days, a crystalline solid was obtained which was Crystal Form A of Compound Z. Its XRPD pattern was substantially the same as that of Crystal Form A obtained in Example 3.

2. Crystal Form P

Approximately 20 mg of Crystal Form A of Compound Z was suspended in 0.5 mL of $CHCl_3$/EtOAc (1: 1, v:v). The resulting suspension was magnetically stirred at 5°C for about 3 days, and then the solid was separated to give a crystalline solid which was Crystal Form P of Compound Z with an XRPD pattern shown in Figure 5.

3. Crystal Form C

Approximately 20 mg of Crystal Form A of Compound Z was suspended in 0.5 mL of 1,4-dioxane/$H_2O$ (6:1, v:v). The resulting suspension was magnetically stirred at room temperature for about 3 days, and then the solid was separated to give a crystalline solid which was Crystal Form C of Compound Z with an XRPD pattern shown in Figure 8.

4. Crystal Form F

Approximately 20 mg of Compound Z was dissolved in 0.2 mL of NMP/toluene (1:1, v:v) and filtered to obtain a clear solution. This solution was placed in a 20 mL vial containing 3 mL of IPAc. The vial was sealed and kept at room temperature to allow for the interaction between the organic vapor and the solution for sufficient time. After 3 days of gas-liquid diffusion, the solid was separated to give a crystalline solid which was Crystal Form F of Compound Z with an XRPD pattern shown in Figure 11.

5. Crystal Form G

Approximately 20 mg of Crystal Form A of Compound Z was suspended in 0.5 mL of 1,4-dioxane/EtOAc (1:1, v:v). The resulting suspension was magnetically stirred at 50°C for about 3 days, and then the solid was separated to give a crystalline solid which was Crystal Form G of Compound Z with an XRPD pattern shown in Figure 14.

6. Crystal Form H

Approximately 50 mg of Compound Z was dissolved in 3.0 mL of TFE and filtered. The filtrate was rotary-evaporated at 50°C, and the solid was collected to give a crystalline solid which was Crystal Form H of Compound Z with an XRPD pattern shown in Figure 17.

7. Crystal Form Ix

Approximately 20 mg of Crystal Form A of Compound Z was suspended in 0.5 mL of anisole. The resulting suspension was magnetically stirred at 50°C for about 3 days, and then the solid was separated to give a crystalline solid which was Crystal Form Ix of Compound Z with an XRPD pattern shown in Figure 20.

8. Crystal Form N

Approximately 20 mg of Crystal Form A of Compound Z was suspended in 0.5 mL of ACN/1,4-dioxane (1:1, v:v). The resulting suspension was magnetically stirred under temperature-cycling conditions for 2 days, and then the solid was separated to give a crystalline solid which was Crystal Form N of Compound Z with an XRPD pattern shown in Figure 23. The temperature-cycling program was as follows, and 2 cycles were carried out: $50°C \xrightarrow{120\ min} 50°C \xrightarrow{0.1°C/min} 5°C \xrightarrow{120\ min} 5°C$.

9. Crystal Form O

Approximately 20 mg of Crystal Form A of Compound Z was suspended in 0.5 mL of acetone/CPME (1:1, v:v). The resulting suspension was magnetically stirred at 50°C for about 3 days, and then the solid was separated to give a crystalline solid which was Crystal Form O of Compound Z with an XRPD pattern shown in Figure 26.

10. Crystal Form S

Approximately 20 mg of Compound Z was dissolved in 0.6 mL of TFE/ACN (1:1, v:v) and filtered to obtain a clear solution. 1.5 mL of MTBE was added to the solution with magnetic stirring until precipitation occurred. The solid was separated to give a crystalline solid which was Crystal Form S of Compound Z with an XRPD pattern shown in Figure 38-1.

11. Crystal Form V

Method 1: 5.0 g of Compound Z was added to a 250 ml three-necked flask, then added with 60 ml of acetonitrile and 10 ml of water, and transferred to 25°C and stirred for 3 hours after dissolving by heating at 55°C, and then filtered. The filter cake was dried with forced air at 50°C for 18 hours, and 4.52 g of a solid was obtained (yield: 90.4%) which had an XRPD pattern shown in Figure 45-1.

Method 2:

8.0 g of Compound Z was added to a 100 ml three-necked flask, add with 48 ml of methanol, and heated at 60°C to dissolve, and then the temperature was lowered to 40°C and 8 mg of crystal seeds of Crystal Form V were added. The solution was cooled to 15°C at a rate of 8°C/h, and then further cooled to -18°C at a rate of 12°C/h, and filtered with suction after stirring for 2 hours while maintaining the temperature. The filter cake was vacuum-dried at 40°C for 17 hours, and 6.67 g of a solid was obtained (yield: 83.3%) which had an XRPD pattern substantially the same as that in Figure 45-1.

Method 3:

A solid mixture of Crystal Forms V, A, and C of Compound Z was suspended in a saturated solution of Compound Z in a mixture of ethanol and water (0.205 ≤ water activity ≤ 0.49). The slurry was maintained at room temperature for 4 days, and then the resulting solid was separated which had an XRPD pattern substantially the same as that in Figure 45-1.

12. Crystal Form W

Method 1: Crystal Form V of Compound Z was heated at 100°C for 10 minutes under the protection of nitrogen to obtain Crystal Form W with an XRPD pattern shown in Figure 46.

Method 2:

A solid mixture of Crystal Forms V, A, and C of Compound Z was suspended in a saturated solution of Compound Z in a mixture of ethanol and water (water activity ≤ 0.12). The slurry was maintained at room temperature for 1 day, and then the resulting solid was separated which had an XRPD pattern substantially the same as that in Figure 46.

**Example 7: Preparation of Metastable Crystal Forms of Compound Z**

1. Crystal Form B

A suspension of Compound Z in ethanol:water (93:7, v:v) that had been equilibrated at room temperature for 2 hours was filtered to obtain a saturated solution of Compound Z at room temperature, and then a solid mixture of Crystal Forms A and C of Compound Z was added. After stirring for 3 days, Crystal Form B was obtained with an XRPD pattern shown in Figure 29.

2. Crystal Form D

Approximately 20 mg of Crystal Form A of Compound Z was suspended in 0.5 mL of DMSO/EtOAc (1:9, v:v). The resulting suspension was magnetically stirred at 50°C for about 3 days, and then the solid was separated to give Crystal Form D with an XRPD pattern shown in Figure 30.

3. Crystal Form E

Approximately 20 mg of Crystal Form A of Compound Z was suspended in 0.5 mL of DMF/2-MeTHF (1:9, v:v). The resulting suspension was magnetically stirred for 2 days under the temperature cycling conditions as described for Crystal Form N in Example 6, and then the solid was separated to give Crystal Form E with an XRPD pattern shown in Figure 31.

4. Crystal Form J

Approximately 20 mg of Crystal Form A of Compound Z was suspended in 0.5 mL of 2-MeTHF. The resulting suspension was magnetically stirred at room temperature for about 3 days, and then the solid was separated to give Crystal Form J with an XRPD pattern shown in Figure 32.

5. Crystal Form K

Approximately 20 mg of Crystal Form A of Compound Z was suspended in 0.5 mL of $CHCl_3$/MEK (1: 1, v:v). The resulting suspension was magnetically stirred at 50°C for about 3 days, and then the solid was separated to give Crystal Form K with an XRPD pattern shown in Figure 33.

6. Crystal Form L

Approximately 20 mg of Crystal Form A of Compound Z was suspended in 1.0 mL of EtOH/HEP (3: 1, v:v), and then the resulting suspension was heated to 50°C, and filtered after equilibrating for about two hours. The filtrate was slowly cooled to room temperature in a water bath, and the resulting solid was separated to give Crystal Form L with an XRPD pattern shown in Figure 34.

7. Crystal Form M

Approximately 20 mg of Compound Z was dissolved in 0.2 mL of NMP and filtered to obtain a clear solution. 2.0 mL of MTBE was added to the solution under magnetic stirring until precipitation occurred. The solid was separated to give Crystal Form M with an XRPD pattern shown in Figure 35.

8. Crystal Form Q

Approximately 20 mg of Compound Z was dissolved in 2.0 mL of TFE/MEK (v:v, 1:1) and filtered to obtain a clear solution. The vial containing the filtrate was sealed with Parafilm, and the Parafilm was perforated. After placing at room temperature for slow evaporation for 7 days, a small amount of solid precipitated, and then was dried at 50°C for 6 hours. The resulting solid was collected to give Crystal Form Q with an XRPD pattern shown in Figure 36.

9. Crystal Form R

Approximately 20 mg of Compound Z was dissolved in 1.0 mL of TFE/MEK (v:v, 1:1) and filtered to obtain a clear solution. Then, this solution was placed in a 20 mL vial containing 3 mL of $CHCl_3$. The vial was sealed and kept at room

temperature to allow for interaction between the organic vapor and the solution for sufficient time. After 7 days of gas-liquid diffusion, the solution remained clear, and then was dried at 50°C for 6 hours. The solid was collected to give Crystal Form R with an XRPD pattern shown in Figure 37.

10. Crystal Form T

Approximately 20 mg of Crystal Form A of Compound Z was suspended in 0.5 mL of toluene/IPA (1:1, v:v). The resulting suspension was magnetically stirred for 2 days under the temperature cycling conditions as described for Crystal Form N in Example 6, and then the solid was separated to give Crystal Form T with an XRPD pattern shown in Figure 39.

11. Crystal Form U

Crystal Form U was obtained by leaving Crystal Form Q of Compound Z open at room temperature for 1 day, which had an XRPD pattern shown in Figure 40.

**Example 8: Transformation Tests between Crystal Form A and Crystal Form P of Compound Z**

In order to confirm the mutual transformation relationship between Crystal Form A and Crystal Form P of Compound Z, suspension competition tests in different solvents at room temperature and 50°C were set up.

The suspensions of Compound Z that had been equilibrated for 2 hours under the corresponding temperature and solvent conditions were filtered to obtain saturated solutions of Compound Z under different temperature and solvent conditions, and then solid mixtures of Crystal Forms A and P of Compound Z were added. After the suspension competition was carried out for 1 day, the solids were separated for XRPD characterization.

The test results are as shown in Figure 48, Figure 49, and Table E-3. In the EtOH system (at room temperature and 50°C), Crystal Form A and Crystal Form B (the water activity test proved that Crystal Form B was a metastable crystal form, which transformed into Crystal Form A during air-drying at room temperature) of Compound Z were obtained. In the EtOAc system (at room temperature and 50°C), Crystal Form A of Compound Z was obtained. The results show that Crystal Form A of Compound Z is thermodynamically more stable at room temperature and 50°C.

Table E-3: Suspension Competition Tests

| Initial Crystal Form | Solvent | Temperature | XRPD Results |
|---|---|---|---|
| A mixture of Crystal Form A and Crystal Form P of Compound Z | EtOH | r. t. | Crystal Form A + Crystal Form B |
| | EtOAc | r. t. | Crystal Form A |
| | EtOH | 50°C | Crystal Form A + Crystal Form B |
| | EtOAc | 50°C | Crystal Form A |

**Example 9: Determination of Equilibrium Solubility of Compound Z**

The equilibrium solubility of the amorphous Compound I (in free state, Figure 41) and Crystal Form A of Compound Z in 24 hours was determined at 37°C in four media, namely $H_2O$ (purified water), SGF (simulated gastric fluid), FaSSIF (fasted-state simulated intestinal fluid), and FeSSIF (fed-state simulated intestinal fluid).

The amorphous Compound I and Crystal Form A of Compound Z each in an amount of 10 mg were separately added to 1 mL of the corresponding medium to prepare a suspension, which was magnetically stirred at 37±2°C. After 24 hours, samples were taken. The samples were centrifuged for separation, and the supernatant after filtration was used for solubility determination.

The results shown in Table E-4 indicate that, compared with the amorphous Compound I, the solubility of Crystal Form A of Compound Z in water and the simulated gastric and intestinal fluids was increased.

Table E-4: Equilibrium Solubility of Crystal Form A of Compound Z and the Amorphous Compound I

| Samples | Media | Equilibrium Solubility (mg/mL) |
|---|---|---|
| Amorphous form of Compound I | $H_2O$ | 0.43 |
| | SGF | 0.41 |
| | FaSSIF | 0.15 |
| | FeSSIF | 0.80 |
| Crystal Form A of Compound Z | $H_2O$ | 1.94 |
| | SGF | 1.00 |
| | FaSSIF | 1.19 |
| | FeSSIF | 1.17 |

**Example 10: Determination of Hygroscopicity of Crystal Form A of Compound Z**

In order to evaluate the risk of the stability of samples changing with humidity at 25°C, DVS tests were carried out on the amorphous Compound I (in free state, Figure 41) and Crystal Form A of Compound Z. Hygroscopicity is based on the weight gain of the sample when the humidity rises to 80%RH at 25°C, wherein a weight gain of 0.2%-2% indicates slightly hygroscopic, and a weight gain of 2%-15% indicates hygroscopic.

The DVS pattern of Crystal Form A of Compound Z is shown in Figure 4-2, and the DVS pattern of the free compound is shown in Figure 4-3. The results show that, at 80%RH, Compound I in the amorphous form absorbed water to a weight gain of 4.029%, indicating that it was hygroscopic; and Crystal Form A of Compound Z absorbed water to a weight gain of 1.696%, indicating that it was slightly hygroscopic.

Using the same test method, it was found that Crystal Forms P, C, V, and W of Compound Z also had favorable low hygroscopicity.

**Example 11: Determination of Solid-state Stability of Crystal Form A of Compound Z**

A crystalline form of Compound I (in free state) and Crystal Form A of Compound Z were separately placed under long-term (25°C/60%RH) and accelerated (40°C/75%RH) conditions for 10 days, and then the HPLC purity and crystal form change were determined. The free Compound I was in a crystalline form, and had the XRPD pattern indicated by the arrow in Figure 50.

The test results are summarized in Figure 50, Figure 51, and Table E-5. The results show that the purity of Crystal Form A of Compound Z had no obvious decrease after 10 days under both long-term and accelerated conditions, and the chemical stability was good; and the purity of the crystalline form of Compound I had no obvious decrease after 10 days under long-term conditions, but the purity decreased by about 2.4% after 10 days under accelerated conditions. The results indicate that the solid-state chemical stability of Crystal Form A of Compound Z was better than that of the crystalline form of Compound I. No crystal form change was observed in all samples.

Table E-5: Results of Solid-state Stability Determination

| Sample | Initial Purity (% Area) | 25°C/60%RH, 10 days | | 40°C/75%RH, 10 days | |
|---|---|---|---|---|---|
| | | Crystal form change | Purity (% Area) | Crystal form change | Purity (% Area) |
| Crystalline form of Compound I | 97.31 | NO | 97.33 | NO | 94.95 |
| Crystal Form A of Compound Z | 97.41 | NO | 97.38 | NO | 97.35 |

Using the same test method, it was found that Crystal Form V of Compound Z also had good solid-state stability.

**Example 12: High-temperature Stability Test of Crystal Form A of Compound Z**

A crystalline form of Compound I (in free state, Figure 50) and Crystal Form A of Compound Z were separately placed under the condition of 60°C, and then the HPLC purity (% area) and crystal form change were tested. The results show that the purity of Crystal Form A of Compound Z had no obvious decrease after 1 day (initial purity 97.41% vs. 97.34%

after 1 day); and the purity of the crystalline form of Compound I decreased by about 0.8% after 1 day (initial purity 97.31% vs. 96.56% after 1 day). Both had a similar decrease in purity after 10 days. No crystal form change was observed in all samples.

Using the same test method, it was found that Crystal Forms P, C, V, and W of Compound Z also had good high-temperature stability.

**Example 13: Determination of High-humidity Stability of Crystal Form A of Compound Z**

A crystalline form of Compound I (in free state, Figure 50) and Crystal Form A of Compound Z were separately placed under high-humidity (90%RH) conditions for 10 days, and then the HPLC purity (% area) and crystal form change were tested. The initial purities of Crystal Form A of Compound Z and the crystalline form of Compound I were 97.41% and 97.31%, respectively. The results show that the purity of Crystal Form A of Compound Z had no obvious decrease; and the purity of the crystalline form of Compound I decreased by about 0.3% (97.06% after 10 days). No crystal form change was observed in all samples.

Using the same test method, it was found that Crystal Forms P, C, V, and W of Compound Z also had good stability under high-humidity conditions.

**Example 14: Determination of Pressure Stability of Crystal Form A of Compound Z**

A crystalline form of Compound I (in free state, Figure 50) and Crystal Form A of Compound Z were separately pressed under a pressure of 1000 Mpa for 5 minutes, and then the HPLC purity (% area) and crystal form change were tested. The initial purities of Crystal Form A of Compound Z and the crystalline form of Compound I were 97.31% and 97.13%, respectively. The results show that the purity of Crystal Form A of Compound Z had no obvious decrease; and the purity of the crystalline form of Compound I decreased by about 0.5% (96.60% at the end of the test). No crystal form change was observed in all samples.

Using the same test method, it was found that Crystal Forms P, C, V, and W of Compound Z also had good pressure stability.

**Example 15: Determination of Dynamic Solubility of Crystal Form A of Compound Z**

The dynamic solubility of the free Compound I and Crystal Form A of Compound Z in water/simulated gastric and intestinal fluids were tested. In the tests, the fed solid amount was about 10 mg/mL of the free Compound I or Crystal Form A of Compound Z in each solvent system, and was mixed at 37°C±2°C. The suspension was sampled at 1, 2, 4, and 24 hours, and the supernatant was taken after filtration for concentration determination, and the solid was taken for XRPD testing. The results are shown in Table E-6.

Table E-6: Dynamic Solubility Test Data

| Solid state | Solvent | Sampling time (h) | Dynamic solubility in water (mg/mL) | XRPD of Residual Solid | Whether the crystal form changed |
|---|---|---|---|---|---|
| Free Compound I | $H_2O$ (water) | 1 | 0.05 | Crystal Form A of Compound I | YES |
| | | 2 | 0.05 | Crystal Form A of Compound I | YES |
| | | 4 | 0.03 | Crystal Form A of Compound I | YES |
| | | 24 | 0.03 | Crystal Form A of Compound I | YES |
| | FaSSGF | 1 | 0.10 | Crystal Form A of the hydrochloride of Compound I | YES |
| | | 2 | 0.09 | Crystal Form A of the hydrochloride of Compound I | YES |
| | | 4 | 0.11 | Crystal Form A of the hydrochloride of Compound I | YES |
| | | 24 | 0.10 | Crystal Form A of the hydrochloride of Compound I | YES |
| | FaSSIF | 1 | 0.04 | Crystal Form A of Compound I | YES |
| | | 2 | 0.07 | Crystal Form A of Compound I | YES |
| | | 4 | 0.12 | Crystal Form A of Compound I | YES |
| | | 24 | 0.21 | Crystal Form A of Compound I | YES |

(continued)

| Solid state | Solvent | Sampling time (h) | Dynamic solubility in water (mg/mL) | XRPD of Residual Solid | Whether the crystal form changed |
|---|---|---|---|---|---|
| Crystal Form A of Compound Z | $H_2O$ | 1 | 5.50 | N/A | / |
| | | 2 | 1.38 | Crystal Form A of Compound I | YES |
| | | 4 | 1.23 | Crystal Form A of Compound I | YES |
| | | 24 | 1.33 | Crystal Form A of Compound I | YES |
| | FaSSGF | 1 | 0.04 | Amorphous | YES |
| | | 2 | 0.14 | Amorphous | YES |
| | | 4 | 0.12 | Crystal Form A of the hydrochloride of Compound I | YES |
| | | 24 | 0.06 | Crystal Form A of the hydrochloride of Compound I | YES |
| | FaSSIF | 1 | 0.13 | Crystal Form A of Compound I | YES |
| | | 2 | 0.13 | Crystal Form A of Compound I | YES |
| | | 4 | 0.11 | Crystal Form A of Compound I | YES |
| | | 24 | 0.28 | Crystal Form A of Compound I | YES |
| Note: "N/A" indicates that the residual solid was insufficient for detection. | | | | | |

The results show that within 24 hours, the solubility of Crystal Form A of Compound Z in water was significantly higher than that of the free Compound I. In FaSSGF (fasted-state simulated gastric fluid), at 1 and 2 hours (gastric emptying time), the solubility of Crystal Form A of Compound Z was significantly higher than that of the free Compound I. In FaSSIF (fasted-state simulated intestinal fluid), at 1 and 2 hours, the solubility of Crystal Form A of Compound Z was also significantly higher than that of the free Compound I, indicating that Crystal Form A of Compound Z may have a faster onset of action than the free Compound I.

Examples herein are used to illustrate the principles and implementation methods of the present invention. The Examples above are merely for helping understand the present invention without any limitation thereto. Of noted is that for those of ordinary skill in the art, several improvements and modifications can be made to the present invention without departing from the principle of the present invention, and these improvements and modifications also fall within the scope of the claims of the present invention.

## Claims

1. A pharmaceutically acceptable salt of Compound I ((S)-2-((4-(6-((2-fluoro-4-(oxetan-3-yl)benzyl)oxy)pyridin-2-yl) piperidin-1-yl)methyl)-1-(oxetan-2-ylmethyl)-1H-benzo[d]imidazole-6-carboxylic acid), wherein the salt includes a mesylate salt, a meglumine salt, and a tromethamine salt, preferably Compound Z ((S)-2-((4-(6-((2-fluoro-4-(oxetan-3-yl)benzyl)oxy)pyridin-2-yl)piperidin-1-yl)methyl)-1-(oxetan-2-ylmethyl)-1H-benzo[d]imidazole-6-carboxylic acid • tromethamine salt).

2. The pharmaceutically acceptable salt of Compound I according to claim 1, which is in a crystalline form, preferably, the salt is a crystalline form of the mesylate salt, or a crystalline form of the meglumine salt, or a crystalline form of Compound Z.

**3.** The pharmaceutically acceptable salt of Compound I according to claim 2, wherein the salt is a crystalline form of Compound Z, and wherein the crystalline form is Crystal Form A, and

the X-ray powder diffraction (XRPD) pattern of the Crystal Form A includes diffraction peaks at the following diffraction angles (2θ): about 3.62±0.20°, 7.28±0.20°, 17.21±0.20° and 20.60±0.20°; preferably further includes diffraction peaks at any one, more, or all of the following diffraction angles (2θ): about 14.66±0.20°, 15.55±0.20°, 16.66±0.20°, 21.94±0.20° and 26.04±0.20°; and/or
the differential scanning calorimetry (DSC) pattern of the Crystal Form A has one endothermic peak with a peak value at about 159.9°C±3.0°C; and/or
the Crystal Form A has a weight loss of about 0.815% during the process of heating to about 120°C±3°C, as measured by thermogravimetric analysis (TGA); and/or
the $^1$H NMR spectrum of the Crystal Form A is substantially as shown in Figure 4; and/or
more preferably, the XRPD pattern of the Crystal Form A further includes diffraction peaks at any one, more, or all of the following diffraction angles (2θ): about 7.74±0.20°, 8.54±0.20°, 10.92±0.20°, 15.28±0.20°, 17.73±0.20°, 18.05±0.20°, 18.44±0.20°, 19.47±0.20°, 19.90±0.20°, 22.38±0.20°, 23.18±0.20°, 25.04±0.20°, 27.28 ±0.20°, 27.82±0.20°, 28.10±0.20°, 29.52±0.20°, 31.06±0.20°, 32.97±0.20°, 33.36±0.20° and 40.57 ±0.20°; or
the X-ray powder diffraction pattern of the Crystal Form A includes diffraction peaks at the following diffraction angles (2θ): 3.623°, 7.281°, 7.742°, 8.537°, 10.916°, 14.655°, 15.279°, 15.553°, 16.660°, 17.207°, 17.732°, 18.047°, 18.444°, 19.472°, 19.896°, 20.604°, 21.935°, 22.382°, 23.183°, 25.043°, 26.041°, 27.280°, 27.820°, 28.097°, 29.520°, 31.063°, 32.971°, 33.357°, 40.566°; and/or
the DSC pattern of the Crystal Form A is substantially as shown in Figure 2; and/or
the TGA pattern of the Crystal Form A is substantially as shown in Figure 3;
more preferably, the XRPD pattern of the Crystal Form A includes diffraction peaks at diffraction angles (2θ) substantially the same as those shown in Figure 1, and further preferably, the XRPD pattern of the Crystal Form A is as shown in Figure 1;
even more preferably, the Crystal Form A is not a solvate, and more preferably is an anhydride.

**4.** The pharmaceutically acceptable salt of Compound I according to claim 2, wherein the salt is a crystalline form of Compound Z, and wherein the crystalline form is Crystal Form P, and

the XRPD pattern of the Crystal Form P includes diffraction peaks at the following diffraction angles (2θ): about 3.55±0.20°, 7.31±0.20°, 14.77±0.20°, 17.01±0.20° and 20.44±0.20°; and/or
the DSC pattern of the Crystal Form P has two endothermic peaks with peak values at about 152.5°C±3.0°C and about 158.1°C±3.0°C, respectively; and/or
the Crystal Form P has a weight loss of about 0.367% during the process of heating to about 140°C±3°C, as measured by TGA; and/or
the $^1$H NMR spectrum of the Crystal Form P is substantially as shown in Figure 7-2; and/or
preferably, the XRPD pattern of the Crystal Form P further includes diffraction peaks at any one, more, or all of the following diffraction angles (2θ): about 15.50±0.20°, 18.01±0.20°, 19.06±0.20°, 19.57±0.20°, 22.07±0.20° and 25.23±0.20°; or
the XRPD pattern of the Crystal Form P includes diffraction peaks at the following diffraction angles (2θ): 3.547°, 7.306°, 14.765°, 15.501°, 17.009°, 18.005°, 19.058°, 19.571°, 20.436°, 22.071°, 22.441° and 25.229°; and/or
the DSC pattern of the Crystal Form P is substantially as shown in Figure 6; and/or
the TGA pattern of the Crystal Form P is substantially as shown in Figure 7-1; and/or
more preferably, the XRPD pattern of the Crystal Form P includes diffraction peaks at diffraction angles (2θ) substantially the same as those shown in Figure 5, and further more preferably, the XRPD pattern of the Crystal Form P is as shown in Figure 5;
even more preferably, the Crystal Form P is not a solvate, and more preferably is an anhydride.

**5.** The pharmaceutically acceptable salt of Compound I according to claim 2, wherein the salt is a crystalline form of Compound Z, and wherein the crystalline form is Crystal Form C, and

the XRPD pattern of the Crystal Form C includes diffraction peaks at the following diffraction angles (2θ): about 3.88±0.20°, 7.81±0.20°, 15.60±0.20°, 17.49±0.20° and 19.64±0.20°; and/or
the DSC pattern of the Crystal Form C has three endothermic peaks with peak values at about 81.8°C±3.0°C, about 93.6°C±3.0°C and about 155.2°C±3.0°C, respectively; and/or
the Crystal Form C has a weight loss of about 4.560% during the process of heating to about 100°C±3°C, as

measured by TGA; and/or

the $^{1}$H NMR spectrum of the Crystal Form C is substantially as shown in Figure 10-2; and/or

preferably, the XRPD pattern of the Crystal Form C further includes diffraction peaks at any one, more, or all of the following diffraction angles (2θ): about 14.86±0.20°, 19.99±0.20°, 20.24±0.20°, 22.37±0.20°, 24.21±0.20° and 24.51±0.20°;

more preferably, the XRPD pattern of the Crystal Form C further includes diffraction peaks at any one, more, or all of the following diffraction angles (2θ): about 10.44±0.20°, 13.13±0.20°, 16.09±0.20°, 19.39±0.20°, 23.57 ±0.20°, 26.82±0.20°, 27.18±0.20° and 33.37±0.20°;

even more preferably, the XRPD pattern of the Crystal Form C further includes diffraction peaks at any one, more, or all of the following diffraction angles (2θ): about 6.50±0.20°, 7.42±0.20°, 9.90±0.20°, 11.74±0.20°, 13.75 ±0.20°, 18.15±0.20°, 18.49±0.20°, 20.65±0.20°, 21.02±0.20°, 21.25±0.20°, 21.58±0.20°, 21.82±0.20°, 22.86±0.20°, 23.15±0.20°, 24.97±0.20°, 25.35±0.20°, 26.05±0.20°, 26.40±0.20°, 27.41±0.20°, 28.17 ±0.20°, 28.79±0.20°, 30.19±0.20°, 30.63±0.20°, 31.614±0.20°, 31.87±0.20°, 35.09±0.20° and 35.30 ±0.20°; or

preferably, the XRPD pattern of the Crystal Form C includes diffraction peaks at the following diffraction angles (2θ): 3.877°, 6.503°, 7.415°, 7.807°, 9.896°, 10.442°, 11.742°, 13.126°, 13.75°, 14.861°, 15.603°, 16.091°, 17.494°, 18.154°, 18.487°, 19.386°, 19.635°, 19.985°, 20.242°, 20.654°, 21.021°, 21.253°, 21.583°, 21.819°, 22.365°, 22.856°, 23.153°, 23.570°, 24.214°, 24.506°, 24.970°, 25.345°, 26.048°, 26.396°, 26.824°, 27.178°, 27.413°, 28.172°, 28.793°, 30.193°, 30.627°, 31.614°, 31.868°, 33.370°, 35.086° and 35.303°; and/or

the DSC pattern of the Crystal Form C has one exothermic peak with a peak value at about 125.0±3.0°C;

preferably, the DSC pattern of the Crystal Form C is substantially as shown in Figure 9; and/or

preferably, the TGA pattern of the Crystal Form C is substantially as shown in Figure 10-1; and/or

more preferably, the XRPD pattern of the Crystal Form C includes diffraction peaks at diffraction angles (2θ) substantially the same as those shown in Figure 8, and further preferably, the XRPD pattern of the Crystal Form C is as shown in Figure 8;

even more preferably, the Crystal Form C is a hydrate.

**6.** The pharmaceutically acceptable salt of Compound I according to claim 2, wherein the salt is a crystalline form of Compound Z, and wherein the crystalline form is Crystal Form V, and

the XRPD pattern of the Crystal Form V includes diffraction peaks at the following diffraction angles (2θ): about 4.46±0.20°, 8.98±0.20°, 12.88±0.20°, 18.04±0.20° and 19.26±0.20°; and/or

the DSC pattern of the Crystal Form V has two endothermic peaks with peak values at about 64.5°C±3.0°C and about 164.8°C±3.0°C, respectively; and/or

the Crystal Form V has a weight loss of about 2.987% during the process of heating to about 90°C±3°C, as measured by TGA; and/or

the $^{1}$H NMR spectrum of the Crystal Form V is substantially as shown in Figure 45-4; and/or

preferably, the XRPD pattern of the Crystal Form V further includes diffraction peaks at any one, more, or all of the following diffraction angles (2θ): about 16.68±0.20°, 18.39±0.20°, 20.75±0.20°, 23.84±0.20° and 25.42 ±0.20°;

more preferably, the XRPD pattern of the Crystal Form V further includes diffraction peaks at any one, more, or all of the following diffraction angles (2θ): about 9.16±0.20°, 9.39±0.20°, 10.31±0.20°, 12.70±0.20°, 12.99±0.20°, 14.36±0.20°, 14.70±0.20°, 19.46±0.20°, 19.62±0.20°, 21.29±0.20°, 22.03±0.20°, 22.69±0.20°, 22.93 ±0.20°, 24.23±0.20°, 24.66±0.20° and 27.00±0.20°;

even more preferably, the XRPD pattern of the Crystal Form V further includes diffraction peaks at any one, more, or all of the following diffraction angles (2θ): about 11.89±0.20°, 13.50±0.20°, 16.17±0.20°, 17.71±0.20°, 18.88 ±0.20°, 19.93±0.20°, 20.20±0.20°, 23.20±0.20°, 25.98±0.20°, 27.23±0.20°, 28.02±0.20°, 28.40±0.20°, 28.68±0.20°, 29.01±0.20°, 29.81±0.20°, 30.25±0.20°, 31.52±0.20°, 31.87±0.20°, 32.63±0.20°, 33.74 ±0.20°, 36.47±0.20°, 37.48±0.20° and 7.96±0.20°; and/or

preferably, the DSC pattern of the Crystal Form V is substantially as shown in Figure 45-2; and/or

preferably, the TGA pattern of the Crystal Form V is substantially as shown in Figure 45-3; and/or

more preferably, the XRPD pattern of the Crystal Form V includes diffraction peaks at diffraction angles (2θ) substantially the same as those shown in Figure 45-1, and further more preferably, the XRPD pattern of the Crystal Form V is as shown in Figure 45-1;

even more preferably, the Crystal Form V is a hydrate.

**7.** The pharmaceutically acceptable salt of Compound I according to claim 2, wherein the salt is a crystalline form of Compound Z, and wherein the crystalline form is Crystal Form W, and

the XRPD pattern of the Crystal Form W includes diffraction peaks at the following diffraction angles (2θ): about 4.62 ±0.20°, 12.90 ±0.20°, 18.06 ±0.20°, 19.40 ±0.20° and 19.60±0.20°; and/or
the Crystal Form W has a weight loss of about 1.111% during the process of heating to about 90°C±3°C, as measured by TGA; and/or
preferably, the XRPD pattern of the Crystal Form W further includes diffraction peaks at any one, more, or all of the following diffraction angles (2θ): about 14.86 ±0.20°, 16.74 ±0.20°, 18.41 ±0.20°, 18.68 ±0.20°, 21.63 ±0.20°, 22.03 ±0.20°, 22.60 ±0.20°, 22.99 ±0.20° and 25.34±0.20°;
more preferably, the XRPD pattern of the Crystal Form W further includes diffraction peaks at any one, more, or all of the following diffraction angles (2θ): about 9.14 ±0.20°, 9.37 ±0.20°, 10.21 ±0.20°, 15.35 ±0.20°, 20.52 ±0.20°, 20.77 ±0.20°, 23.88 ±0.20°, 24.27 ±0.20°, 24.66 ±0.20°, 27.04 ±0.20° and 28.01±0.20°;
even more preferably, the XRPD pattern of the Crystal Form W further includes diffraction peaks at any one, more, or all of the following diffraction angles (2θ): about 11.50 ±0.20°, 12.02 ±0.20°, 13.93 ±0.20°, 14.40 ±0.20°, 23.44 ±0.20°, 25.81 ±0.20°, 28.46 ±0.20°, 30.33 ±0.20°, 31.60 ±0.20° and 37.34±0.20°;
preferably, the TGA pattern of the Crystal Form W is substantially as shown in Figure 47; and/or
more preferably, the XRPD pattern of the Crystal Form W includes diffraction peaks at diffraction angles (2θ) substantially the same as those shown in Figure 46, and further more preferably, the XRPD pattern of the Crystal Form W is as shown in Figure 46;
even more preferably, the Crystal Form W is not a solvate, and more preferably is an anhydride.

**8.** A method for preparing the pharmaceutically acceptable salt of Compound I according to claim 1, wherein:

the method comprises reacting the Compound I with a reagent selected from methanesulfonic acid, meglumine, and tromethamine in a solvent to obtain the corresponding mesylate salt, meglumine salt, or tromethamine salt of Compound I;
or
the method comprises reacting the Compound I with tromethamine in a solvent (preferably at room temperature) to obtain the pharmaceutically acceptable salt of Compound I, wherein the salt is the Compound Z according to claim 1;
or
the method comprises stirring the Compound I and tromethamine in a solvent at room temperature to obtain a solid precipitate, and the precipitated solid is the Compound Z, preferably the Crystal Form A according to claim 3; wherein:

the solvent is one or a mixture of two or more selected from isopropyl alcohol, methyl isobutyl ketone, acetonitrile, methyl tert-butyl ether, tetrahydrofuran, ethanol, methanol, and water, and
preferably, the molar ratio of the Compound I to the tromethamine is about 2:1 to about 1:2;
preferably, the stirring at room temperature is carried out for an appropriate period of time, for example, until the salt formation is complete, or for example, for about 3 days;
or
the method comprises suspending the Compound Z according to claim 1, especially the Crystal Form A according to claim 3 in a mixture of $CHCl_3$:EtOAc (for example, at about 1:1, v:v), and stirring the resulting suspension at an appropriate temperature to obtain a solid precipitate, and the precipitated solid is the Crystal Form P according to claim 4, wherein: preferably, the appropriate temperature is about 1-8°C, about 3-8°C, about 4-8°C, or about 5-8°C, preferably about 5°C; and/or preferably, the stirring is carried out for an appropriate period of time, for example, about 3 days;
or
the method comprises suspending the Compound Z according to claim 1, especially the Crystal Form A according to claim 3 in a mixed solvent of 1,4-dioxane and $H_2O$, and stirring the resulting suspension at room temperature to obtain a solid precipitate, and the precipitated solid is the Crystal Form C according to claim 5, wherein: preferably, in the mixed solvent, the volume ratio of 1,4-dioxane:$H_2O$ is about (3-10):1, about (4-8):1, about (5-7):1, about (5.5-6.5):1, or about 6:1; and/or preferably, the stirring can be carried out for an appropriate period of time, for example, about 3 days;
or
the method comprises:

(1) providing a solution of the Compound Z according to claim 1 in acetonitrile:water (for example, at about 6:1, v:v) at an elevated temperature;
(2) transferring the solution to a lower temperature and stirring; and

(3) filtering, and drying the resulting product in a hot air stream to obtain the Crystal Form V according to claim 6;

wherein: preferably, the temperature of the solution in step (1) is about 45-70°C, for example, about 50-65°C or about 50-60°C, preferably about 55°C; and/or preferably, the lower temperature in step (2) is about 20-30°C, for example, about 25°C; and/or preferably, the stirring in step (2) is carried out for an appropriate period of time, for example, about 3h;
and/or preferably, the temperature of the hot air stream in step (3) is about 40-70°C, for example, about 45-65°C or about 50-60°C, preferably about 50°C;
or
the method comprises heating the Crystal Form V according to claim 6 at about 100°C for an appropriate period of time (for example, about 10 minutes) under the protection of an inert gas (e.g., nitrogen) to obtain the Crystal Form W according to claim 7.

**9.** A pharmaceutical composition comprising the pharmaceutically acceptable salt of Compound I of any one of claims 1 to 7, and a pharmaceutically acceptable carrier,
wherein: the pharmaceutically acceptable salt is preferably the Compound Z according to claim 1, more preferably the crystalline form of Compound Z according to claim 2, and even more preferably the Crystal Form A according to claim 3, the Crystal Form P according to claim 4, the Crystal Form C according to claim 5, the Crystal Form V according to claim 6, or the Crystal Form W according to claim 7.

**10.** The pharmaceutically acceptable salt of Compound I according to any one of claims 1 to 7, or the pharmaceutical composition according to claim 9, for use in the treatment and/or prevention of GLP-1 receptor-mediated diseases and related disorders,
wherein: the pharmaceutically acceptable salt is preferably the Compound Z according to claim 1, more preferably the crystalline form of Compound Z according to claim 2, and even more preferably the Crystal Form A according to claim 3, the Crystal Form P according to claim 4, the Crystal Form C according to claim 5, the Crystal Form V according to claim 6, or the Crystal Form W according to claim 7.

**11.** Use of the pharmaceutically acceptable salt of Compound I according to any one of claims 1 to 5, or the pharmaceutical composition according to claim 9 in the manufacture of a medicament for the treatment and/or prevention of GLP-1 receptor-mediated diseases or related disorders,
wherein: the pharmaceutically acceptable salt is preferably the Compound Z according to claim 1, more preferably the crystalline form of Compound Z according to claim 2, and even more preferably the Crystal Form A according to claim 3, the Crystal Form P according to claim 4, the Crystal Form C according to claim 5, the Crystal Form V according to claim 6, or the Crystal Form W according to claim 7.

**12.** A method for treating and/or preventing GLP-1 receptor-mediated diseases or related disorders, comprising administering an effective amount of the pharmaceutically acceptable salt of Compound I according to any one of claims 1 to 7, or the pharmaceutical composition according to claim 9 to a subject in need thereof,
wherein: the pharmaceutically acceptable salt is preferably the Compound Z according to claim 1, more preferably the crystalline form of Compound Z according to claim 2, and even more preferably the Crystal Form A according to claim 3, the Crystal Form P according to claim 4, the Crystal Form C according to claim 5, the Crystal Form V according to claim 6, or the Crystal Form W according to claim 7.

**13.** The pharmaceutically acceptable salt of Compound I or the pharmaceutical composition according to claim 10, the use according to claim 11, or the method according to claim 12, wherein the GLP-1 receptor mediated diseases or related disorders are selected from diabetes, hyperglycemia, insulin resistance, impaired glucose tolerance, diabetic nephropathy, diabetic neuropathy, diabetic retinopathy, adipocyte dysfunction, obesity, non-alcoholic fatty liver disease, dyslipidemia, and hyperinsulinemia;
wherein the diabetes is preferably selected from T1D and/or T2DM, idiopathic T1D, early-onset T2D, latent autoimmune diabetes, atypical diabetes in adolescents, and gestational diabetes.

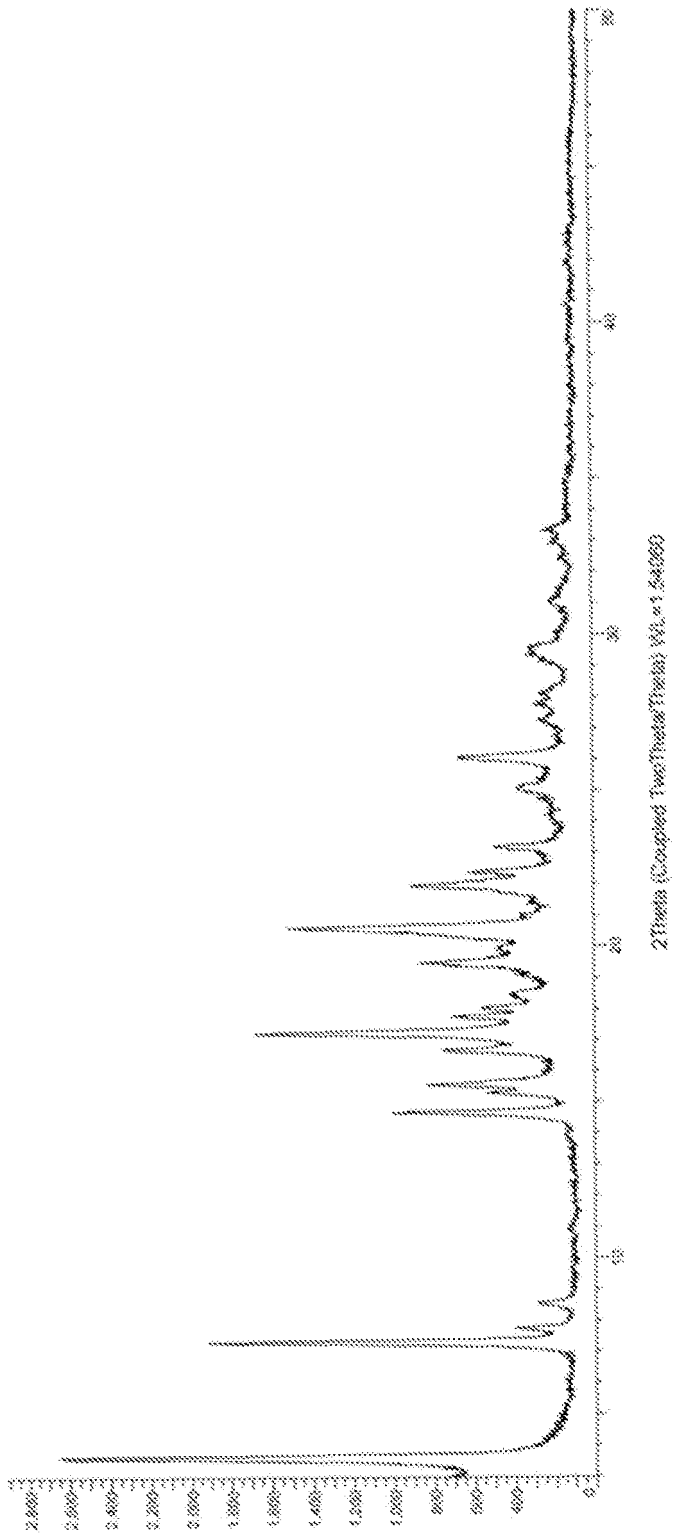

Fig. 1

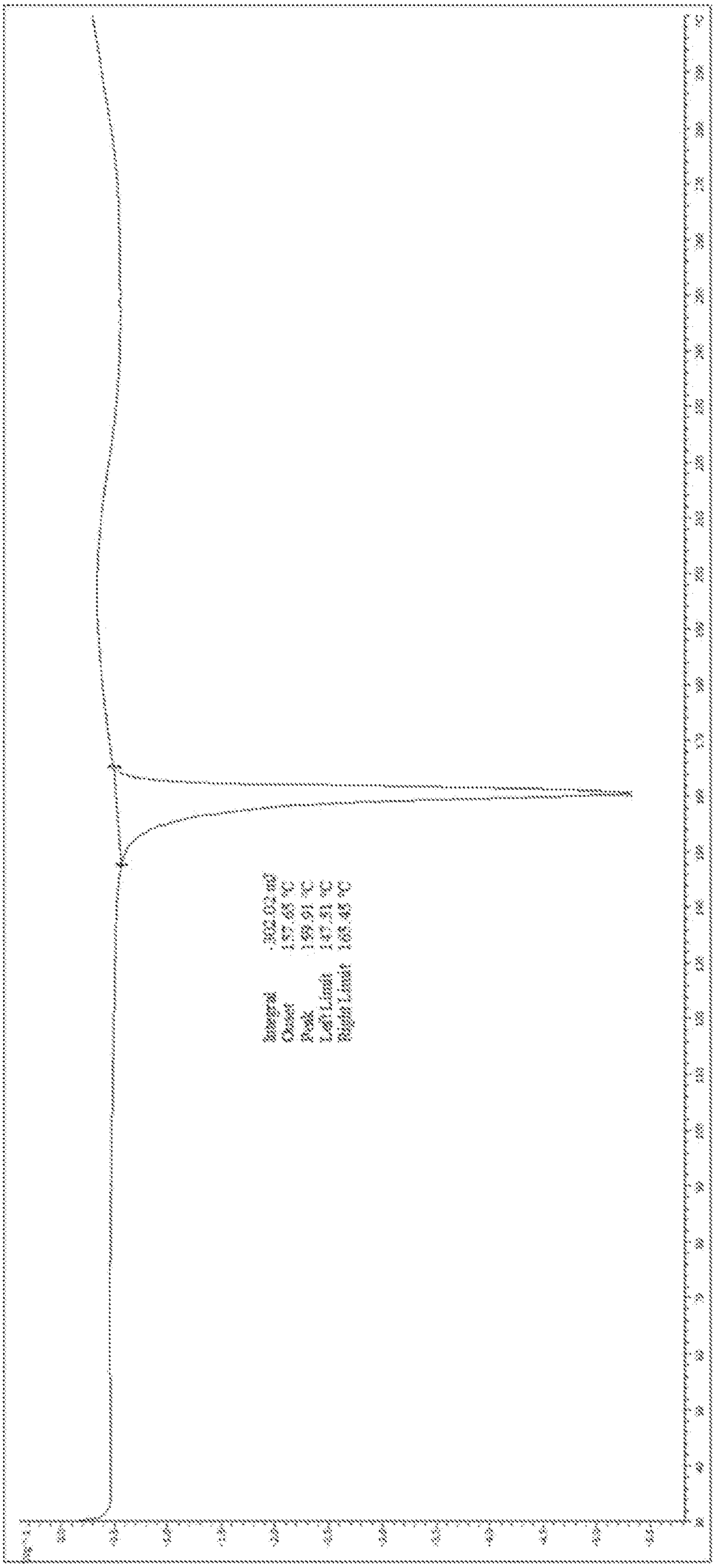

Fig. 2

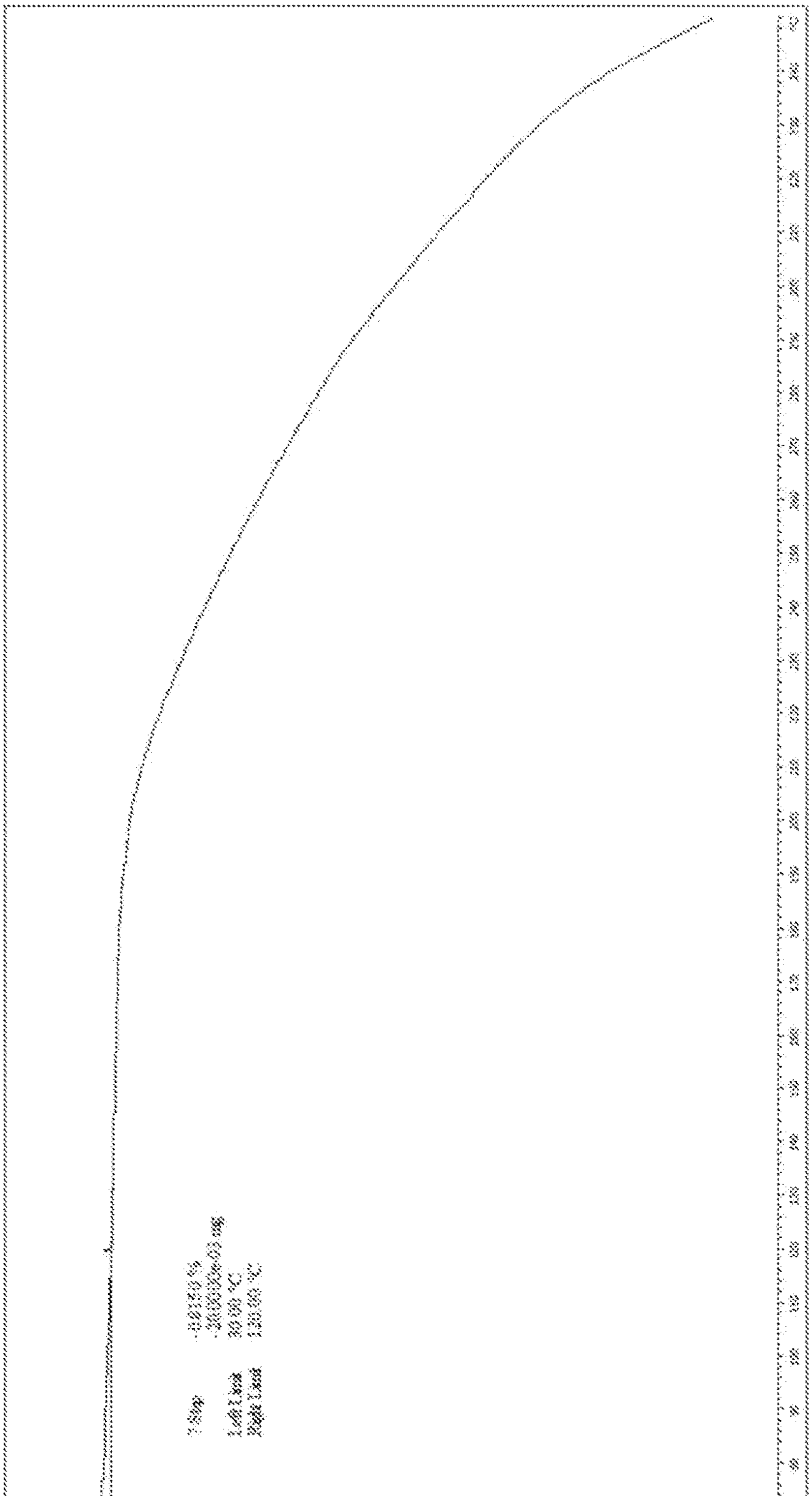

Fig. 3

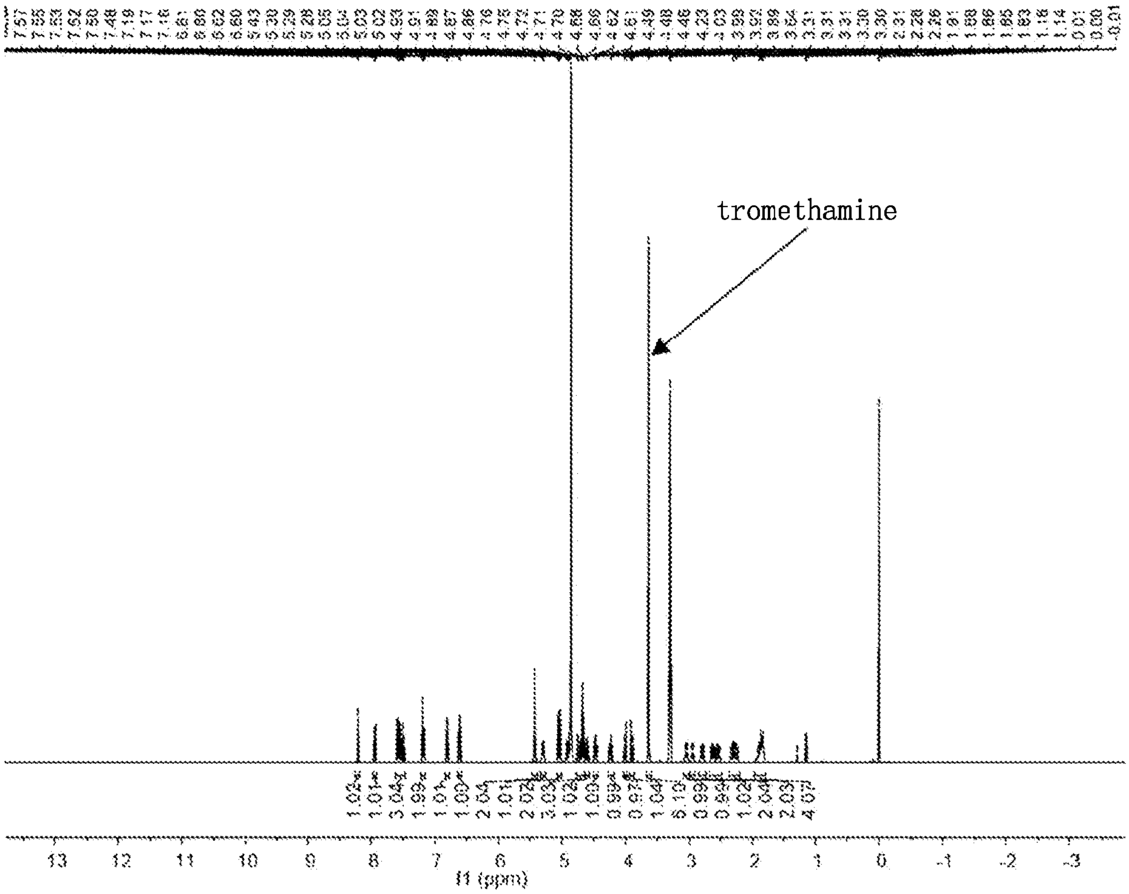
tromethamine
f1 (ppm)

Fig. 4-1

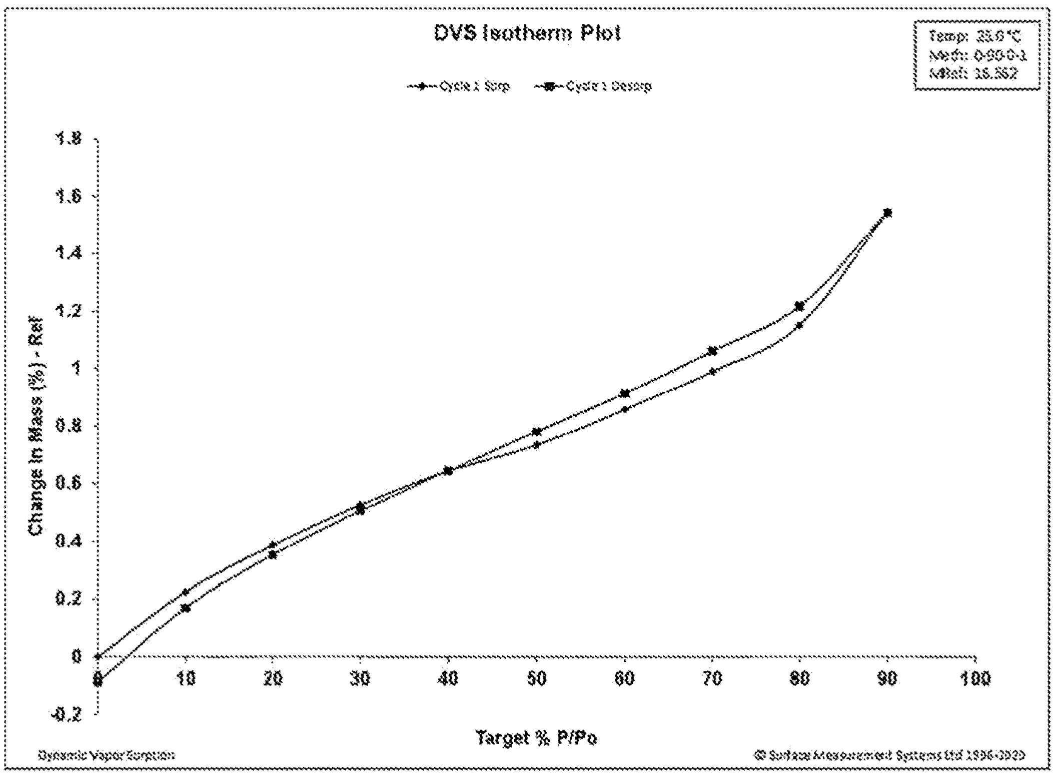
DVS Isotherm Plot
Change In Mass (%) - Ref
Target % P/Po
Dynamic Vapor Sorption

Fig. 4-2

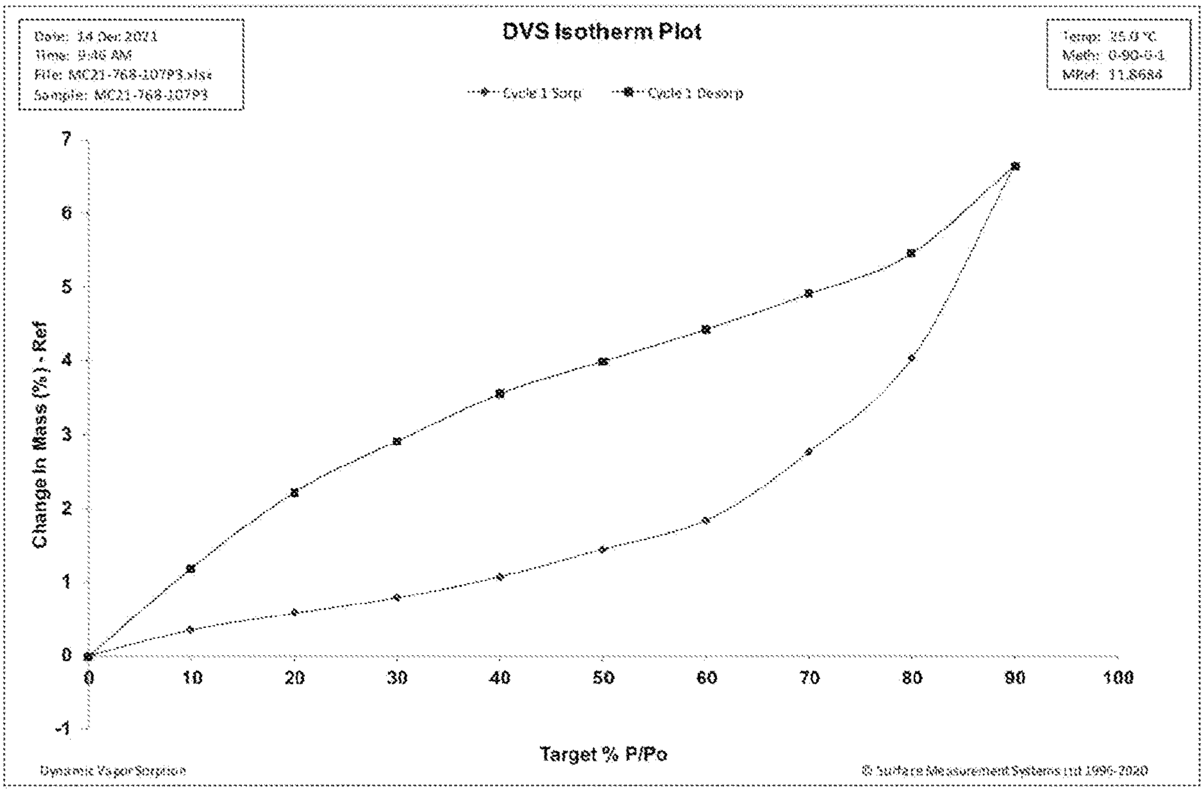
DVS Isotherm Plot
Date: 14 Dec 2021
Time: 9:46 AM
File: MC21-768-107P3.xlsx
Sample: MC21-768-107P3
Temp: 25.0 °C
Meth: 0-90-0-1
MRef: 11.8684
Cycle 1 Sorp
Cycle 1 Desorp
Change In Mass (%) - Ref
Target % P/Po
Dynamic Vapor Sorption
© Surface Measurement Systems Ltd 1996-2020

Fig. 4-3

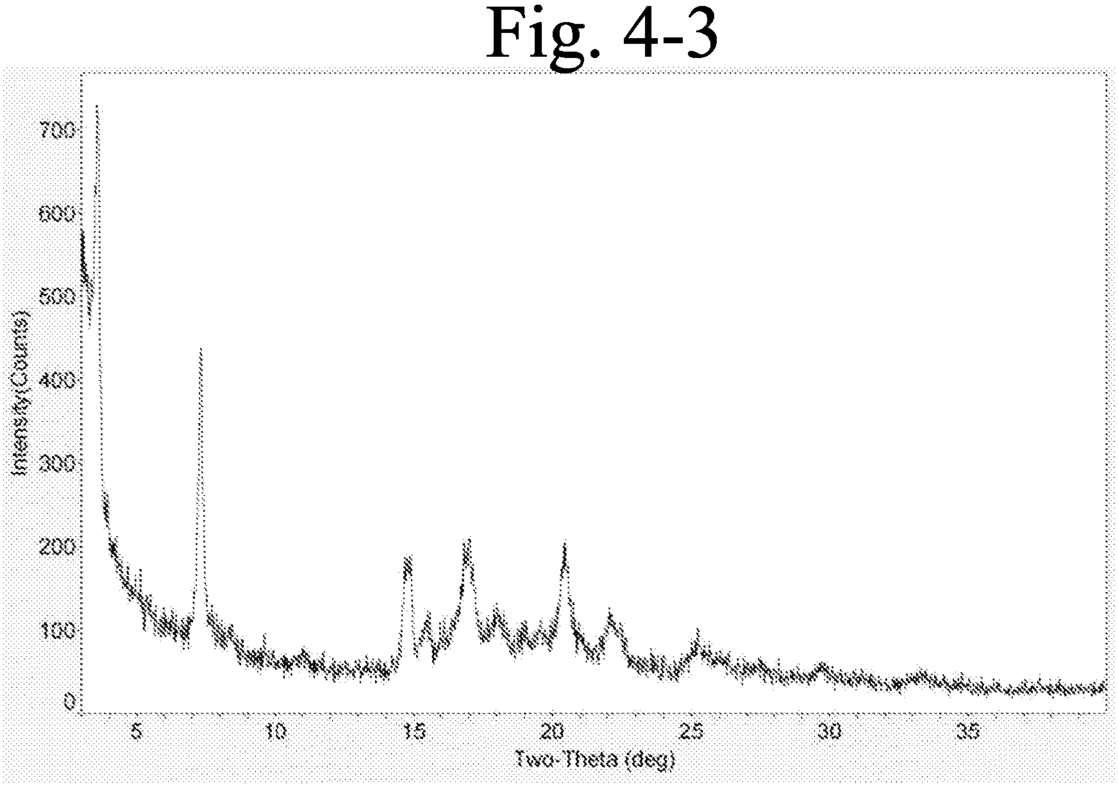
Intensity(Counts)
Two-Theta (deg)

Fig. 5

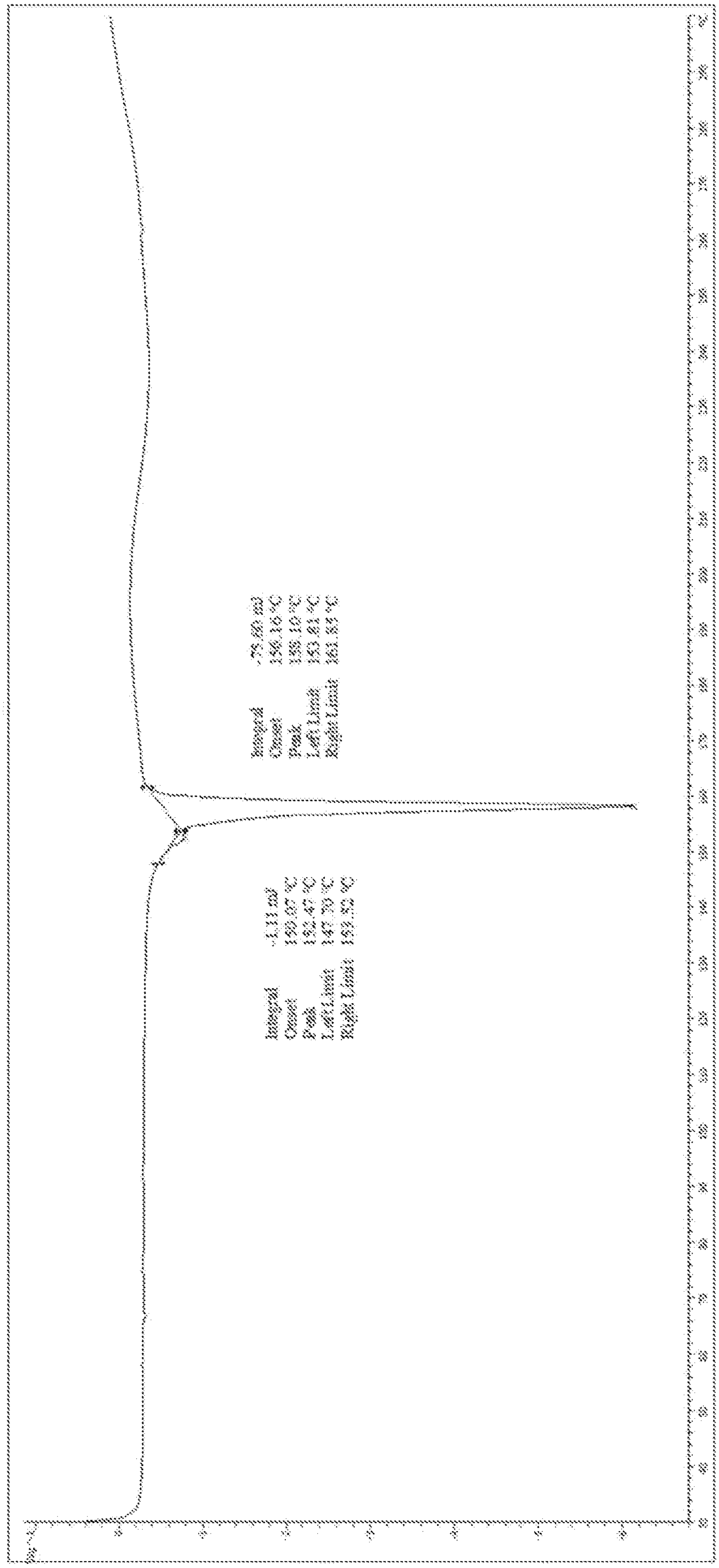

Fig. 6

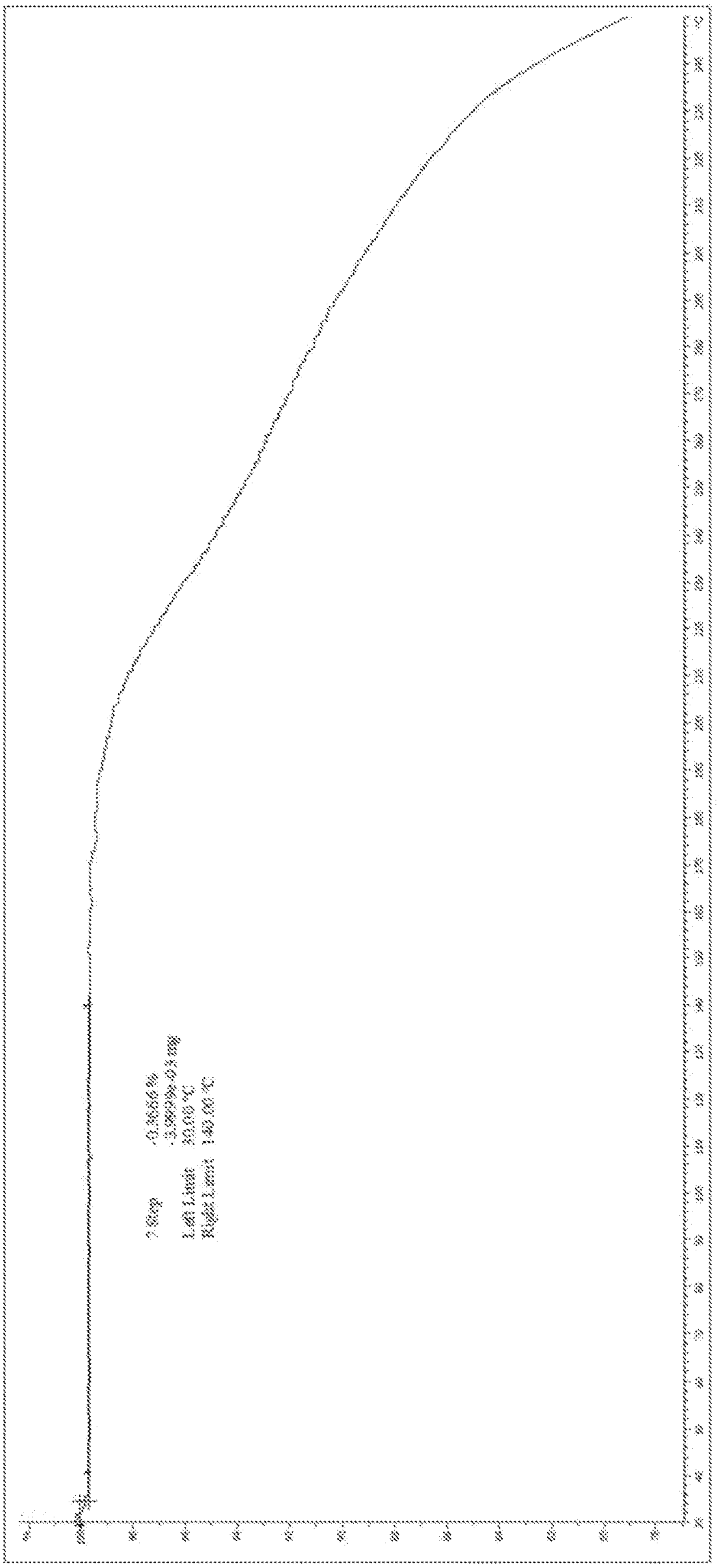

Fig. 7-1

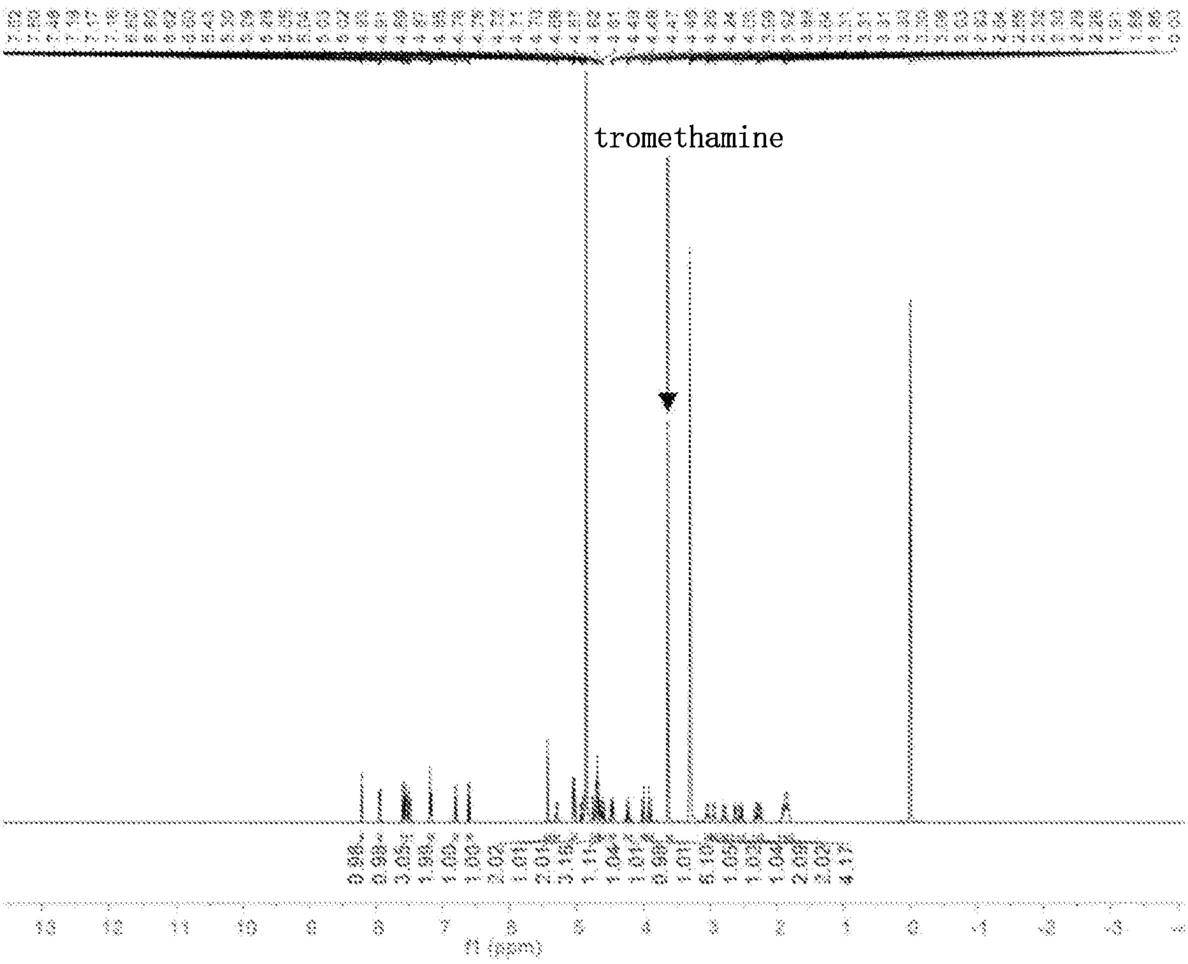


Fig. 7-2

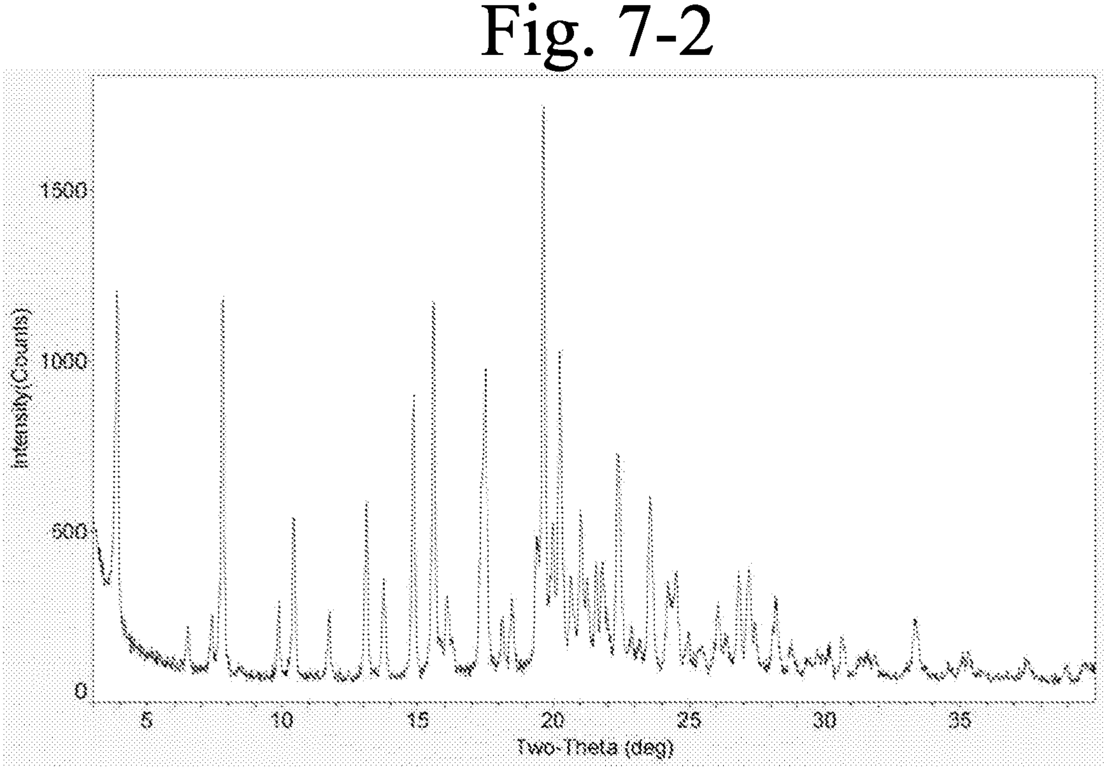


Fig. 8

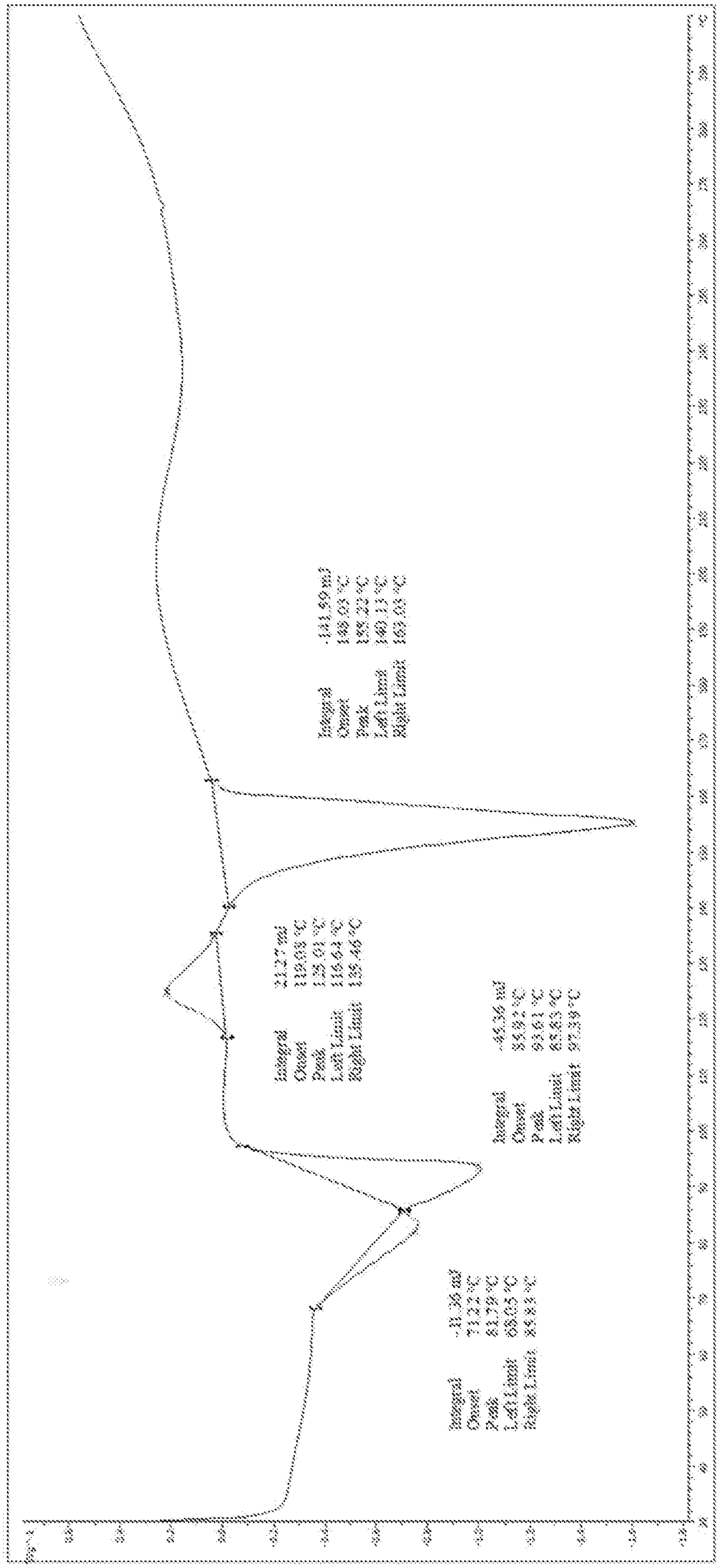

Fig. 9

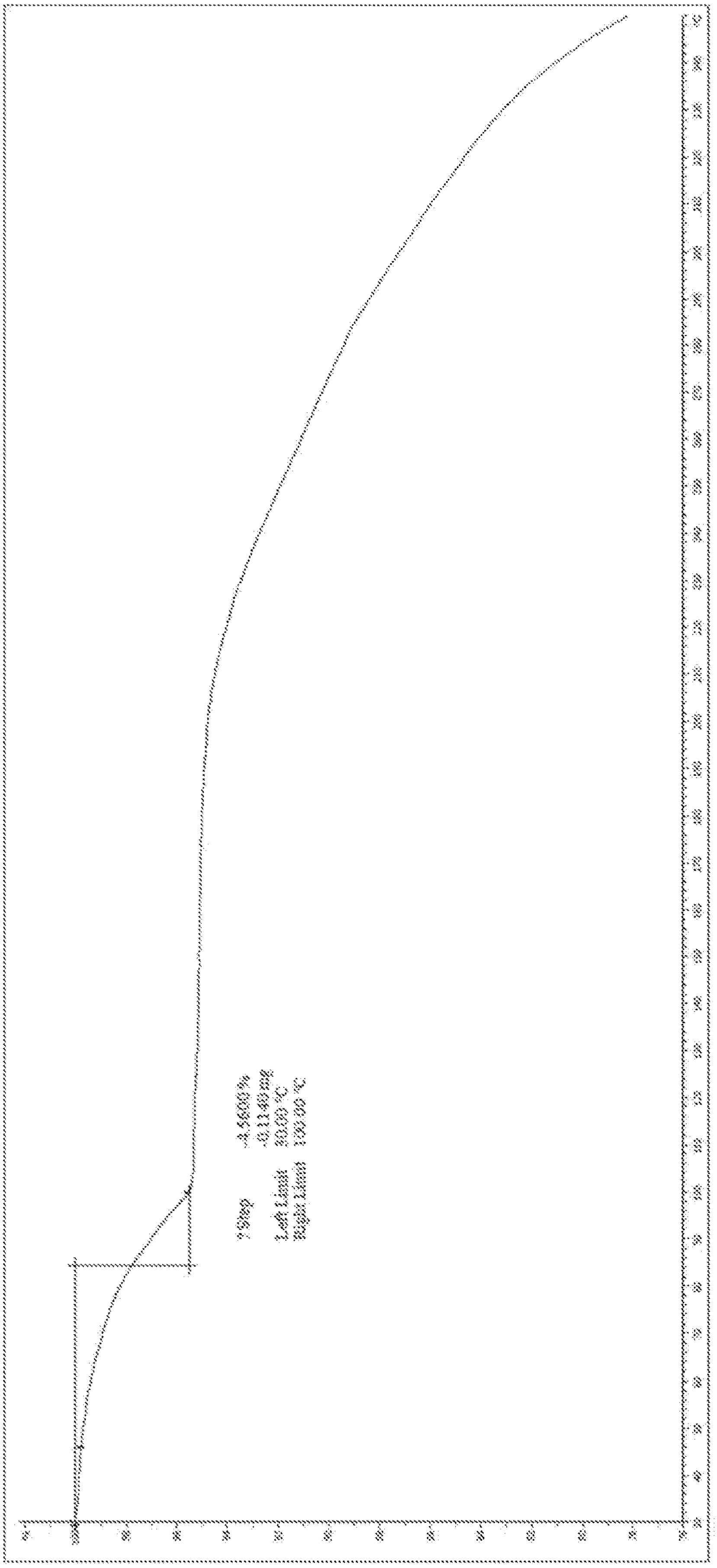


Fig. 10-1

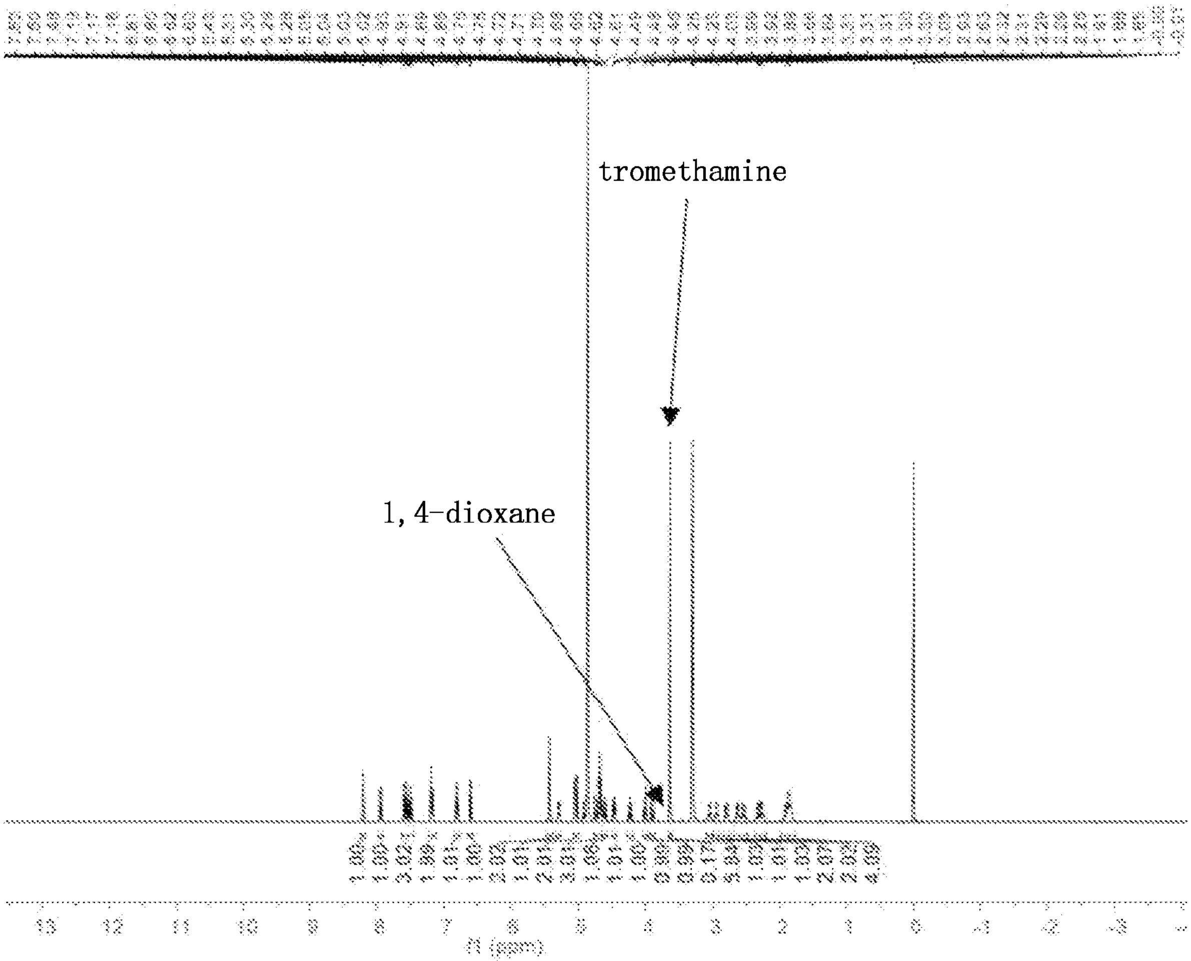


Fig. 10-2

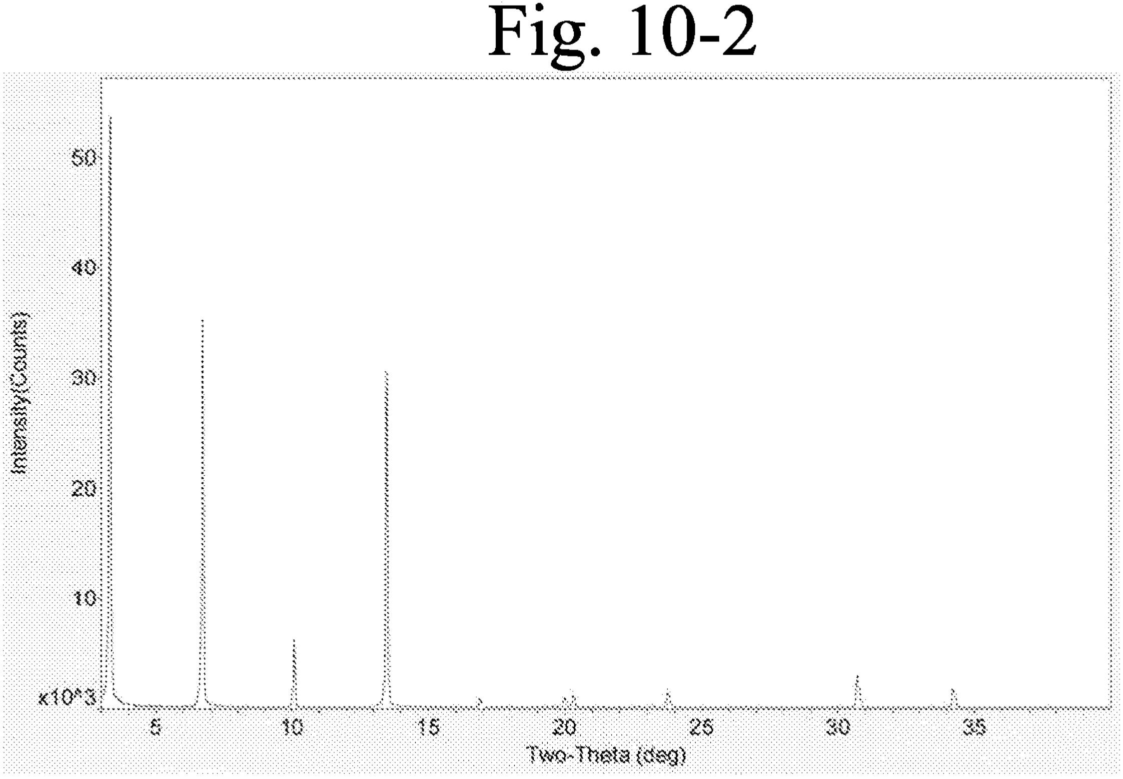


Fig. 11

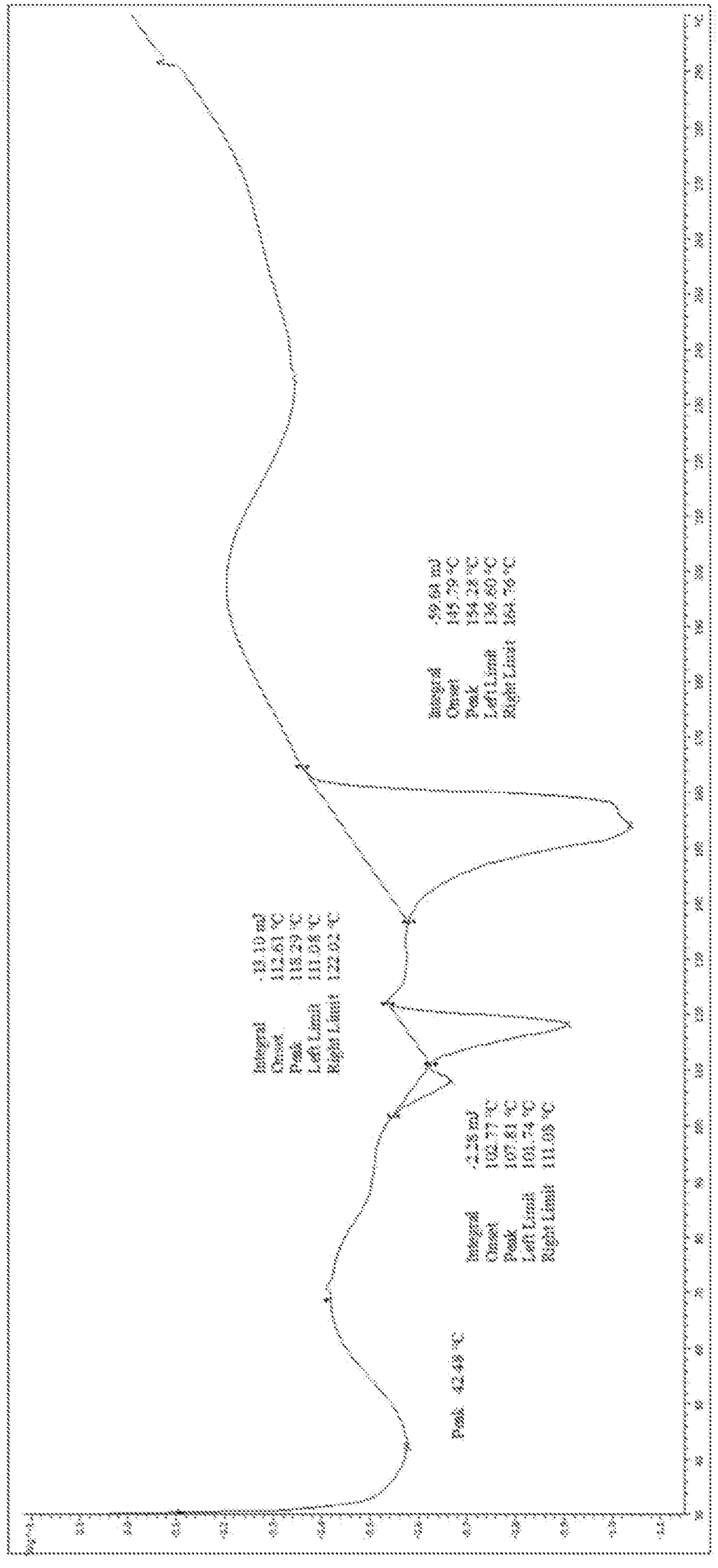

Fig. 12

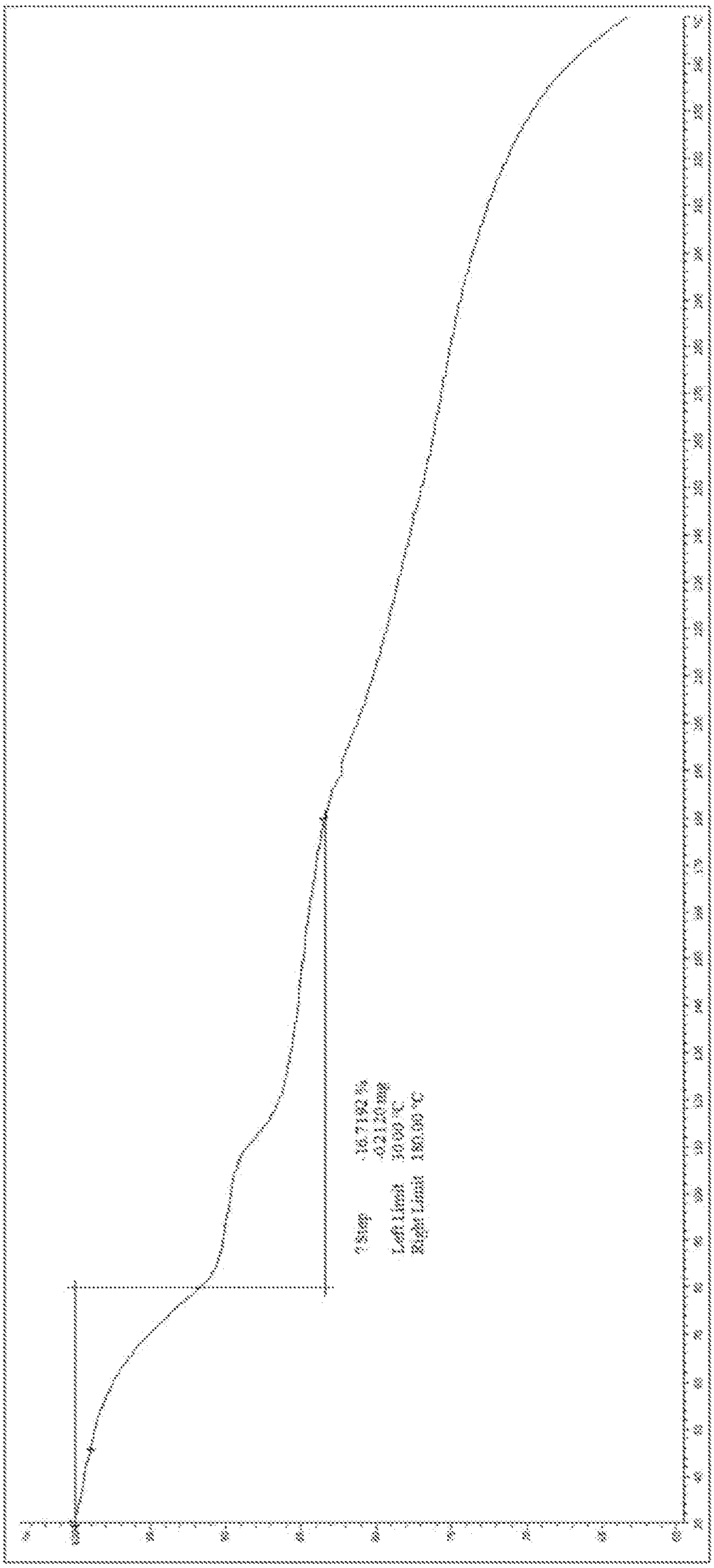


Fig. 13-1

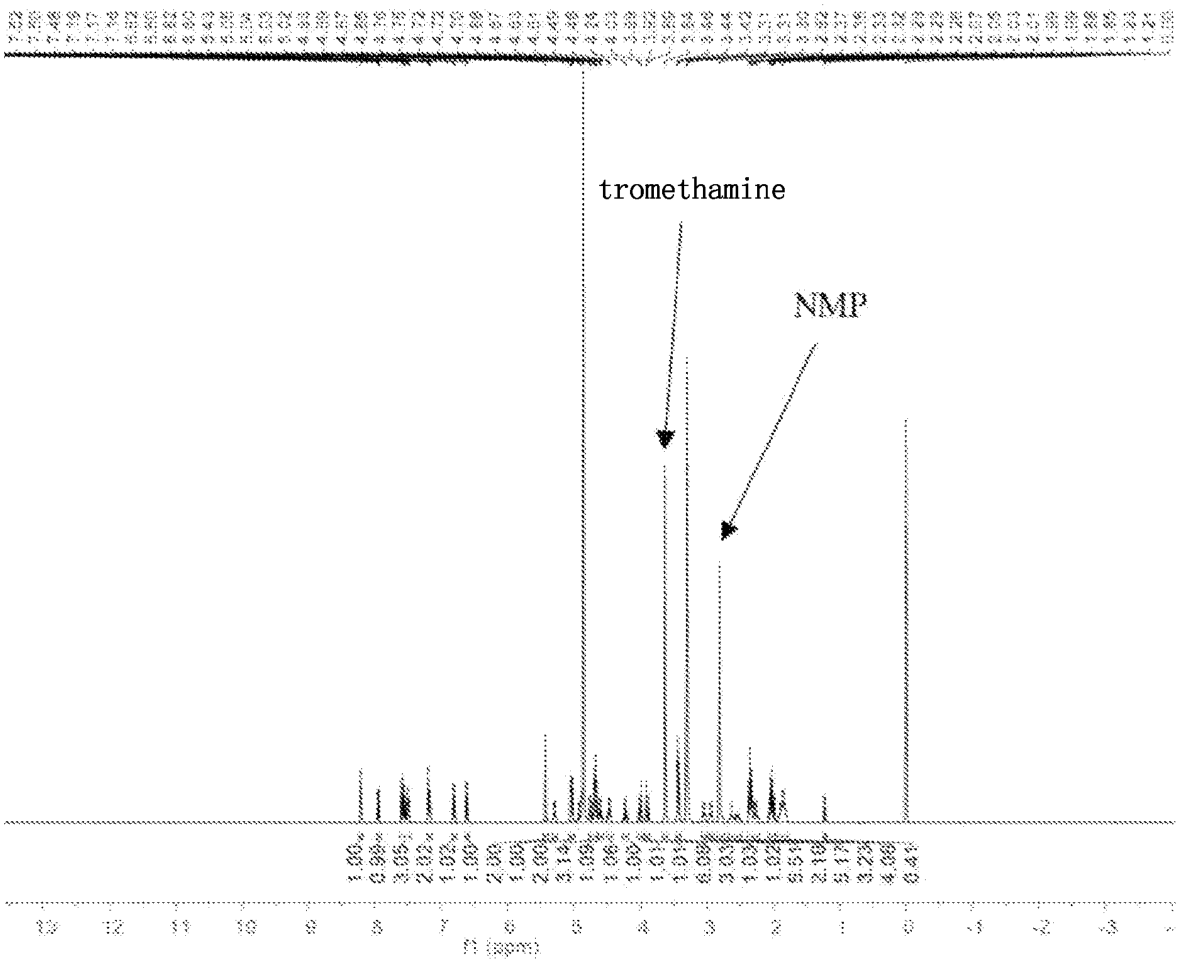


Fig. 13-2

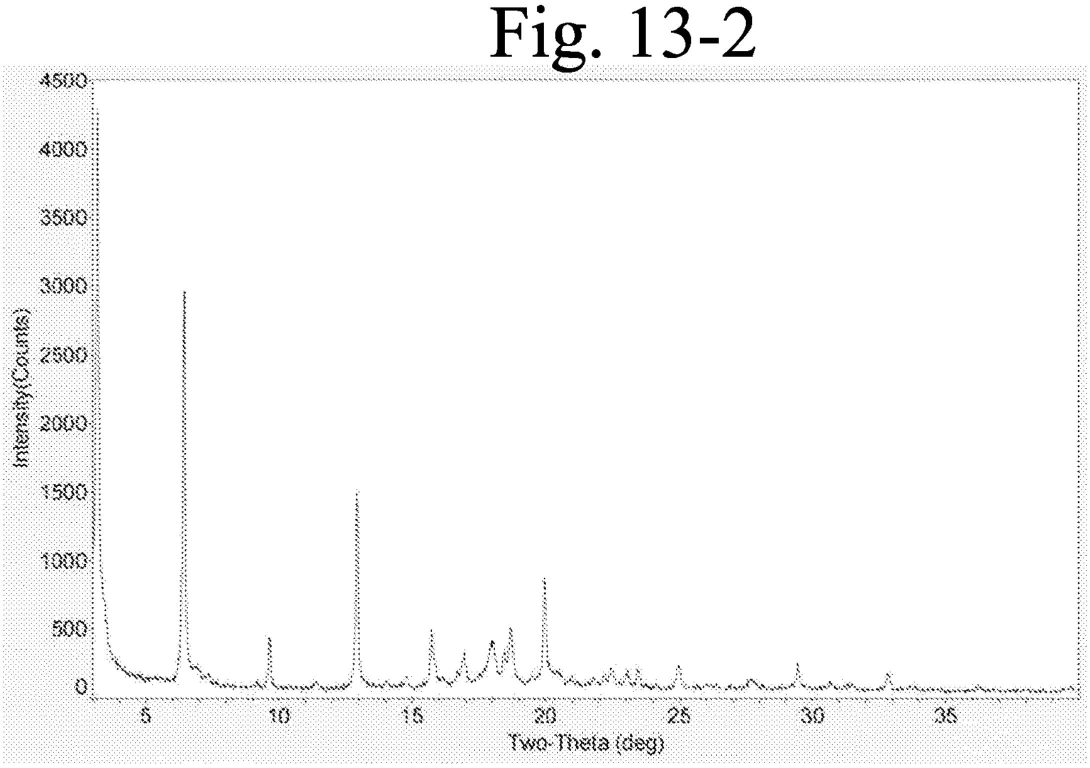


Fig. 14

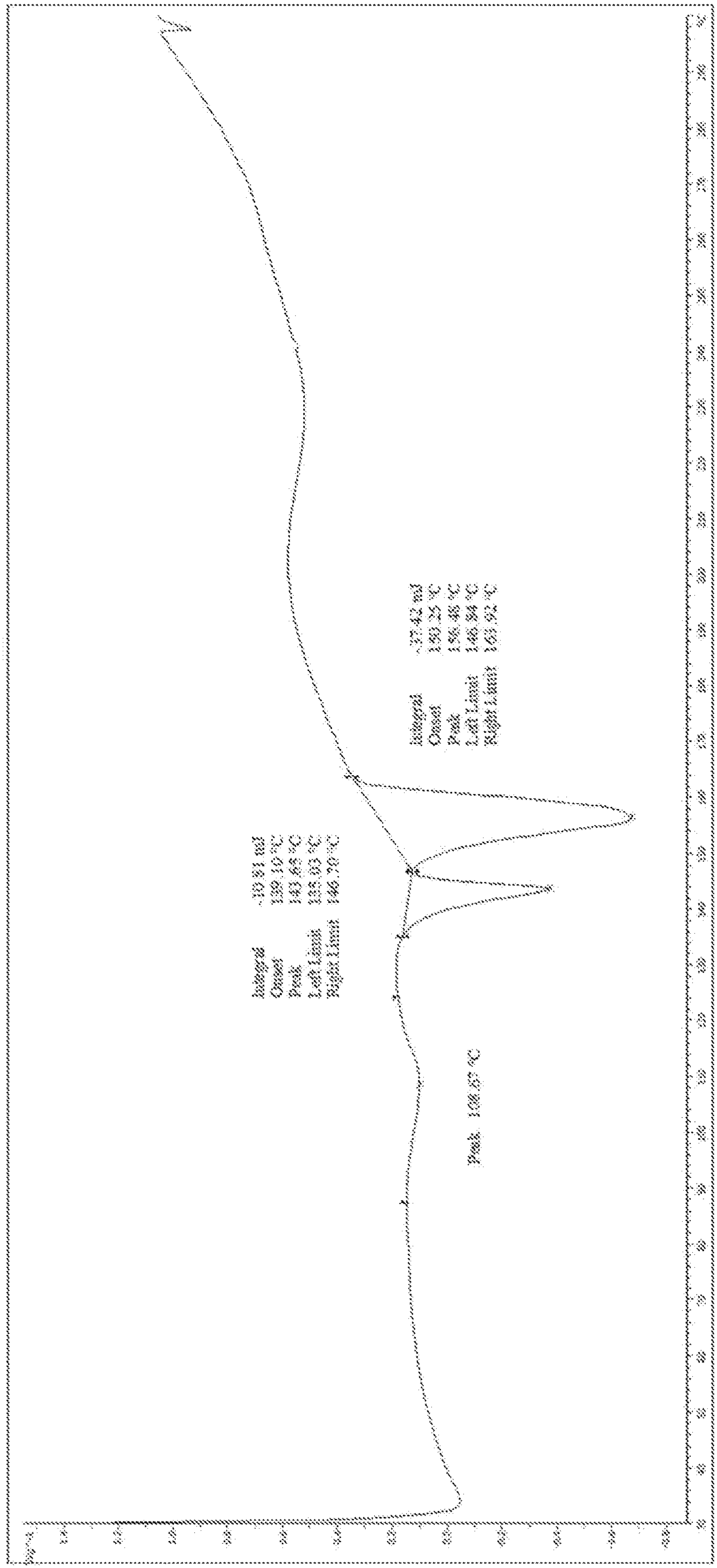

Fig. 15

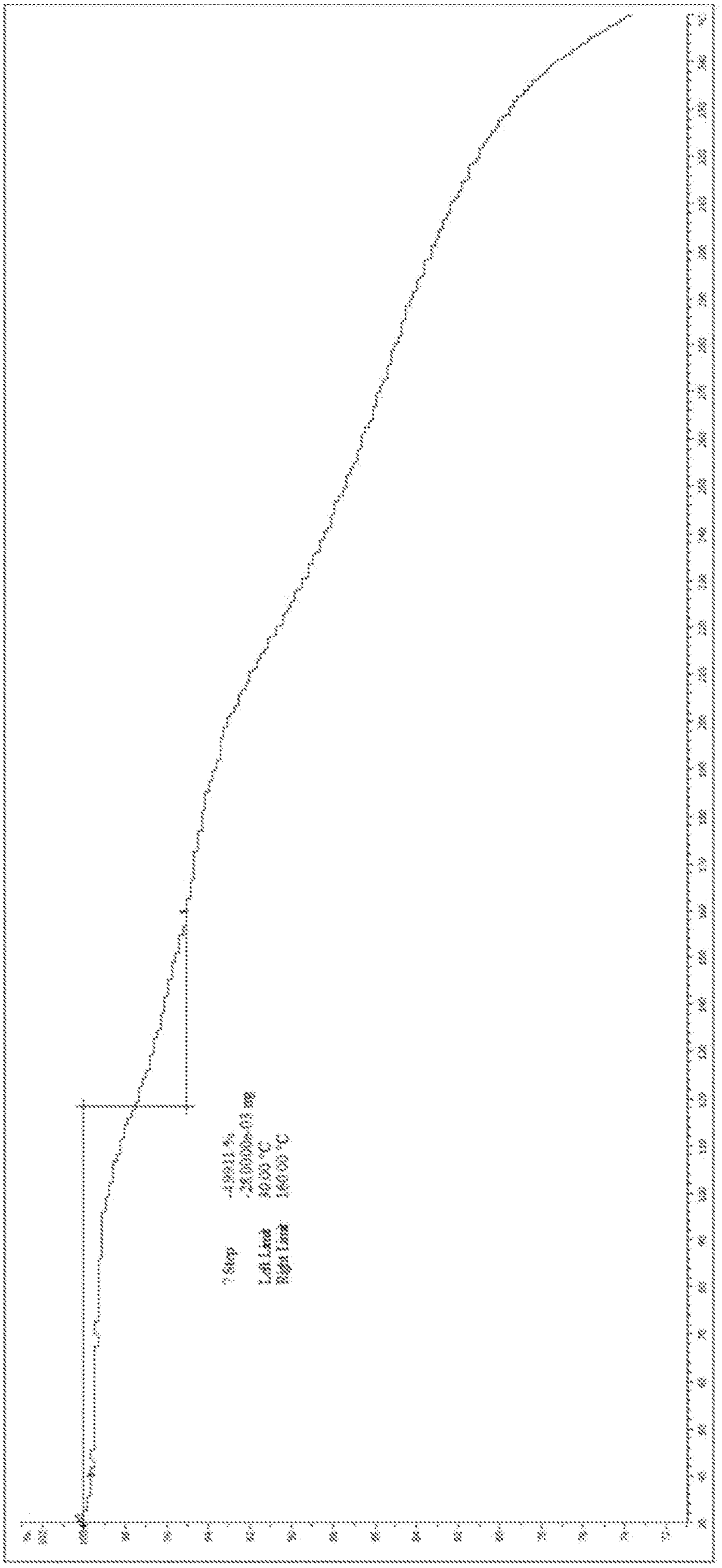

Fig. 16-1

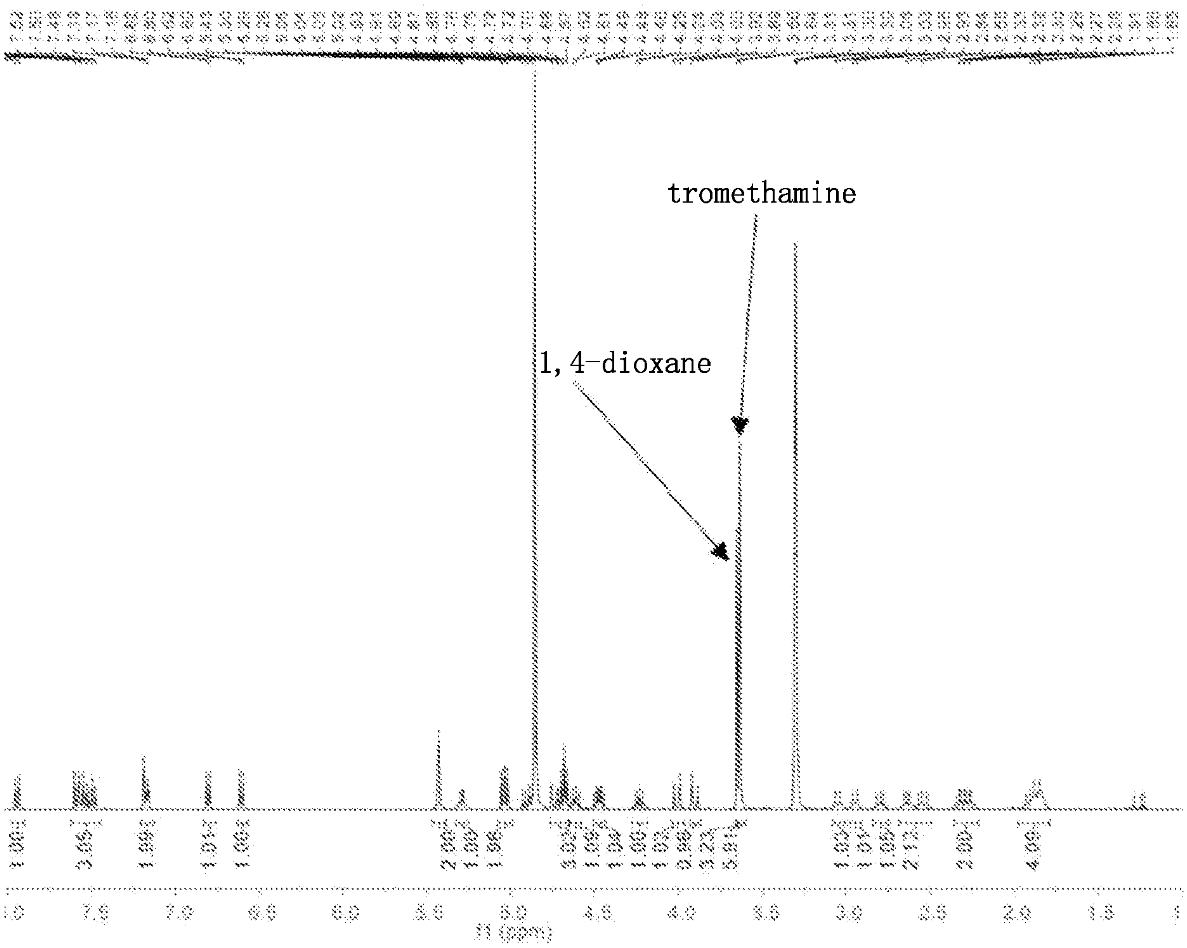


Fig. 16-2

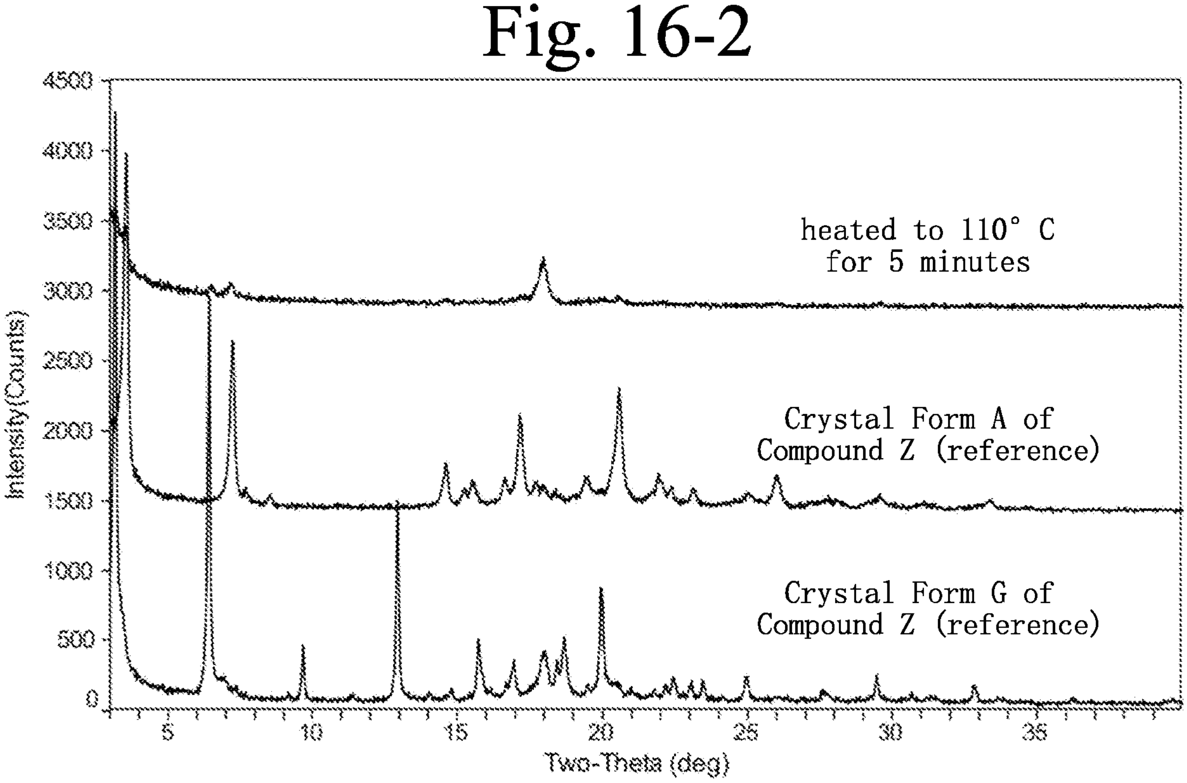


Fig. 16-3

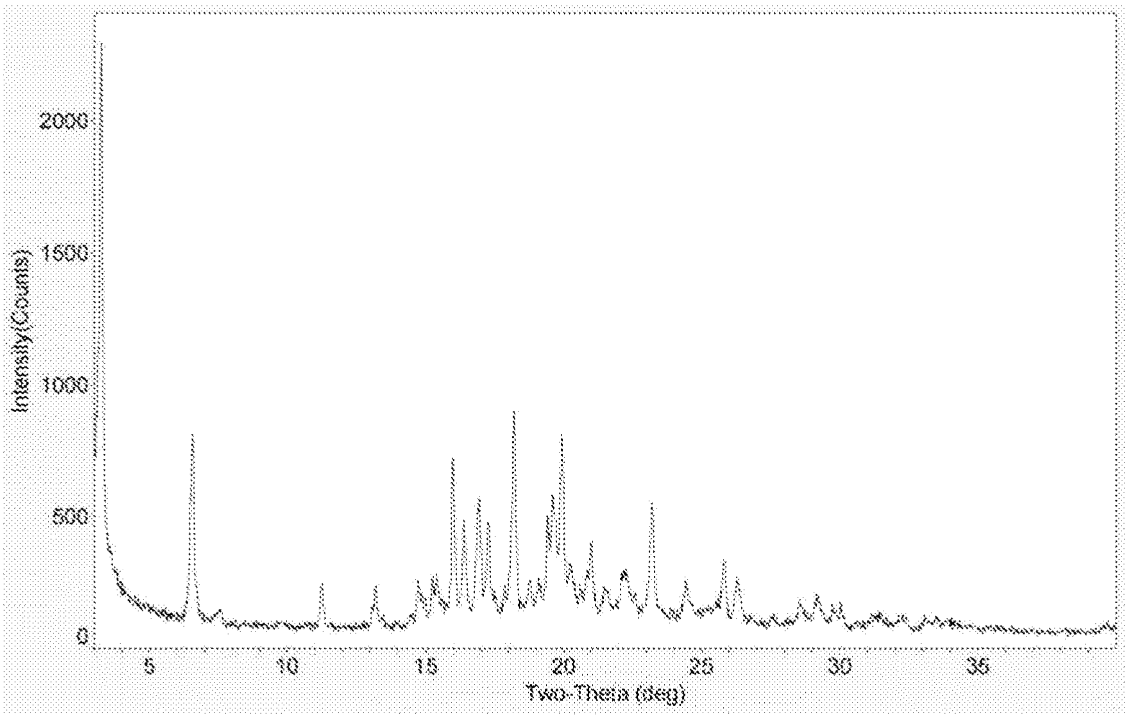
2000
1500
1000
500
0
Intensity(Counts)
5
10
15
20
25
30
35
Two-Theta (deg)

Fig. 17

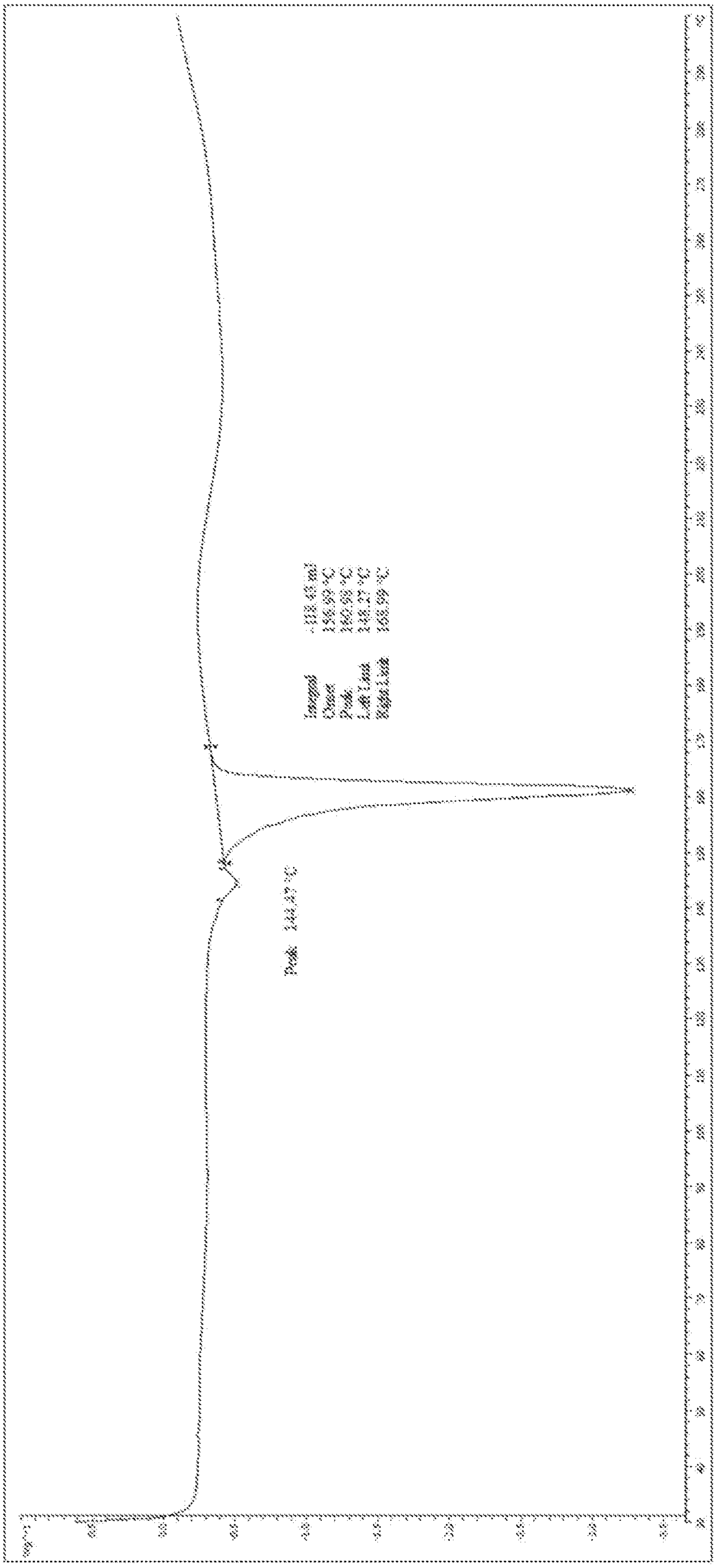

Fig. 18

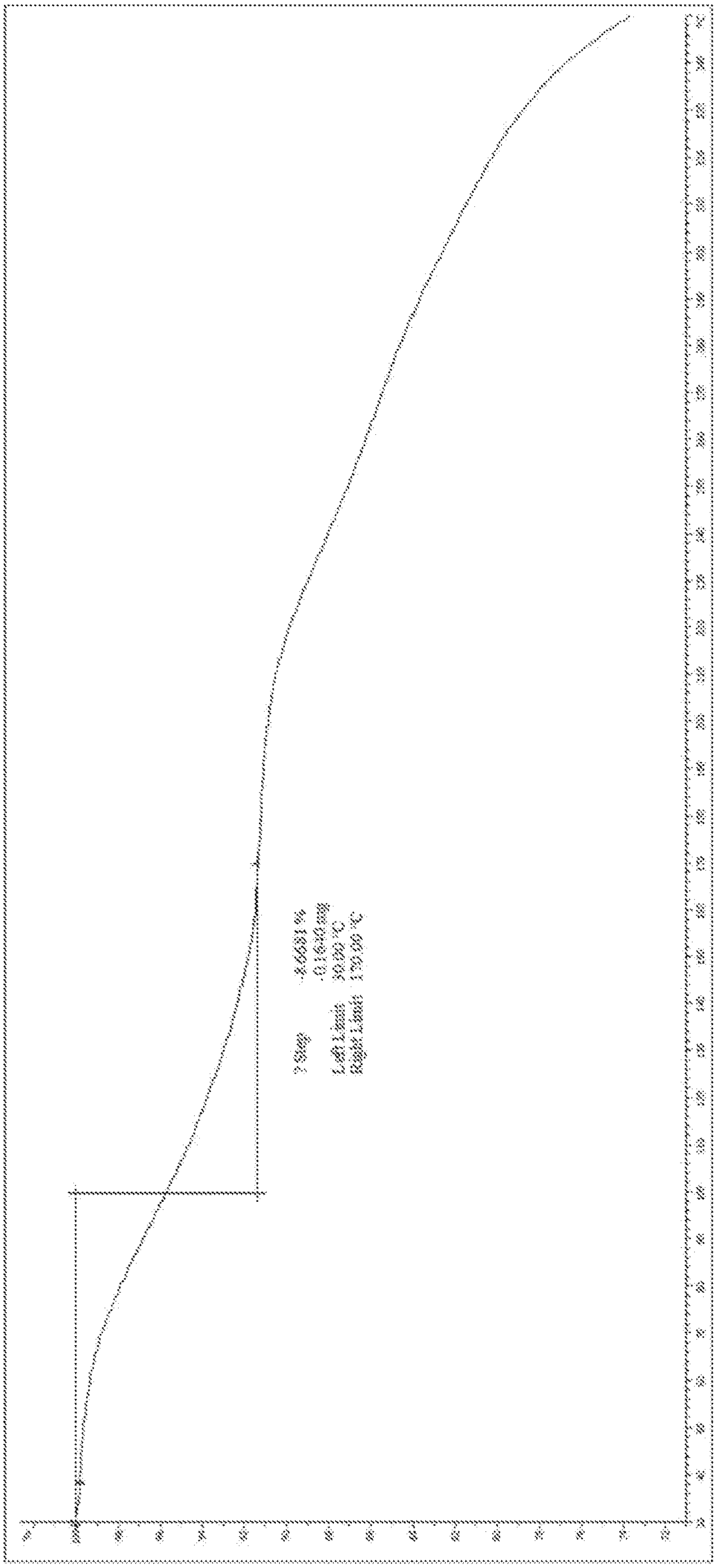

Fig. 19-1

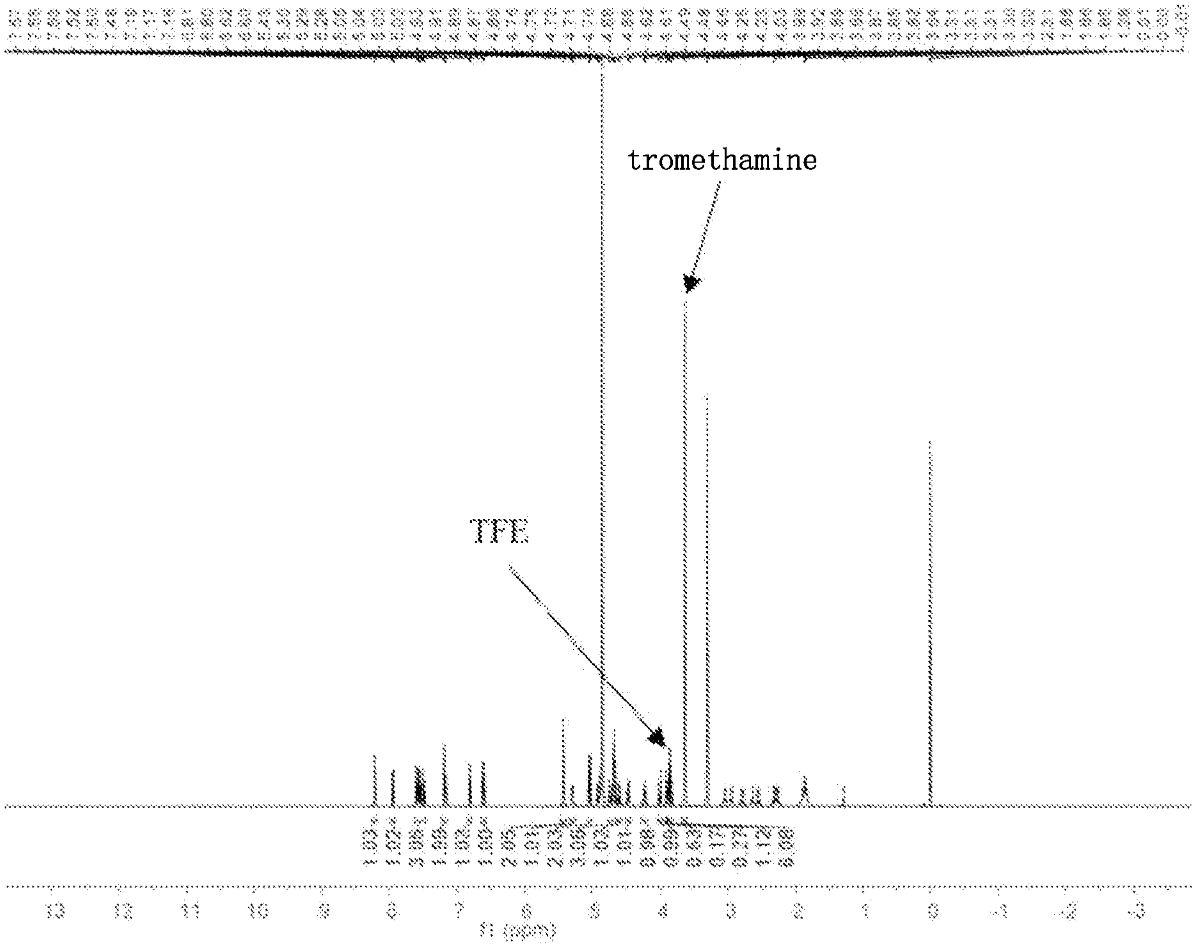


Fig. 19-2

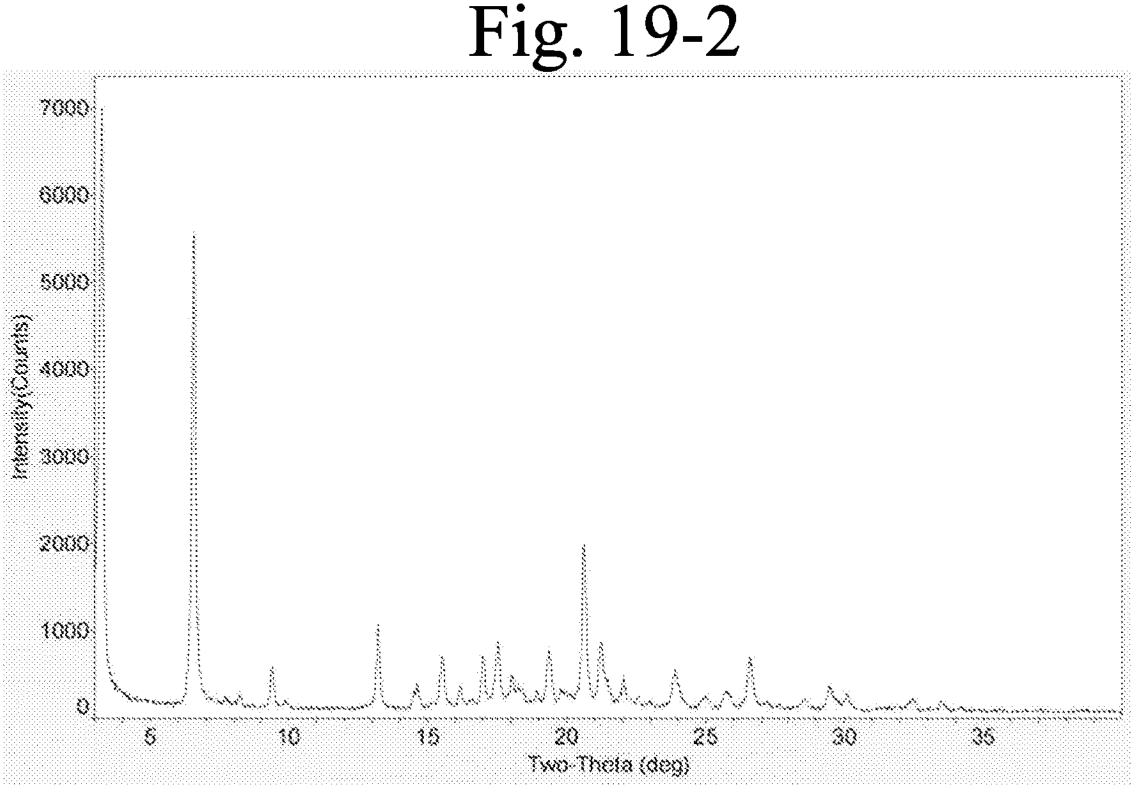


Fig. 20

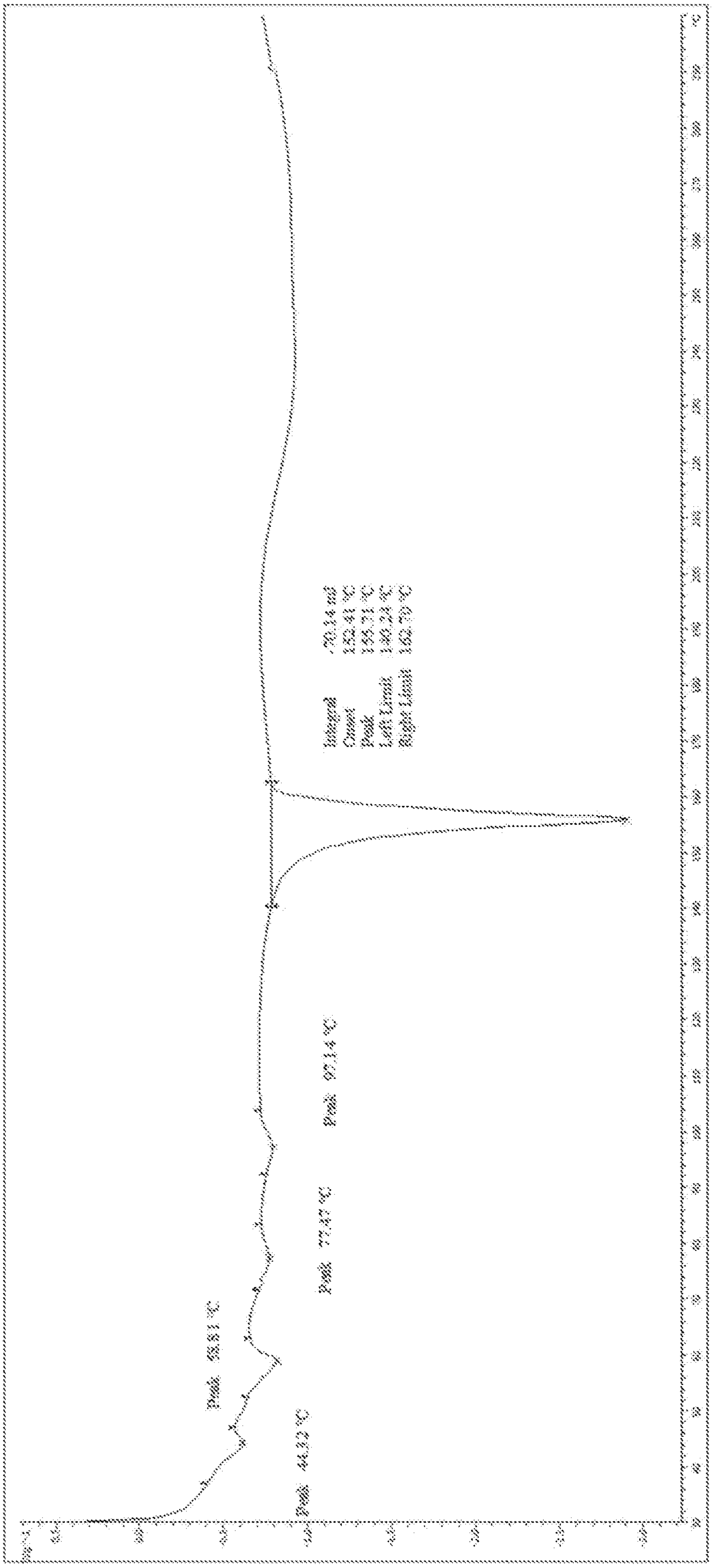

Fig. 21

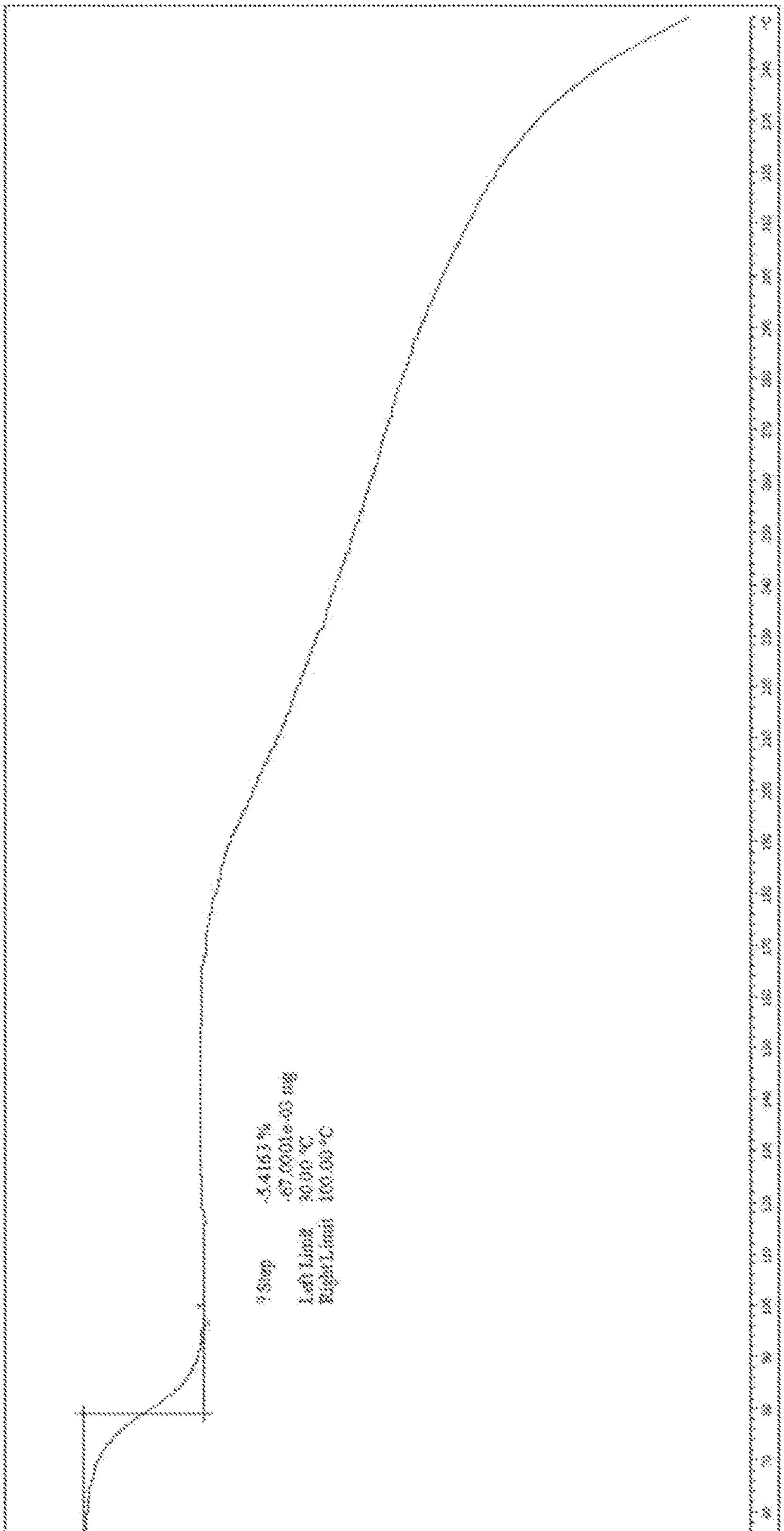

Fig. 22-1

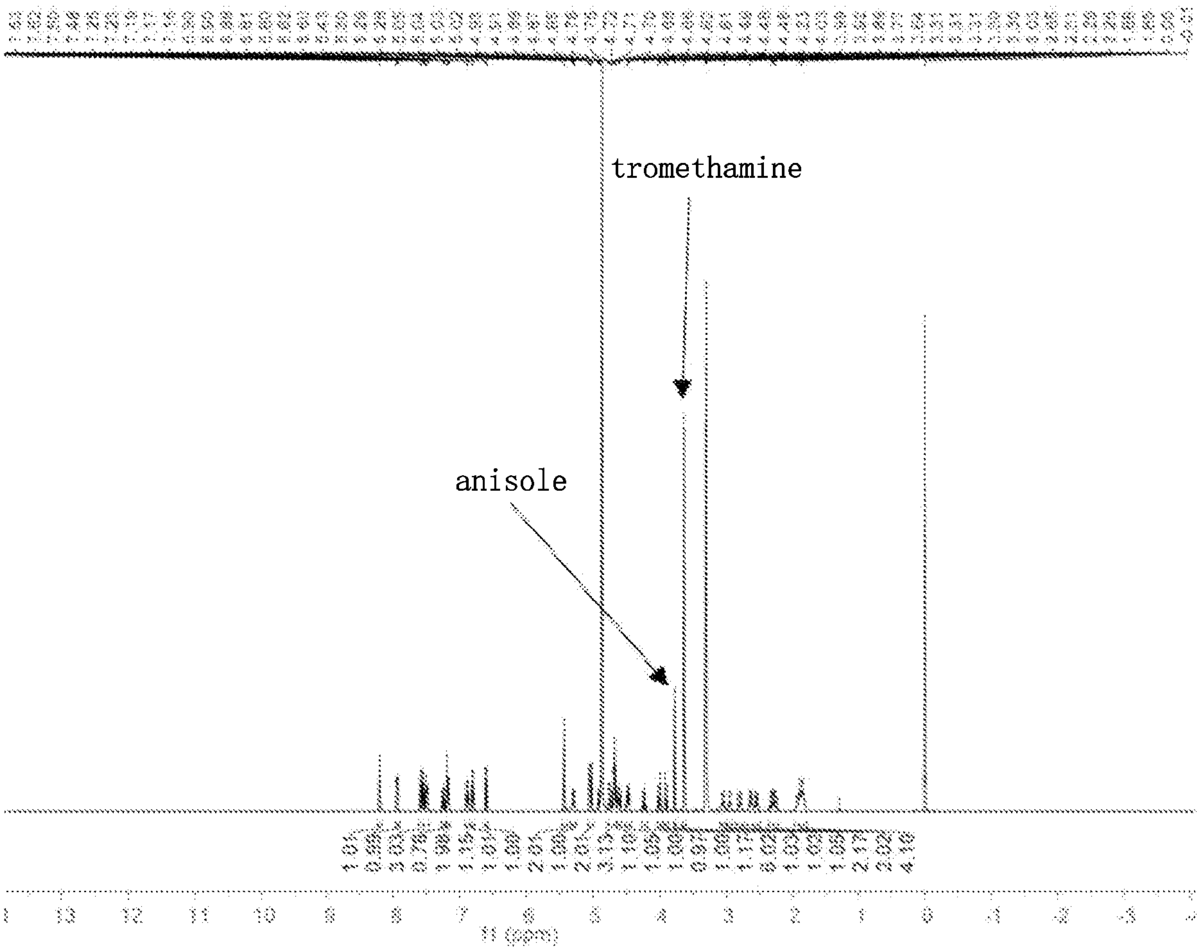


Fig. 22-2

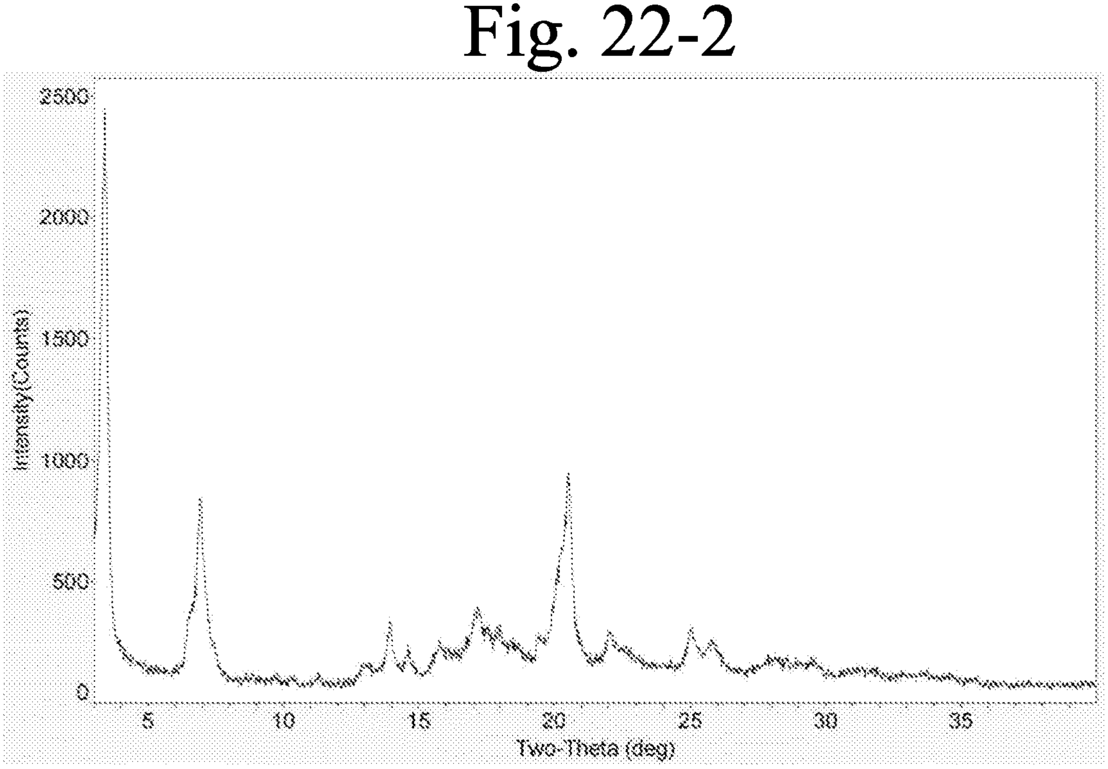


Fig. 23

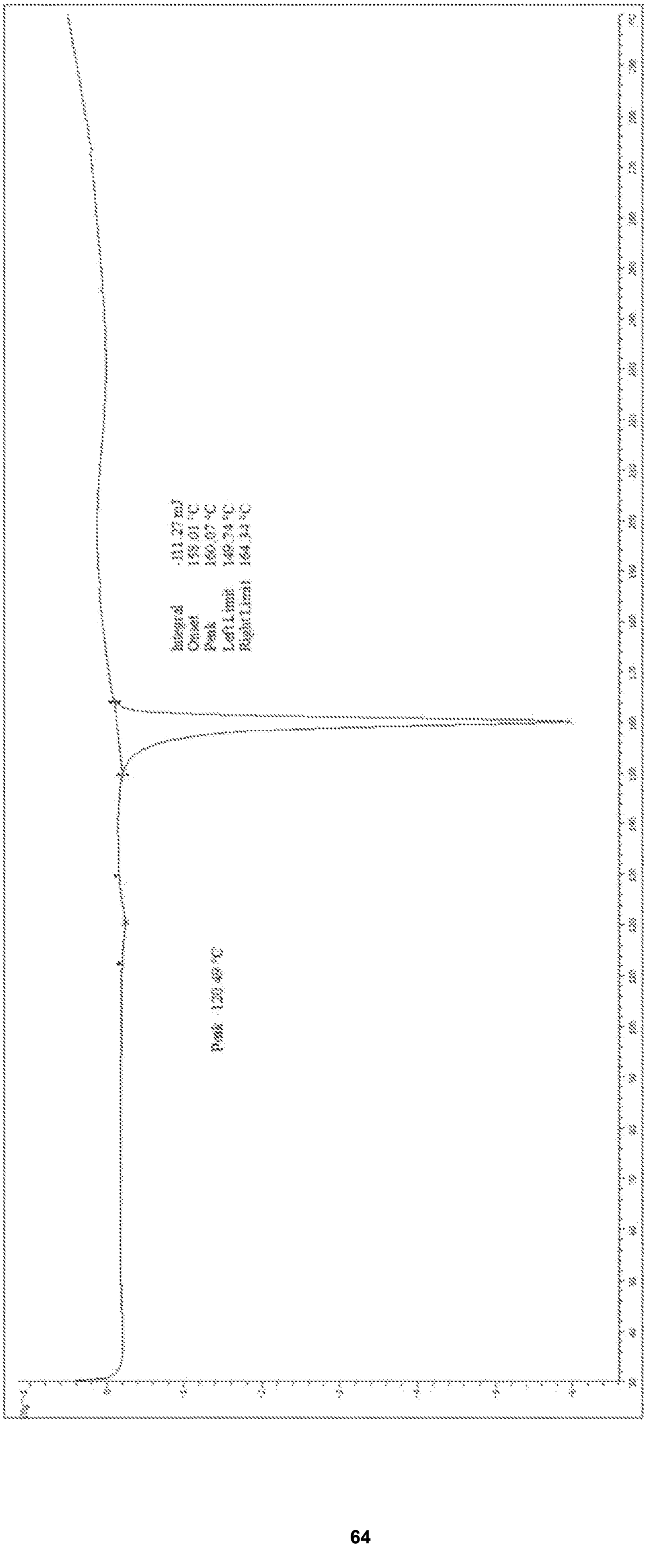

Fig. 24

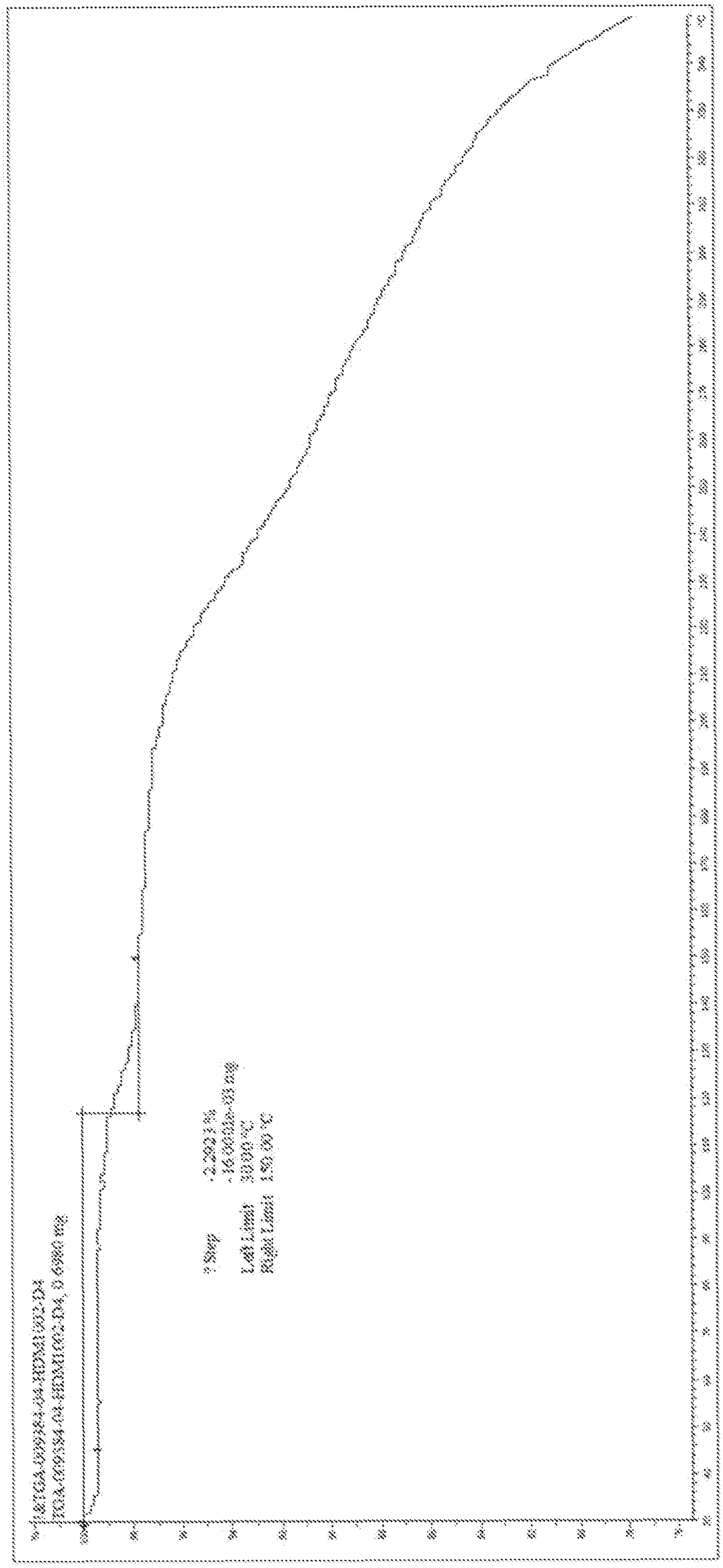

Fig. 25-1

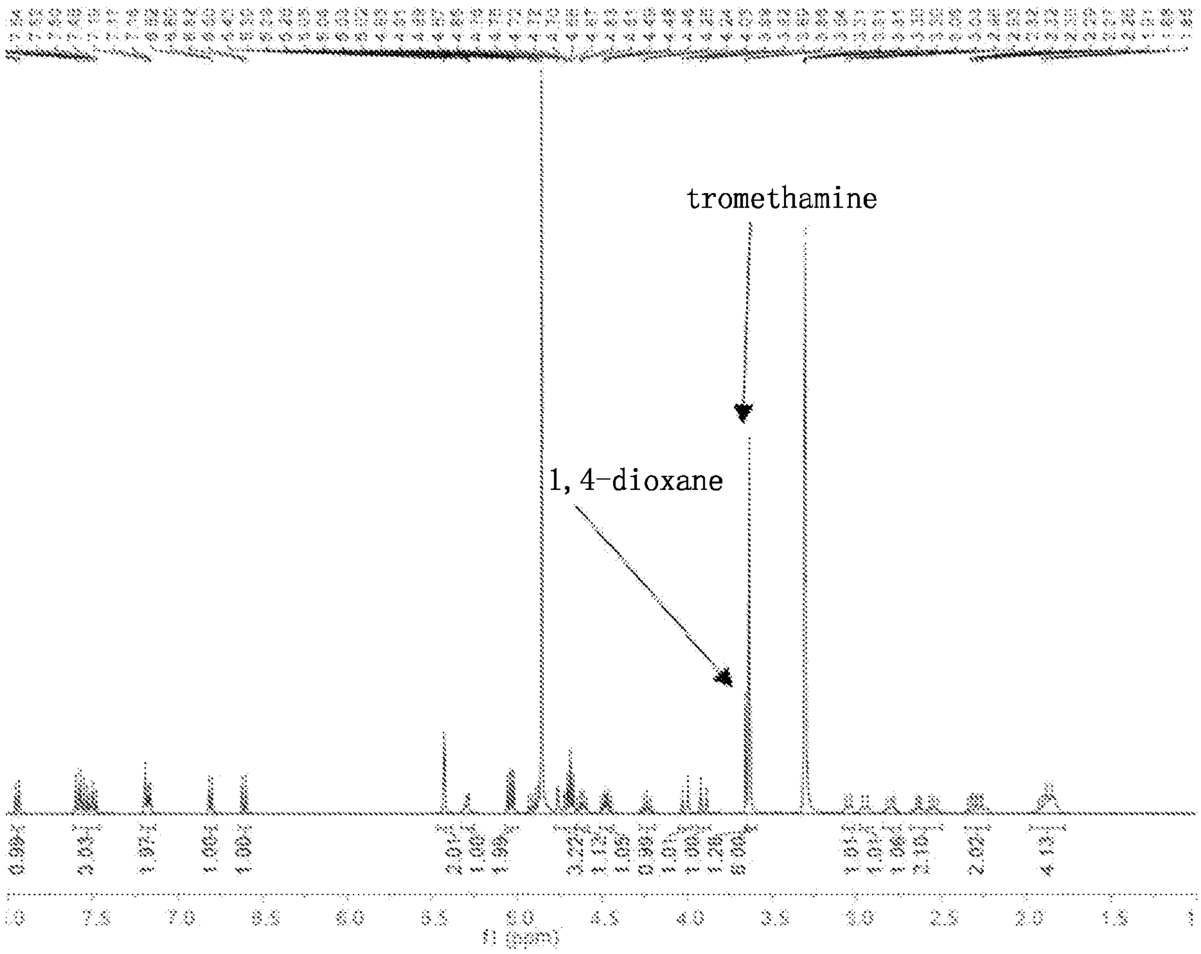


Fig. 25-2

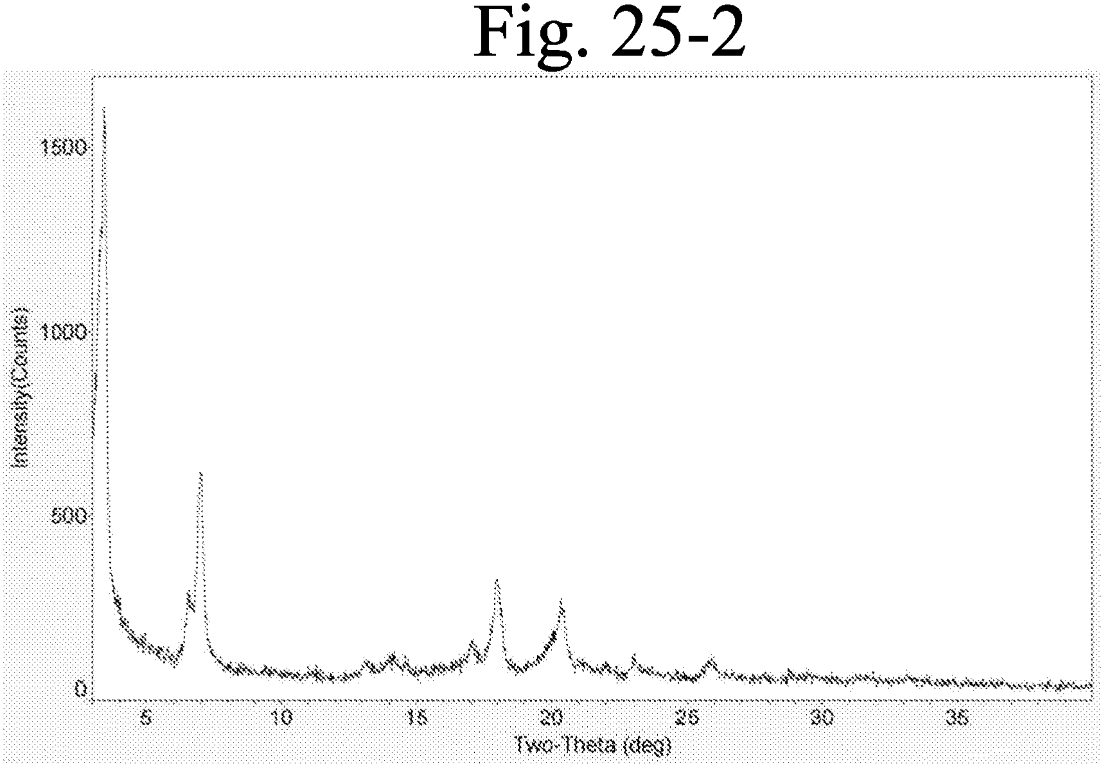


Fig. 26

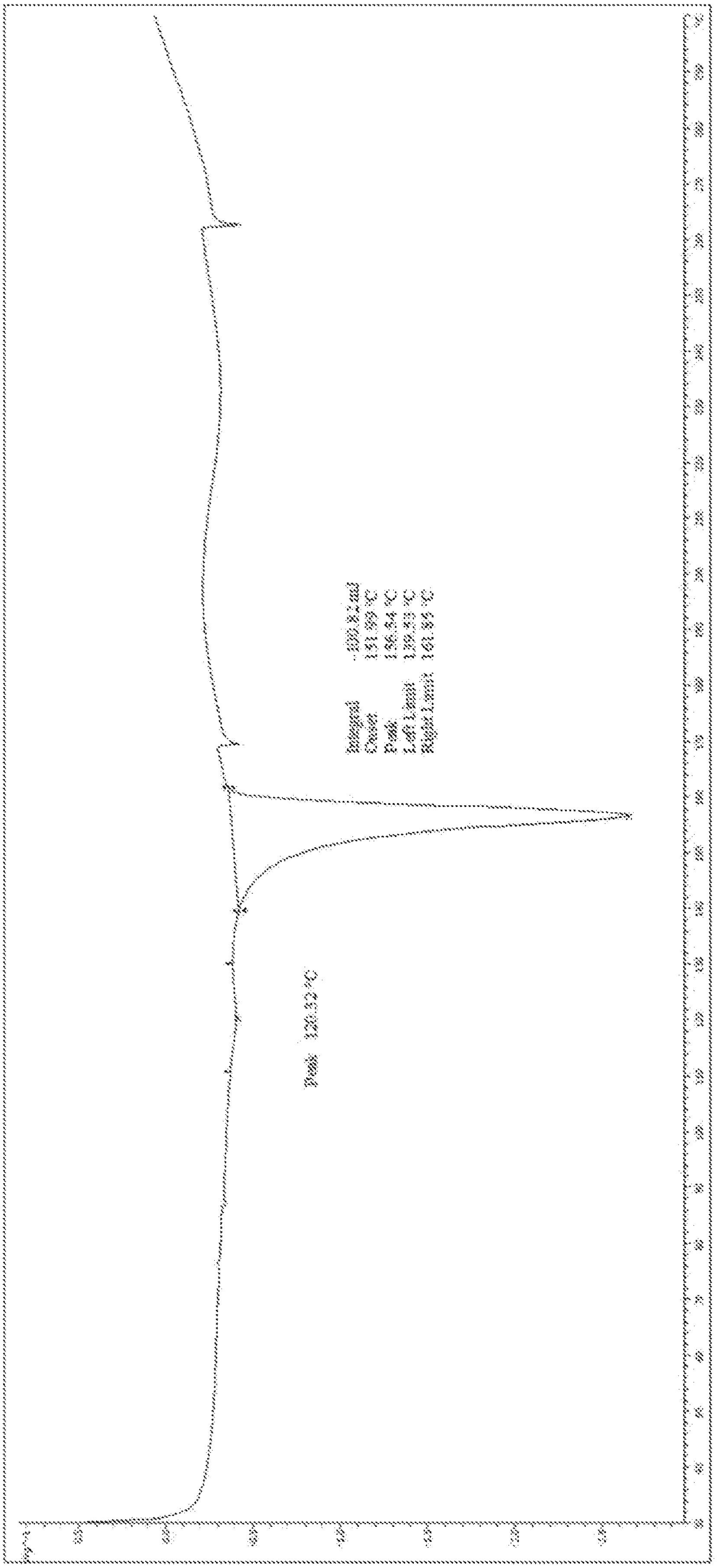

Fig. 27

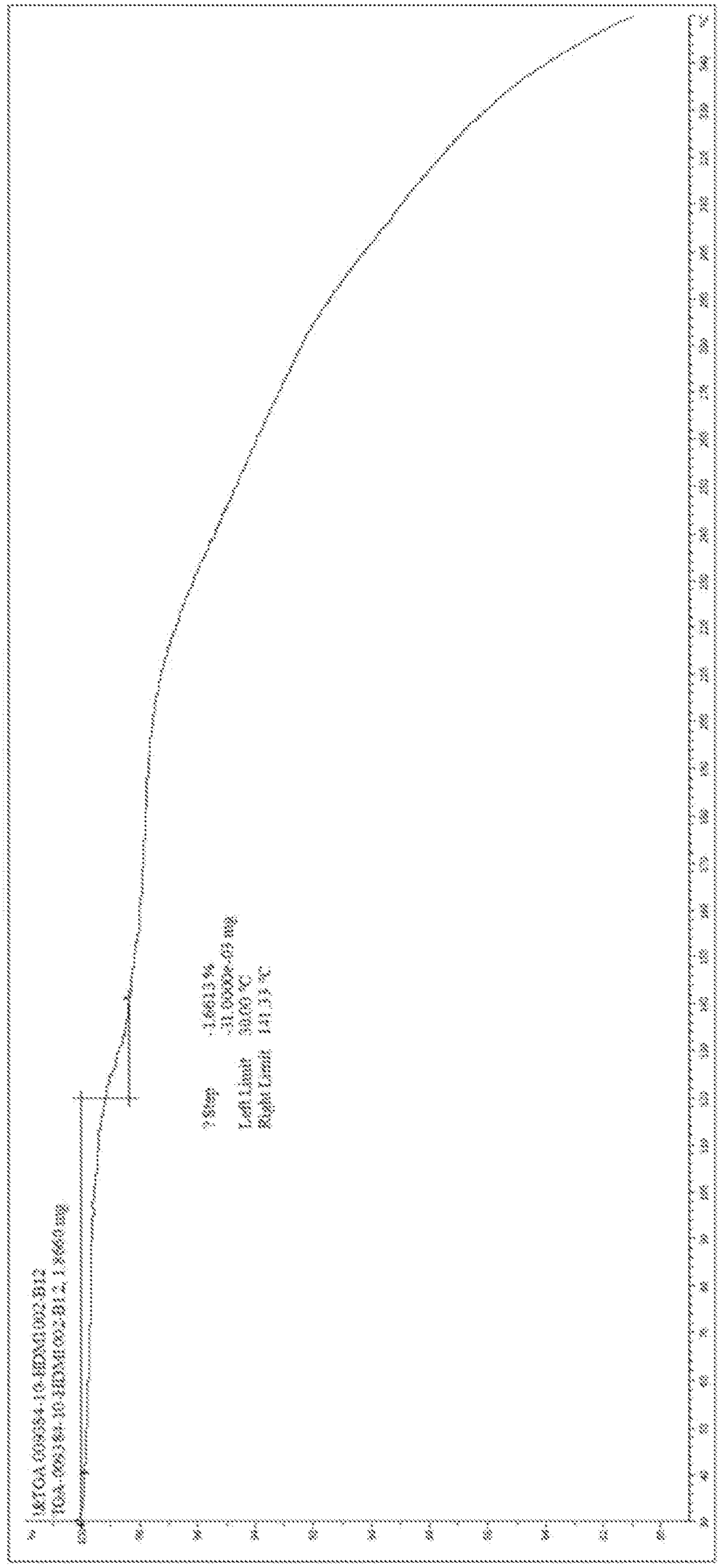

Fig. 28-1

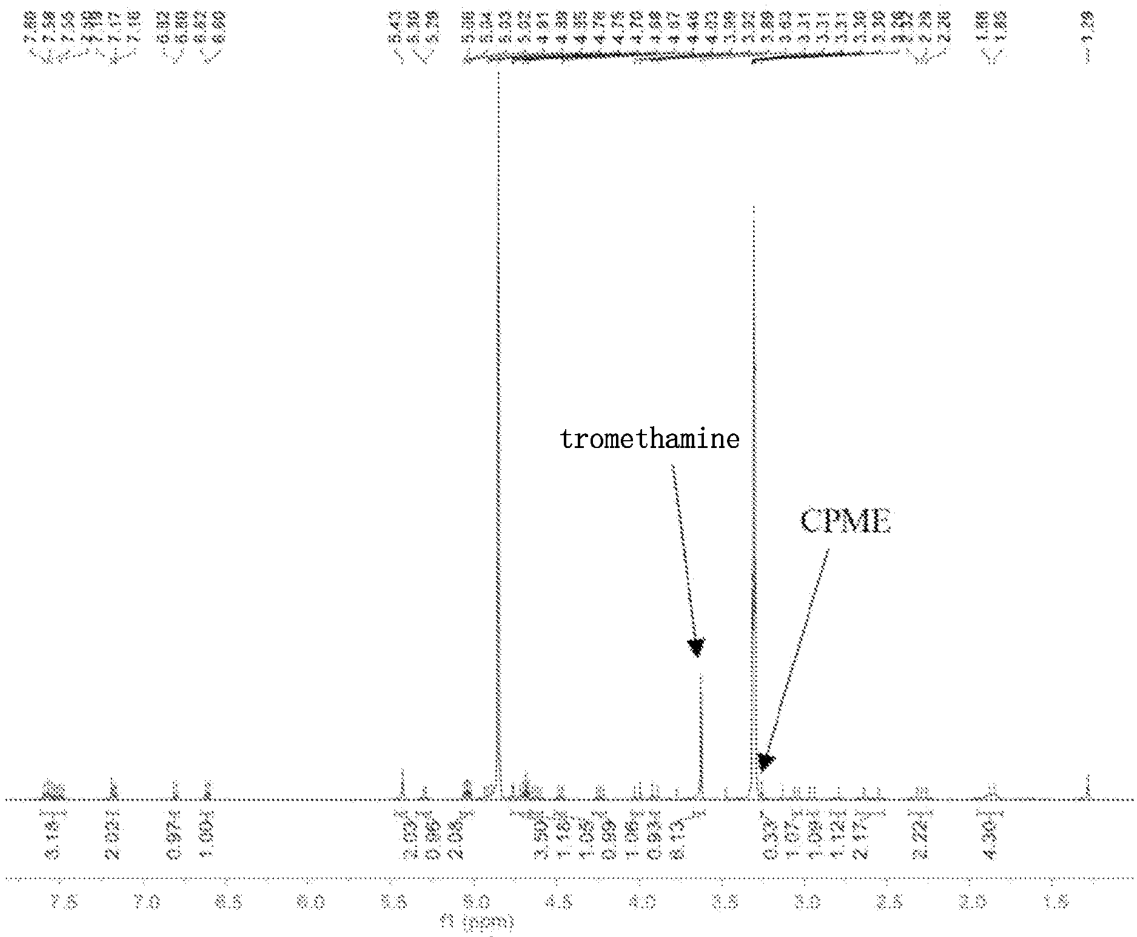


Fig. 28-2

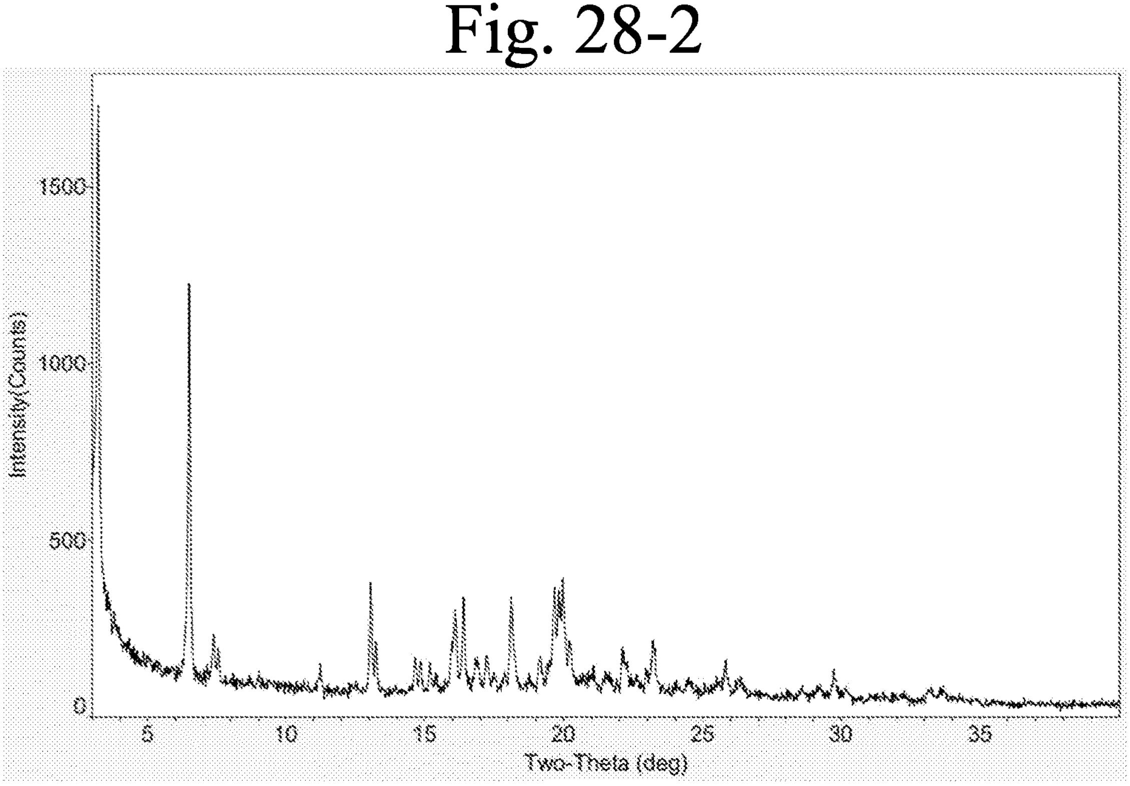


Fig. 29

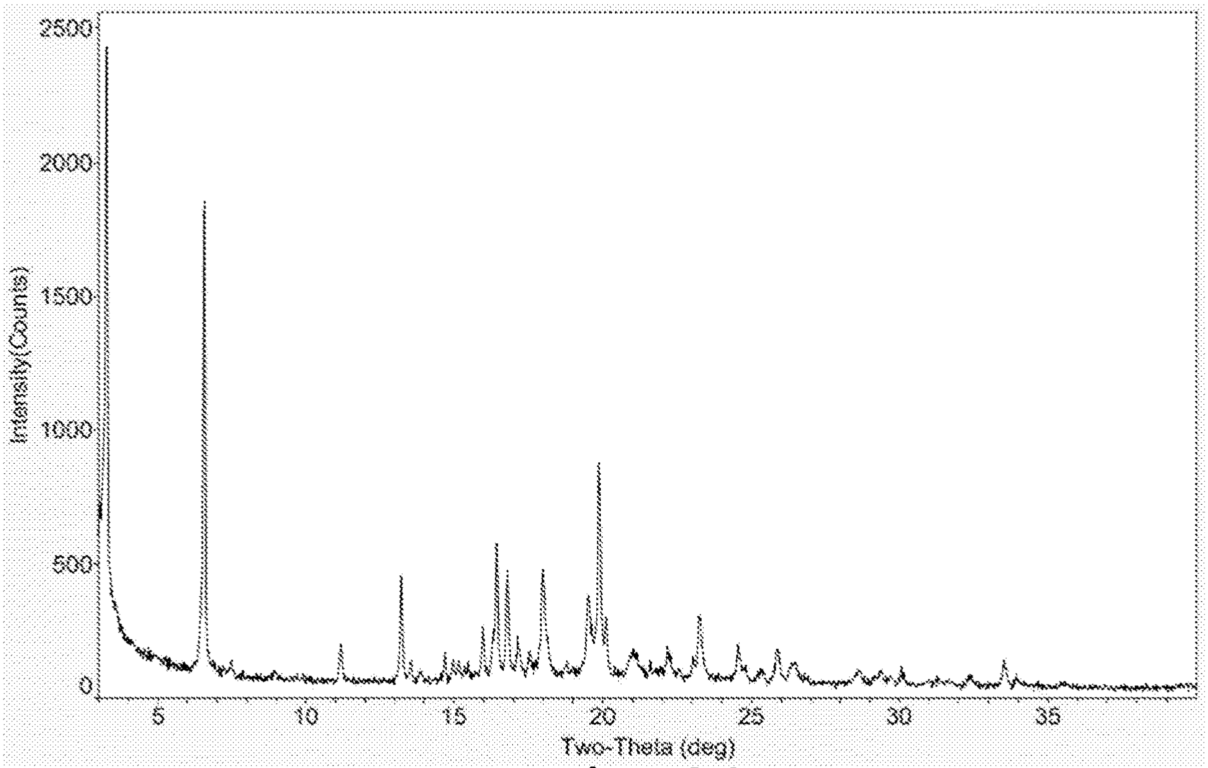
2500
2000
1500
1000
500
0
Intensity(Counts)
5
10
15
20
25
30
35
Two-Theta (deg)

Fig. 30

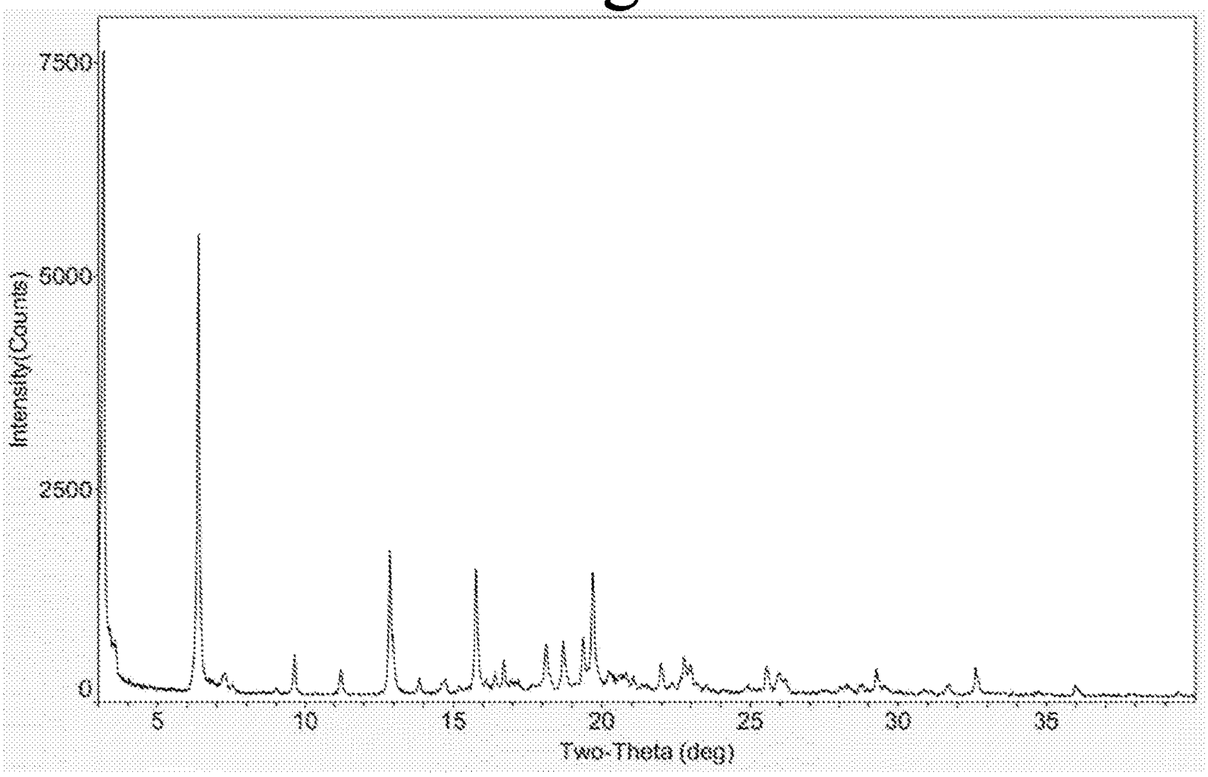
7500
5000
2500
0
Intensity(Counts)
5
10
15
20
25
30
35
Two-Theta (deg)

Fig. 31

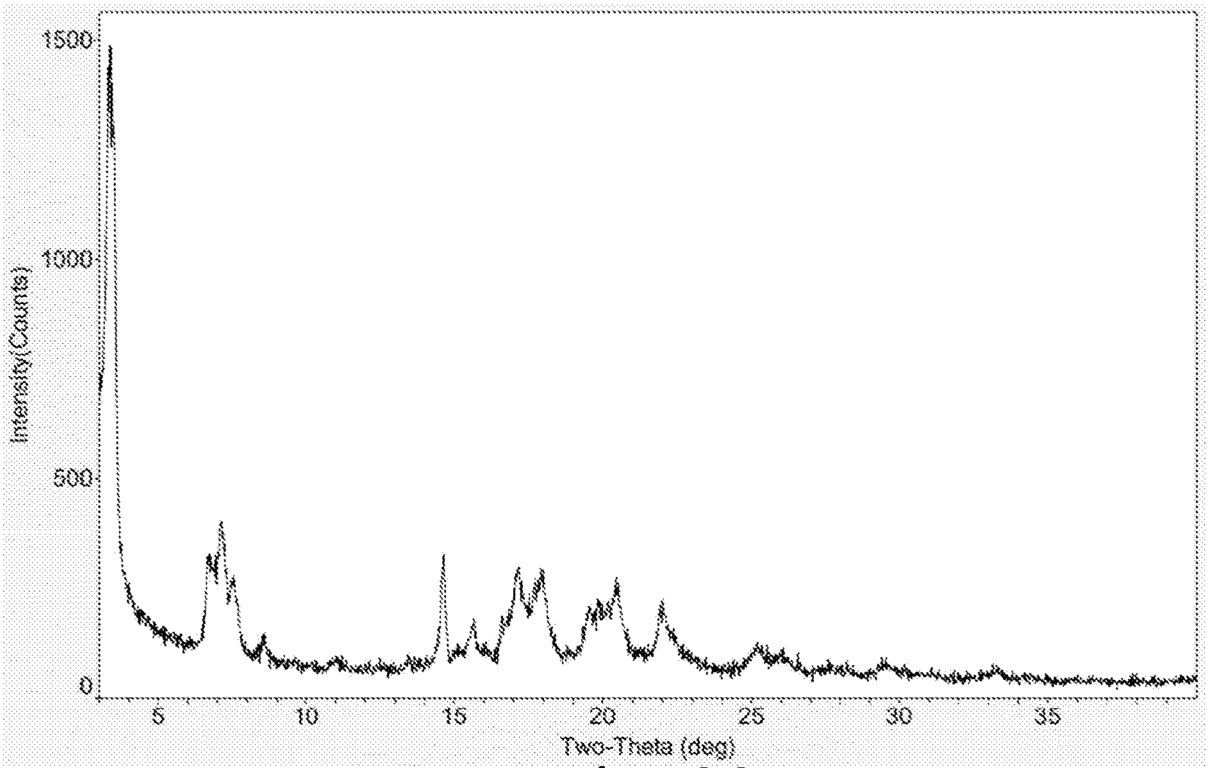
1500
1000
500
0
Intensity(Counts)
5
10
15
20
25
30
35
Two-Theta (deg)

Fig. 32

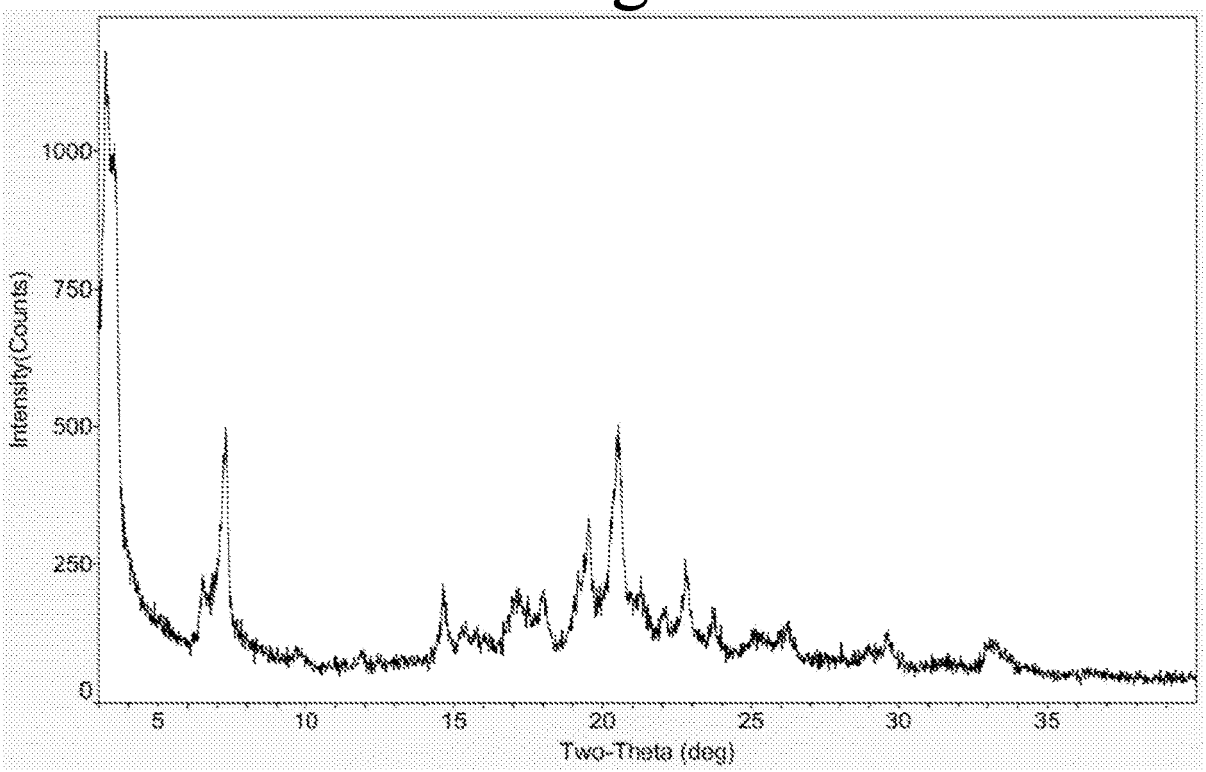
1000
750
500
250
0
Intensity(Counts)
5
10
15
20
25
30
35
Two-Theta (deg)

Fig. 33

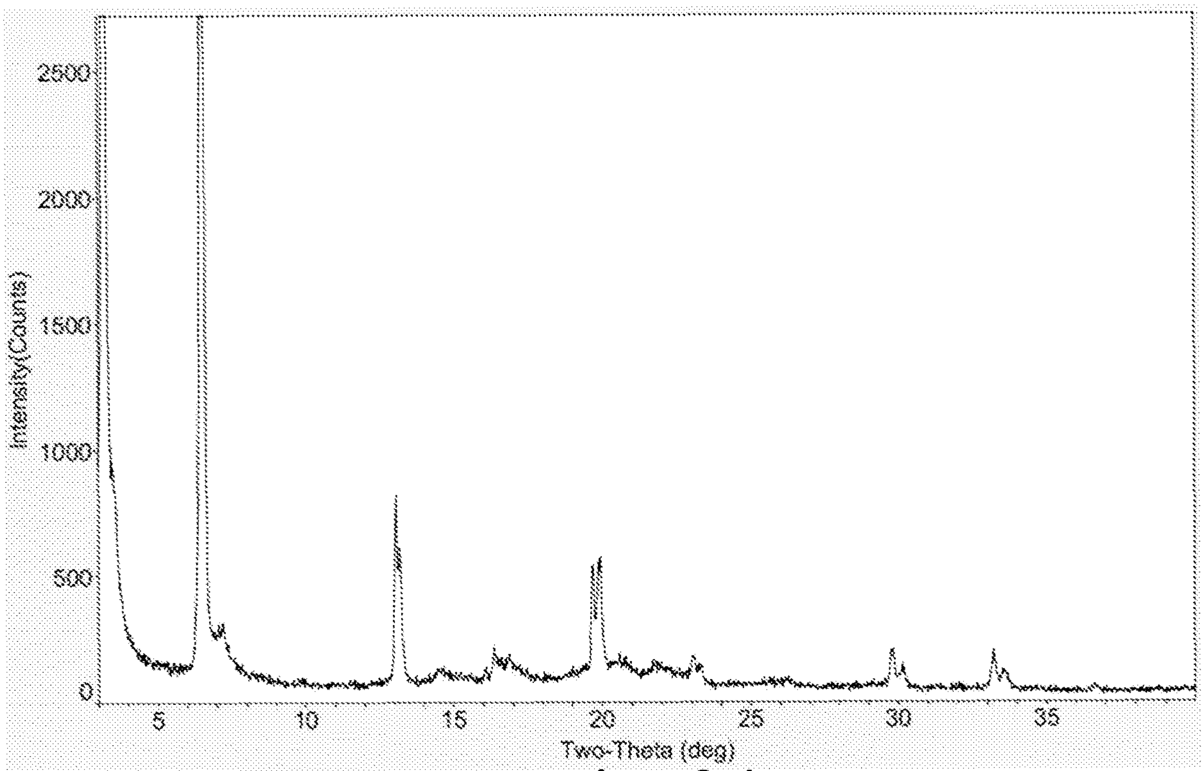
2500
2000
1500
1000
500
0
Intensity(Counts)
5
10
15
20
25
30
35
Two-Theta (deg)

Fig. 34

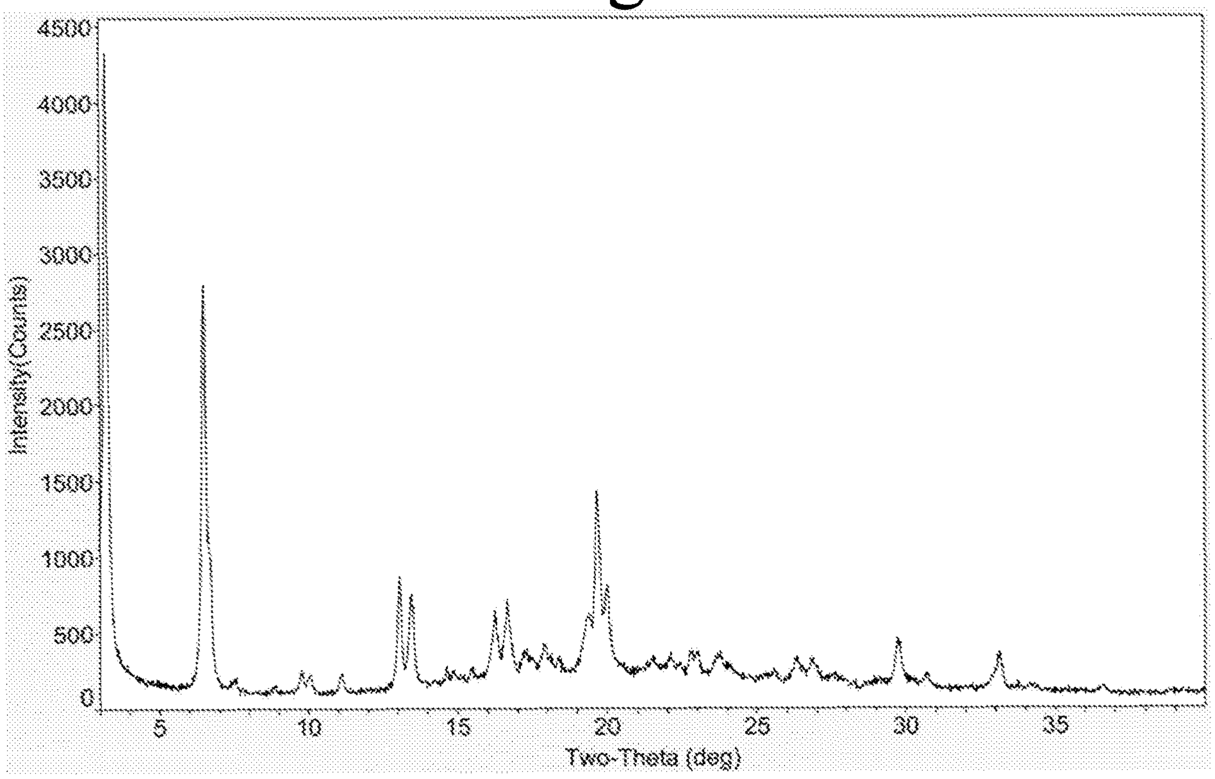
4500
4000
3500
3000
2500
2000
1500
1000
500
0
Intensity(Counts)
5
10
15
20
25
30
35
Two-Theta (deg)

Fig. 35

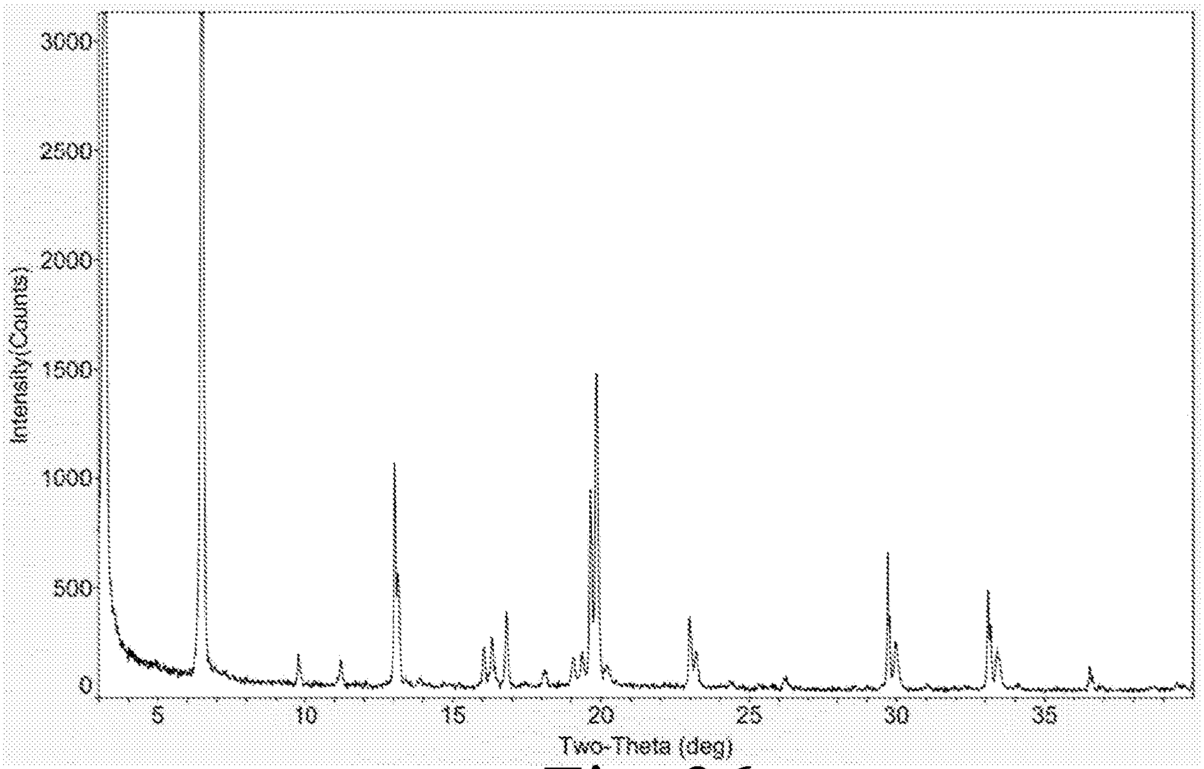

3000
2500
2000
1500
1000
500
0
Intensity(Counts)
5
10
15
20
25
30
35
Two-Theta (deg)


Fig. 36

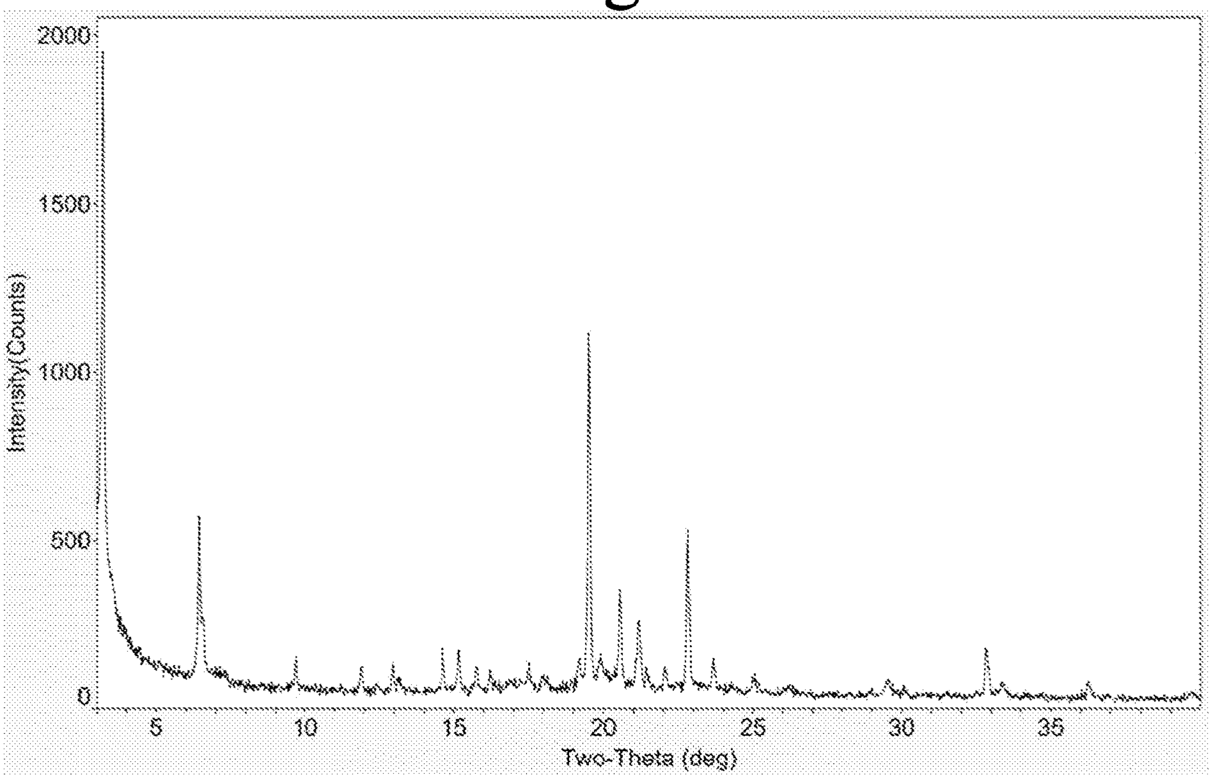

2000
1500
1000
500
0
Intensity(Counts)
5
10
15
20
25
30
35
Two-Theta (deg)


Fig. 37

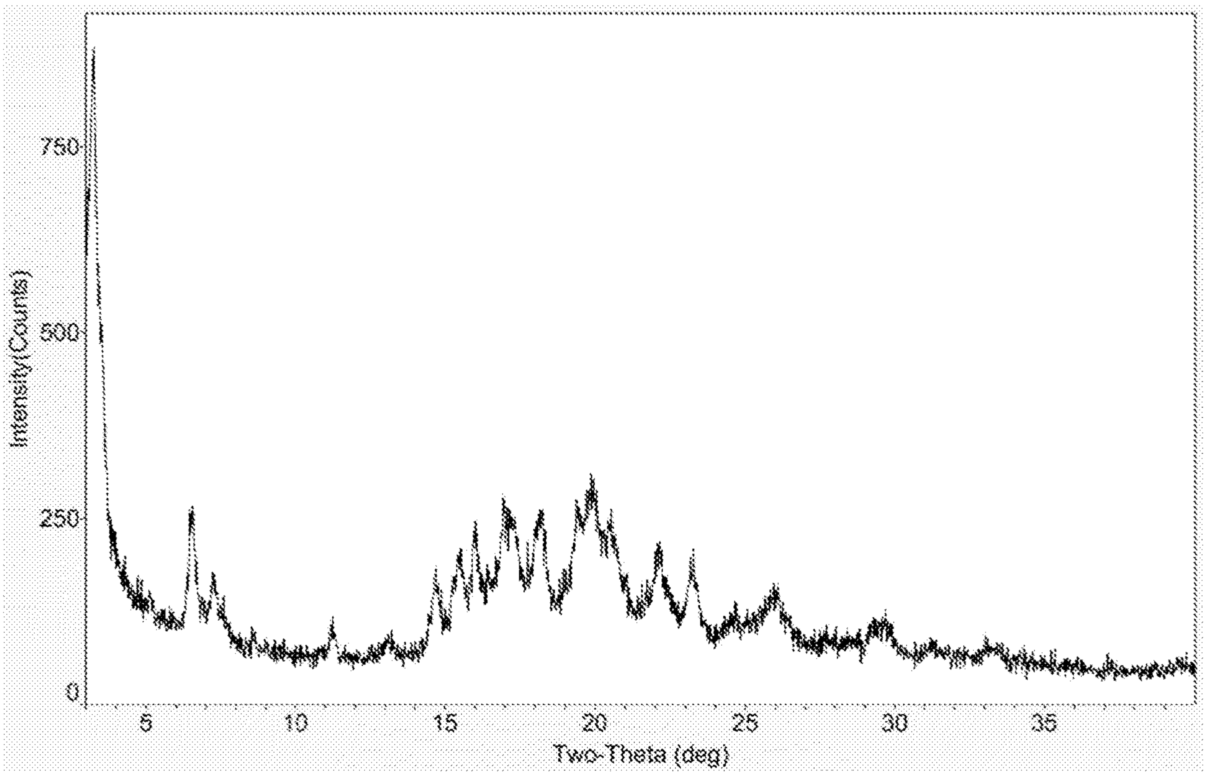


Fig. 38-1

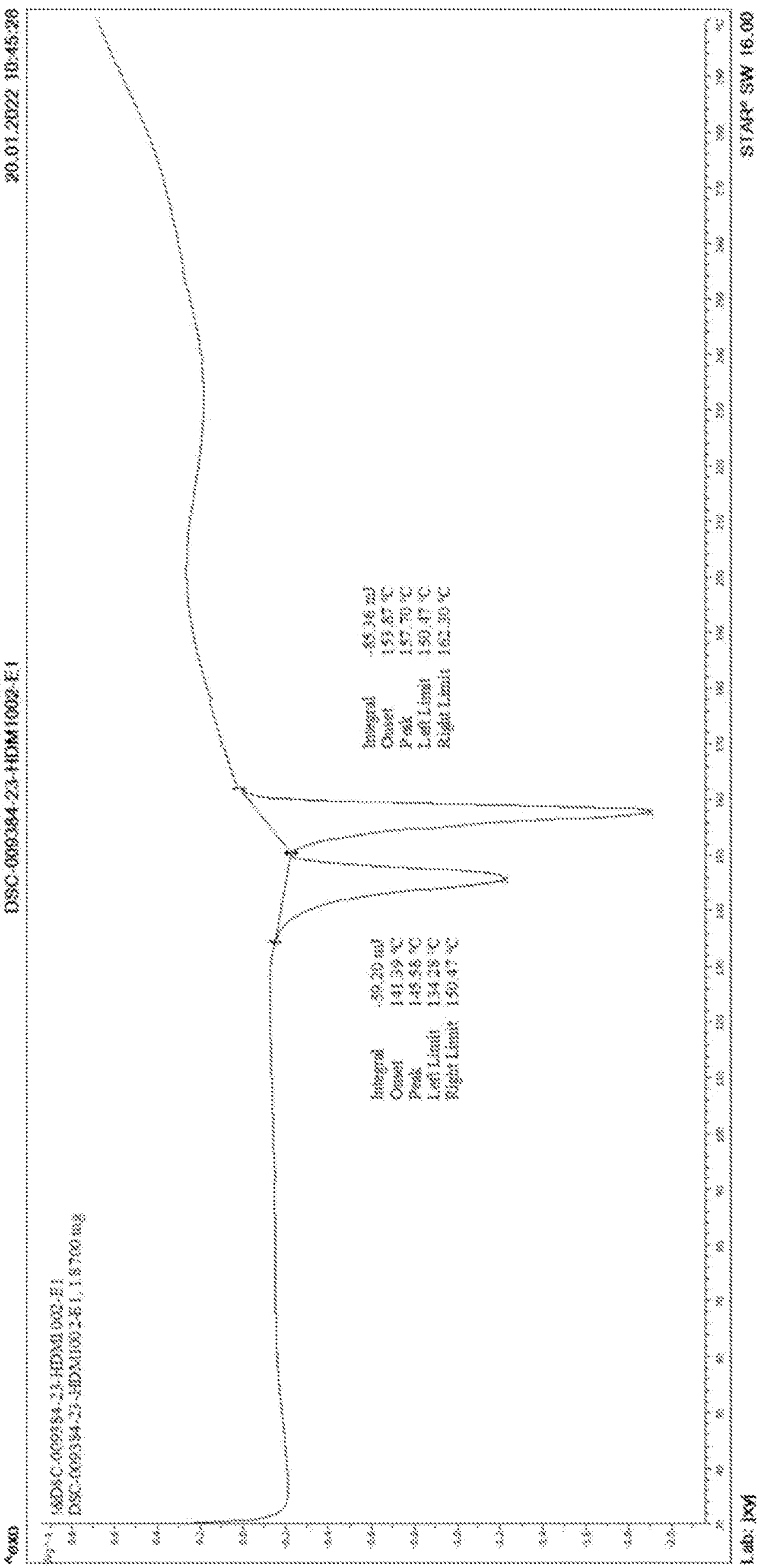


Fig. 38-2

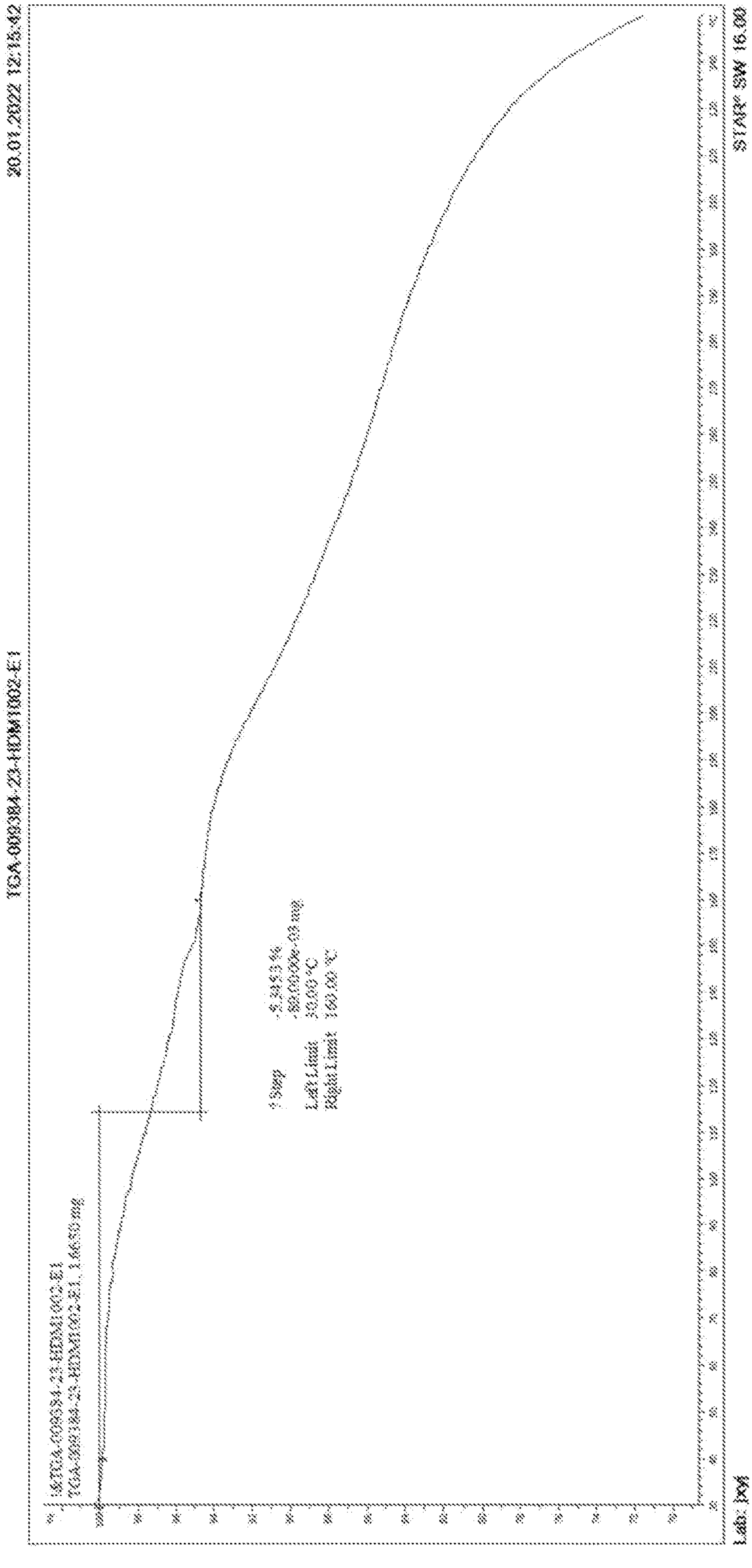


Fig. 38-3

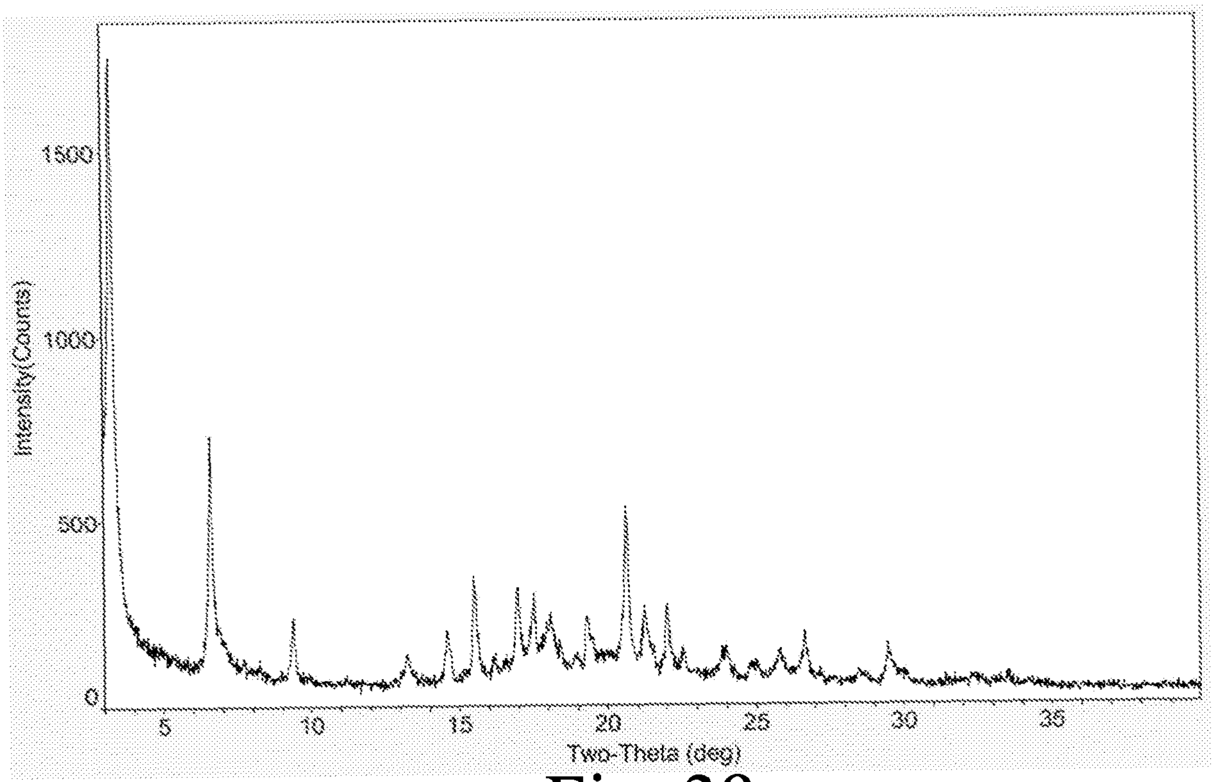
Intensity(Counts)
1500
1000
500
0
5
10
15
20
25
30
35
Two-Theta (deg)

Fig. 39

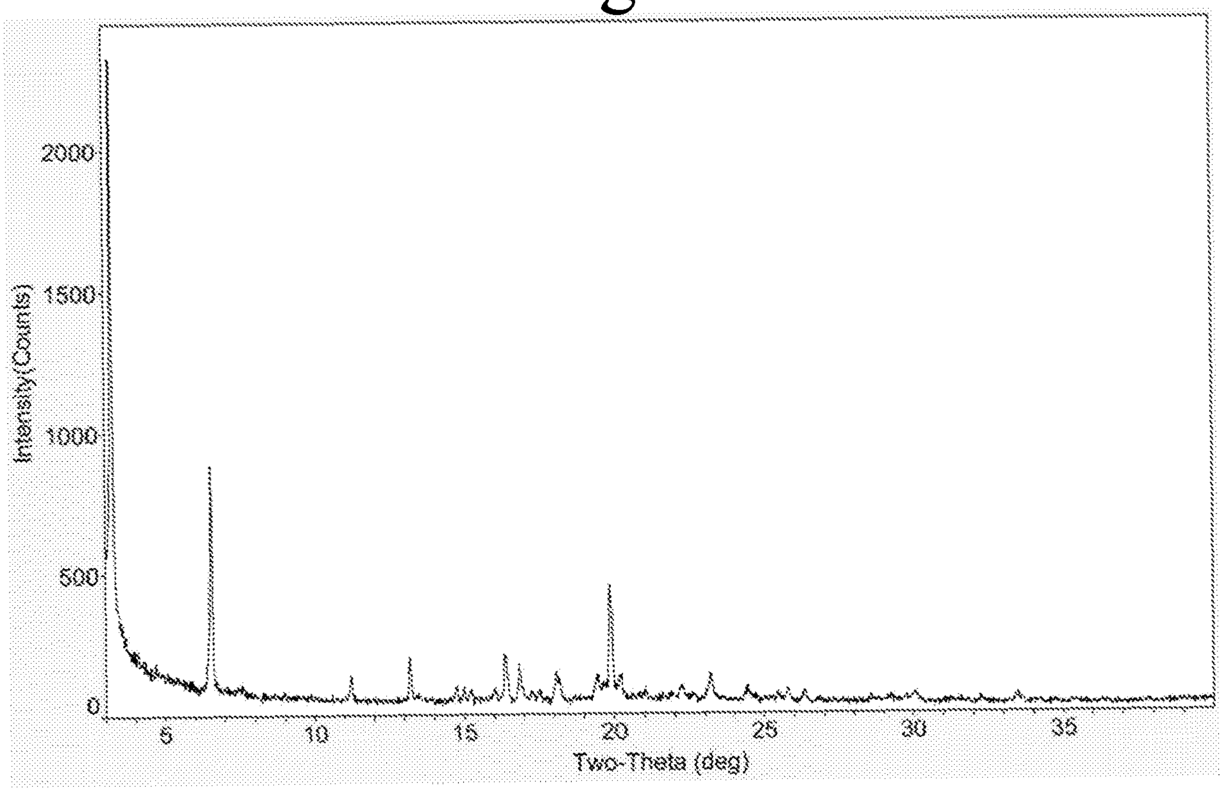
Intensity(Counts)
2000
1500
1000
500
0
5
10
15
20
25
30
35
Two-Theta (deg)

Fig. 40

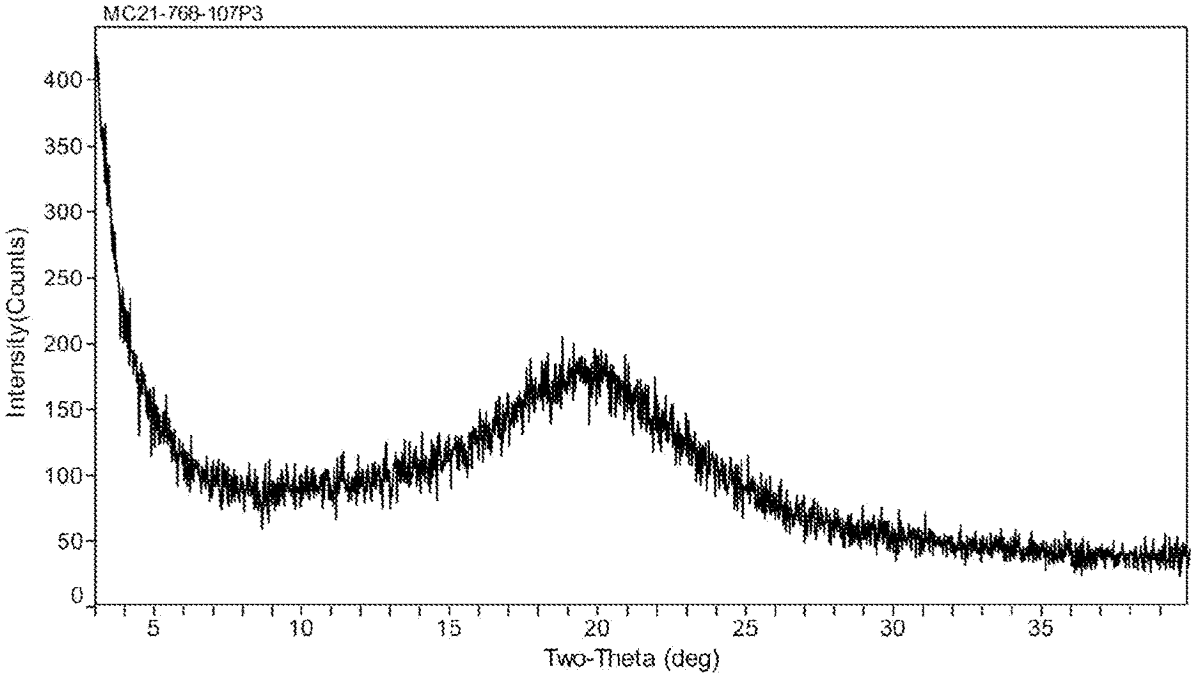
MC21-768-107P3
Intensity(Counts)
Two-Theta (deg)
0
50
100
150
200
250
300
350
400
5
10
15
20
25
30
35

Fig. 41

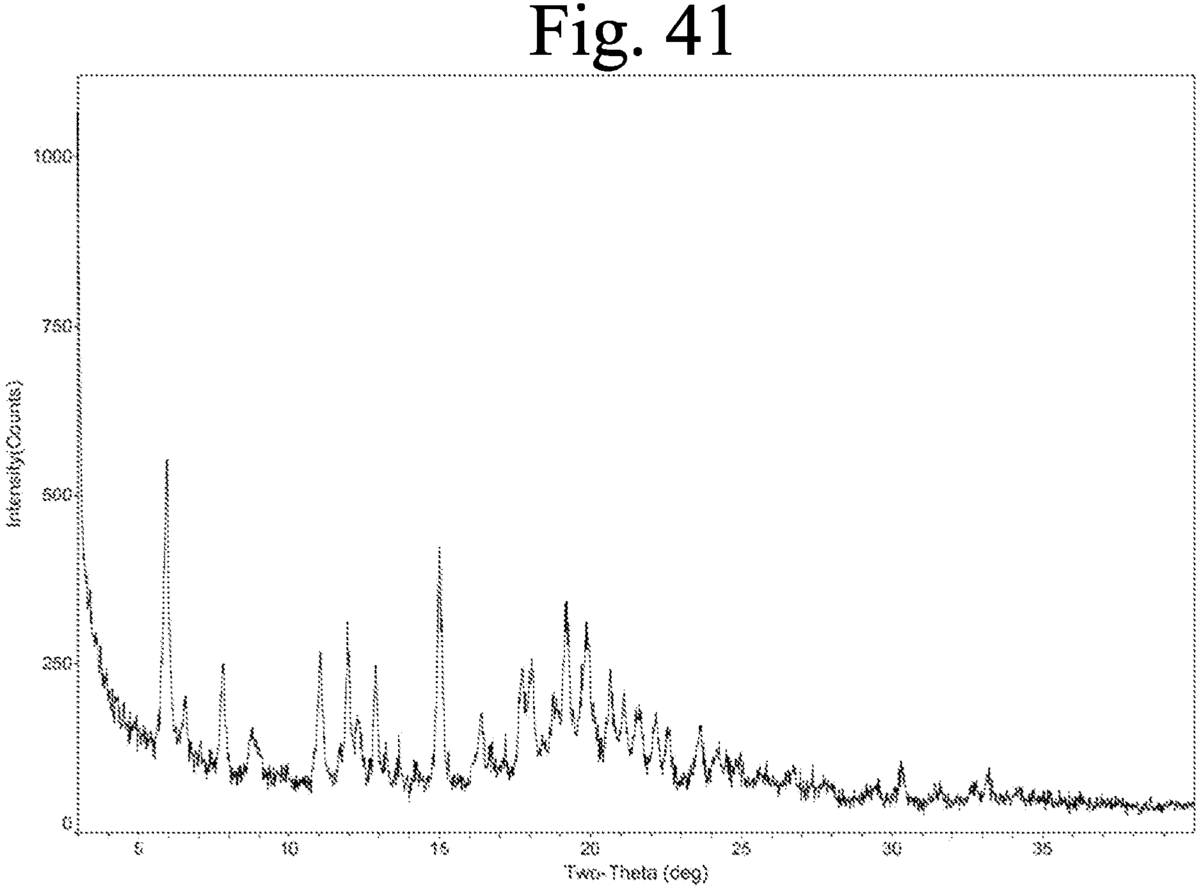
Intensity(Counts)
Two-Theta (deg)
0
250
500
750
1000
5
10
15
20
25
30
35

Fig. 42

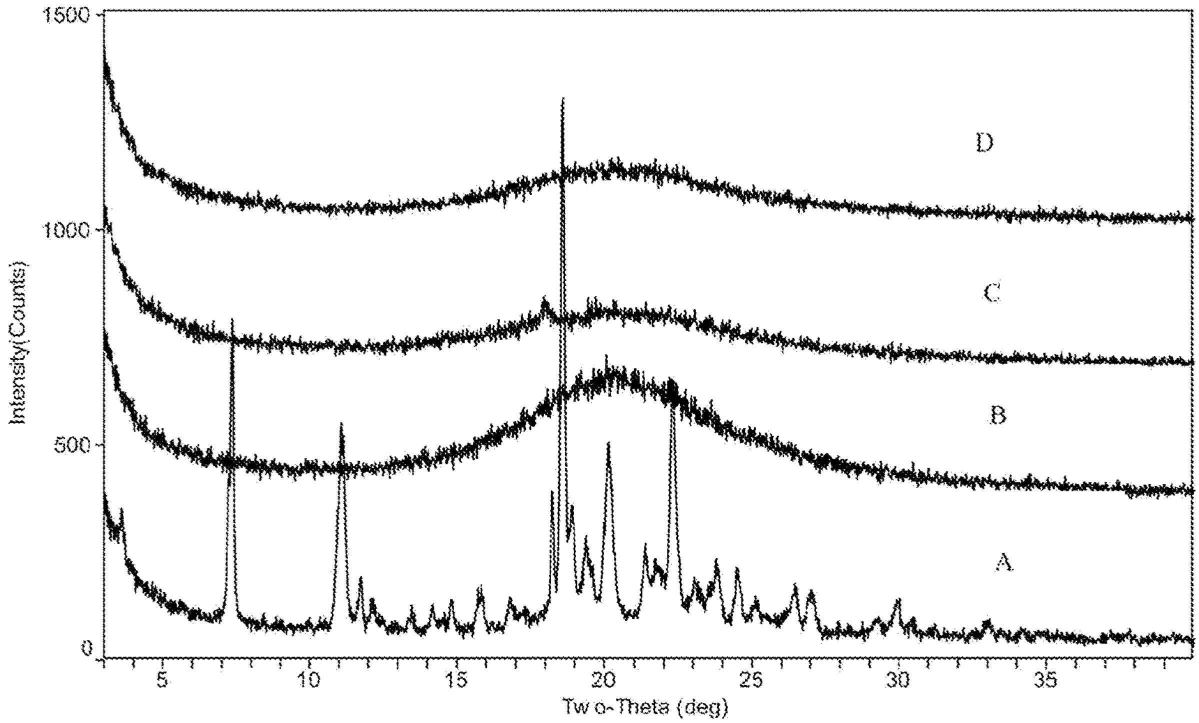
1500
1000
500
0
Intensity(Counts)
D
C
B
A
5
10
15
20
25
30
35
Tw o-Theta (deg)

Fig. 43

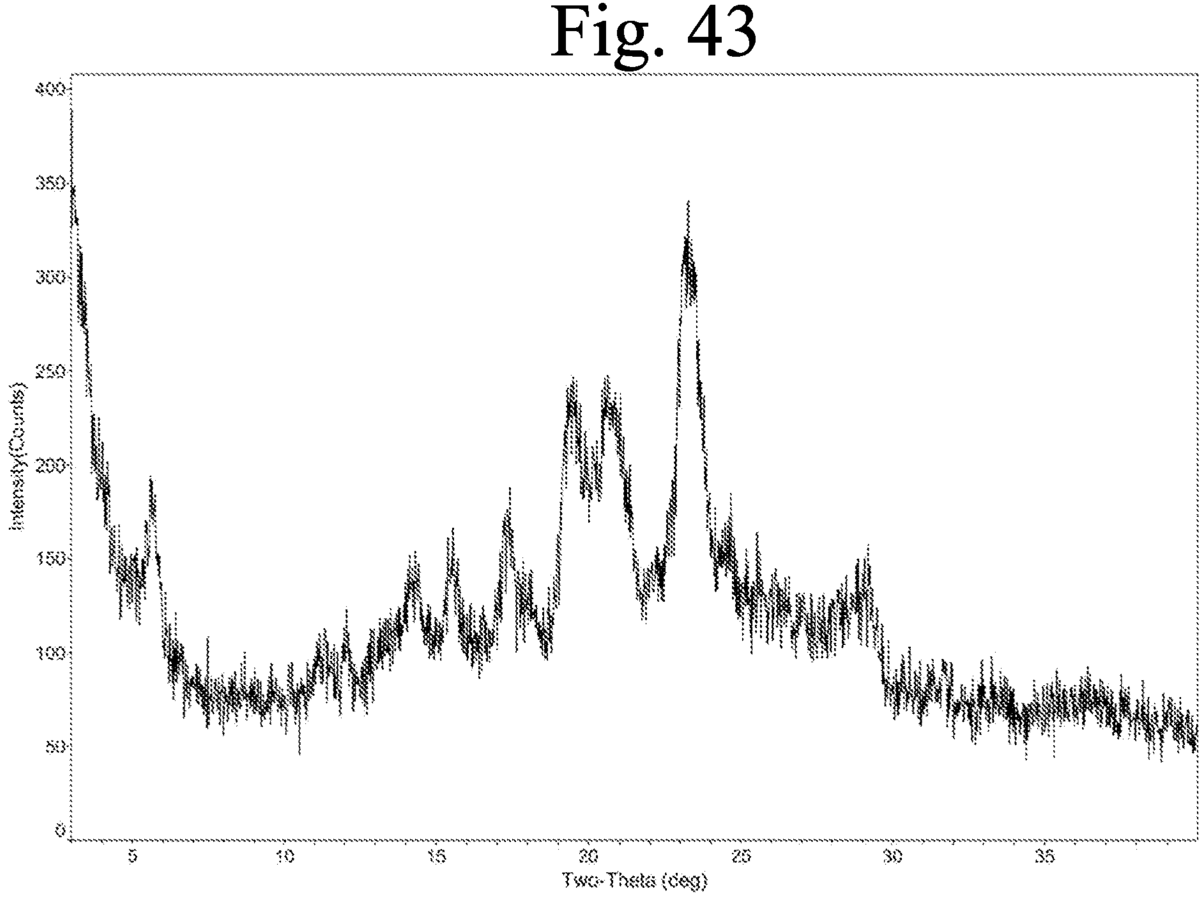
400
350
300
250
200
150
100
50
0
Intensity(Counts)
5
10
15
20
25
30
35
Two-Theta (deg)

Fig. 44

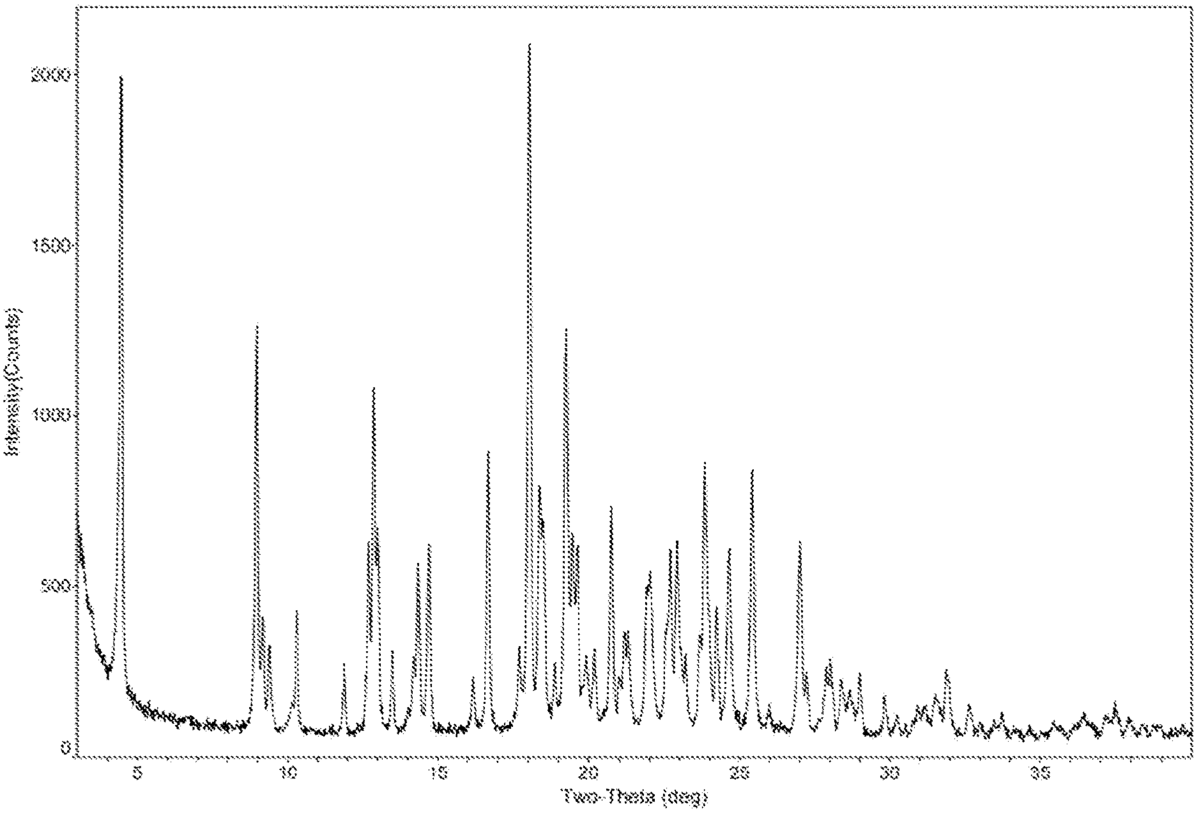
Intensity(Counts)
0
500
1000
1500
2000
5
10
15
20
25
30
35
Two-Theta (deg)

Fig. 45-1

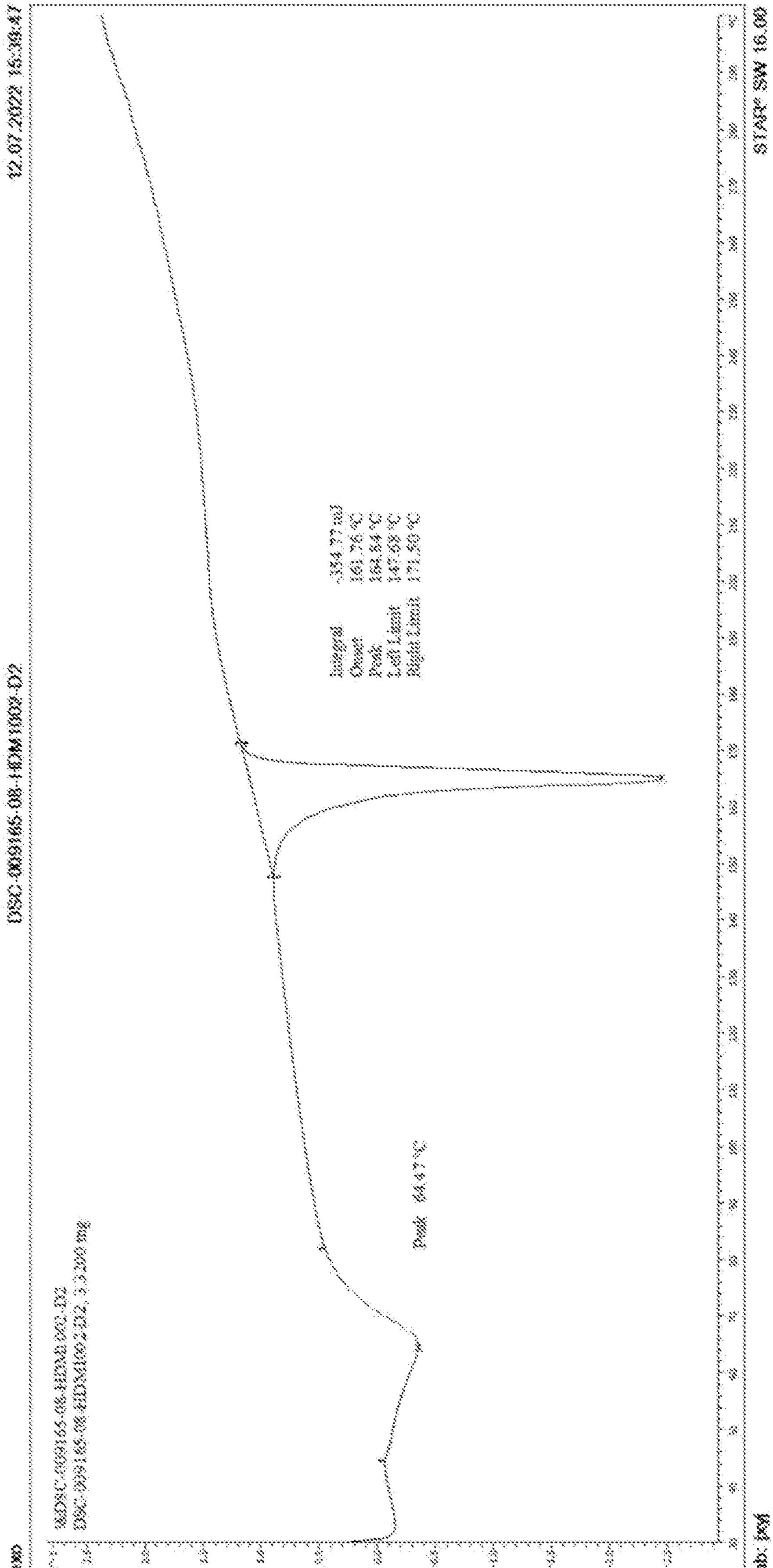


Fig. 45-2

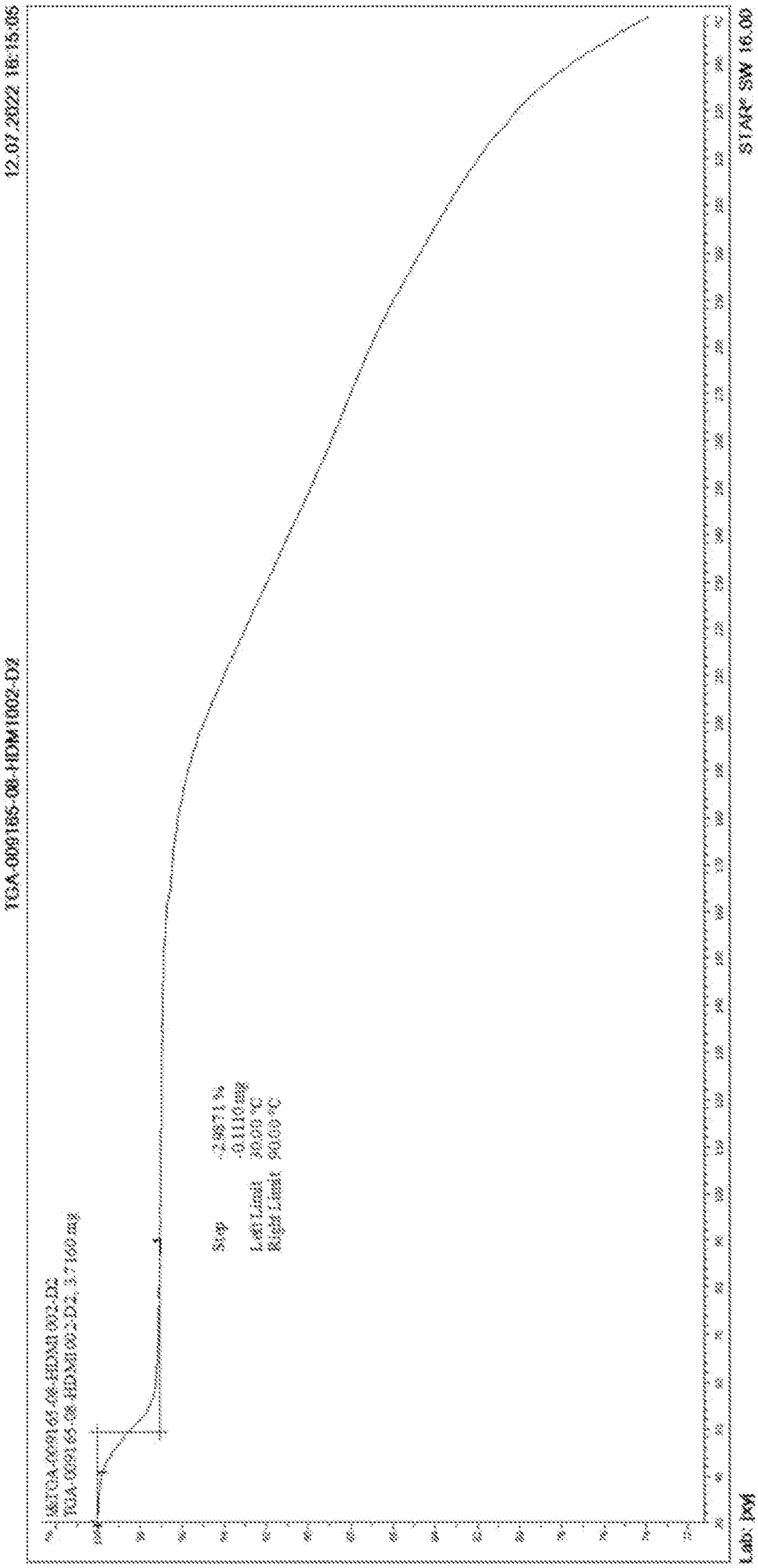

Fig. 45-3

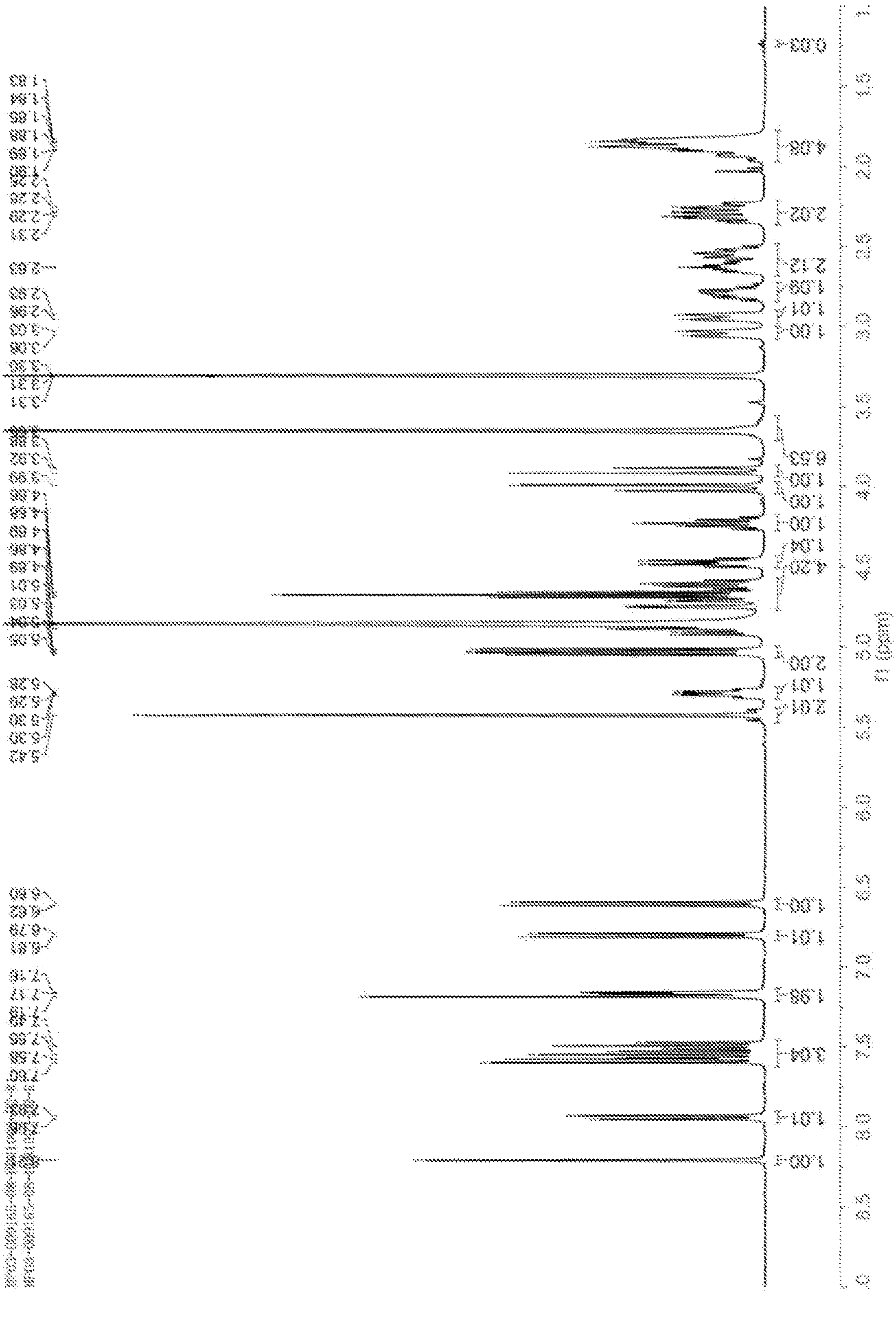

Fig. 45-4

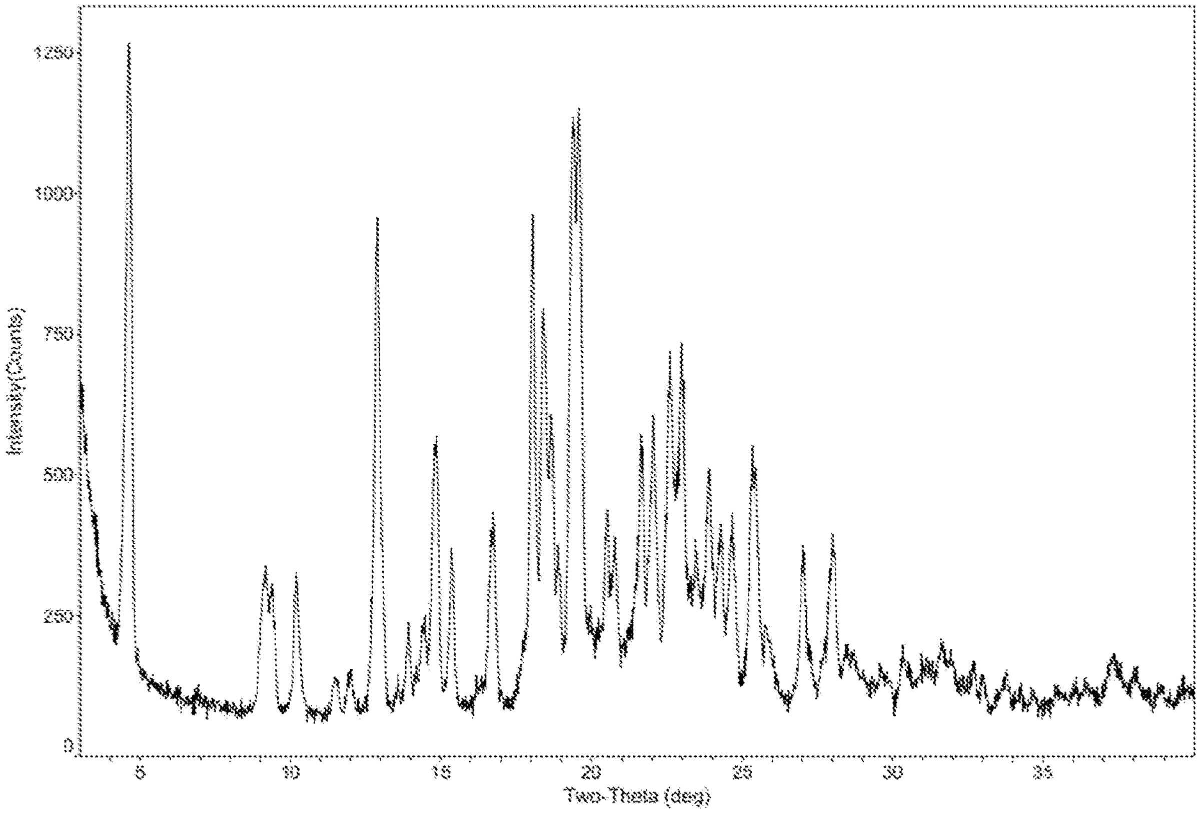
1250
1000
750
500
250
0
Intensity(Counts)
5
10
15
20
25
30
35
Two-Theta (deg)

Fig. 46

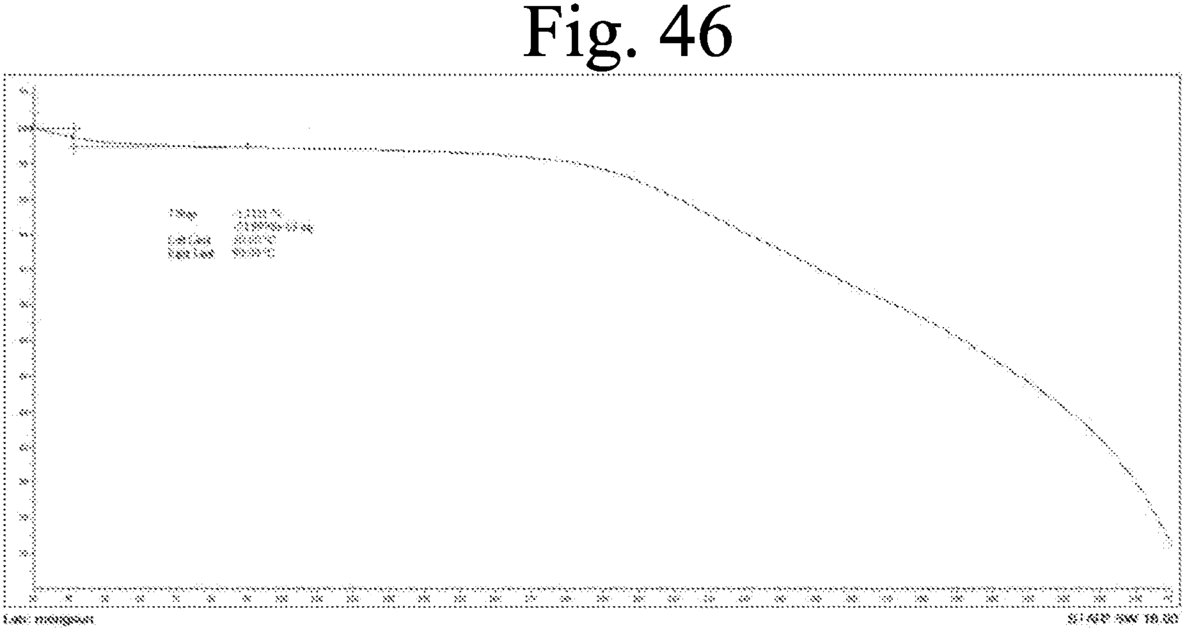

Fig. 47

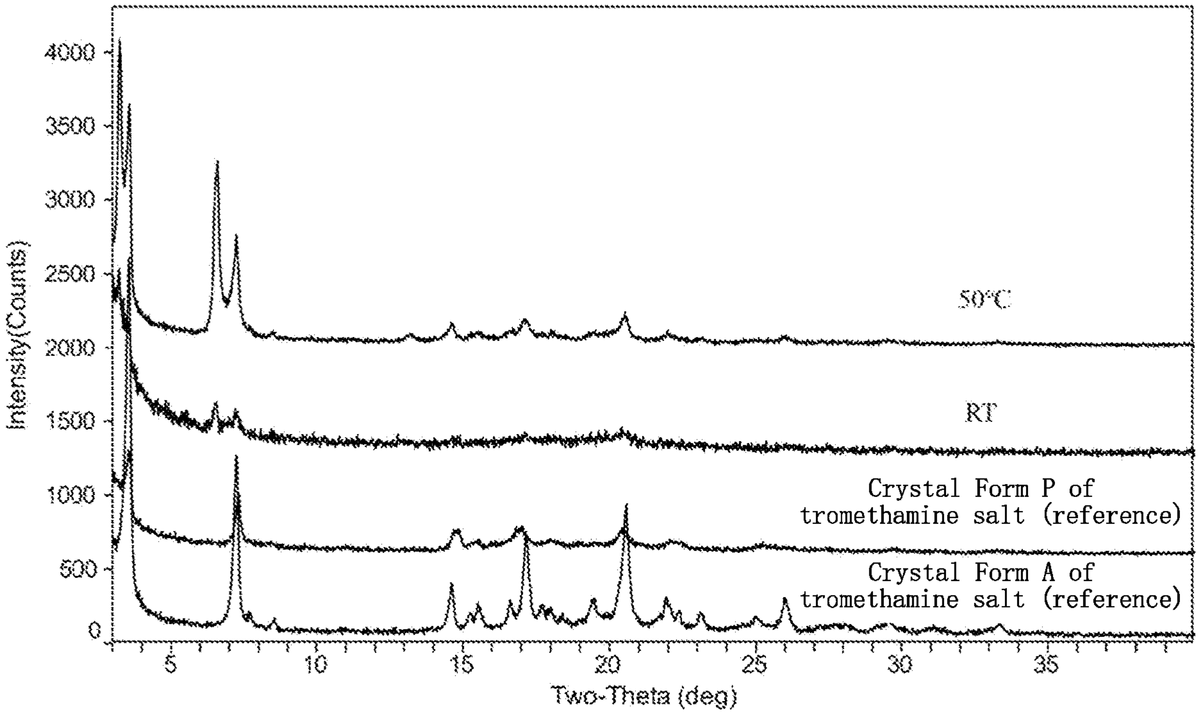
4000
3500
3000
2500
2000
1500
1000
500
0
Intensity(Counts)
50°C
RT
Crystal Form P of
tromethamine salt (reference)
Crystal Form A of
tromethamine salt (reference)
5
10
15
20
25
30
35
Two-Theta (deg)

Fig. 48

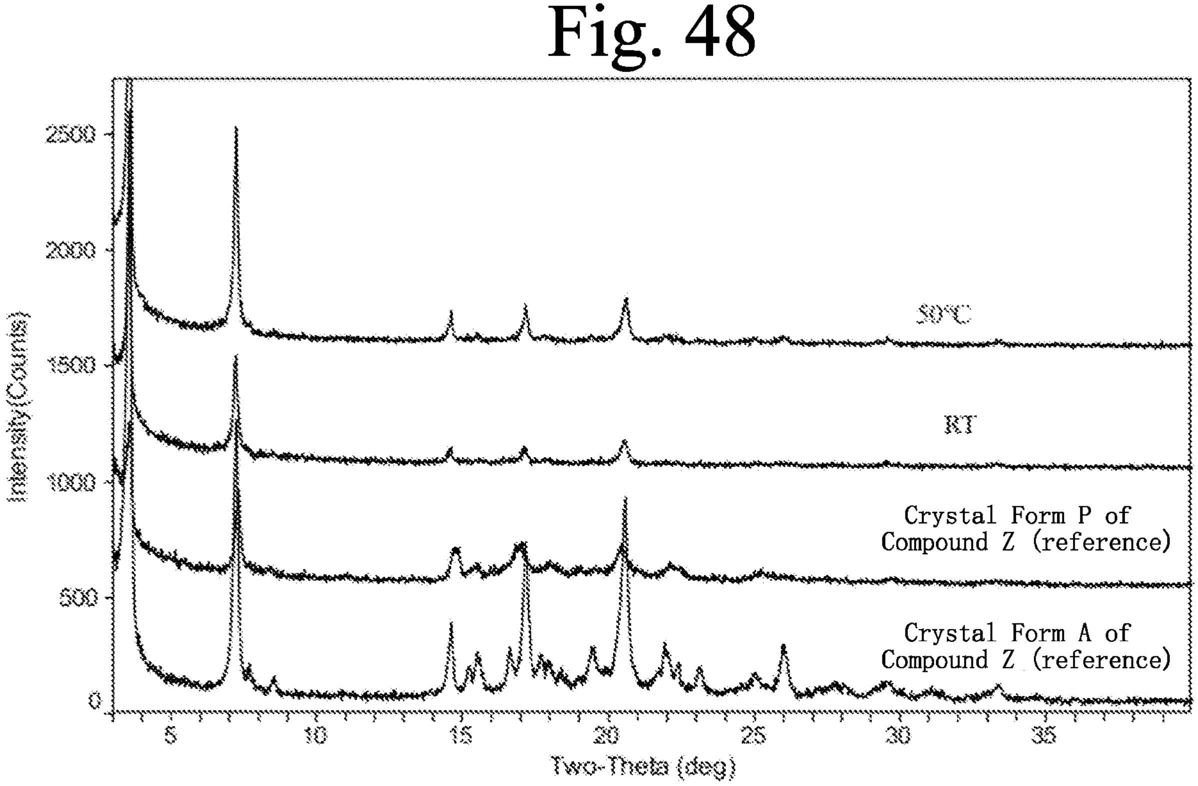
2500
2000
1500
1000
500
0
Intensity(Counts)
50°C
RT
Crystal Form P of
Compound Z (reference)
Crystal Form A of
Compound Z (reference)
5
10
15
20
25
30
35
Two-Theta (deg)

Fig. 49

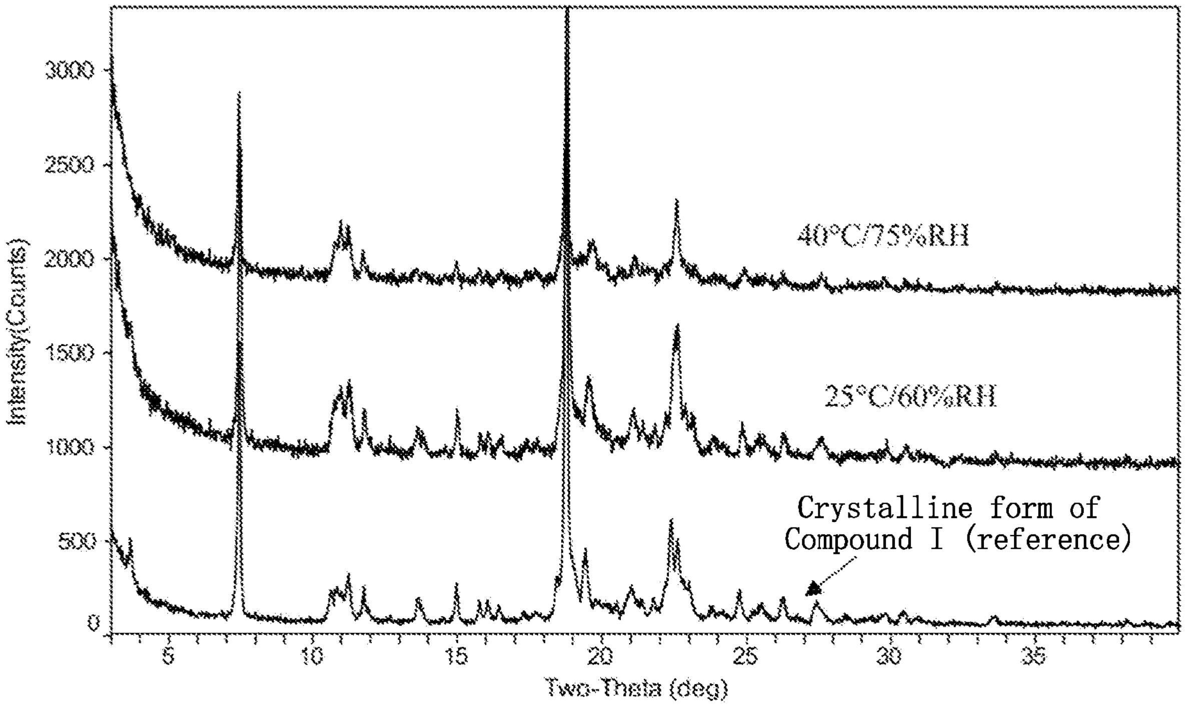
Intensity(Counts)
Two-Theta (deg)
40°C/75%RH
25°C/60%RH
Crystalline form of
Compound I (reference)

Fig. 50

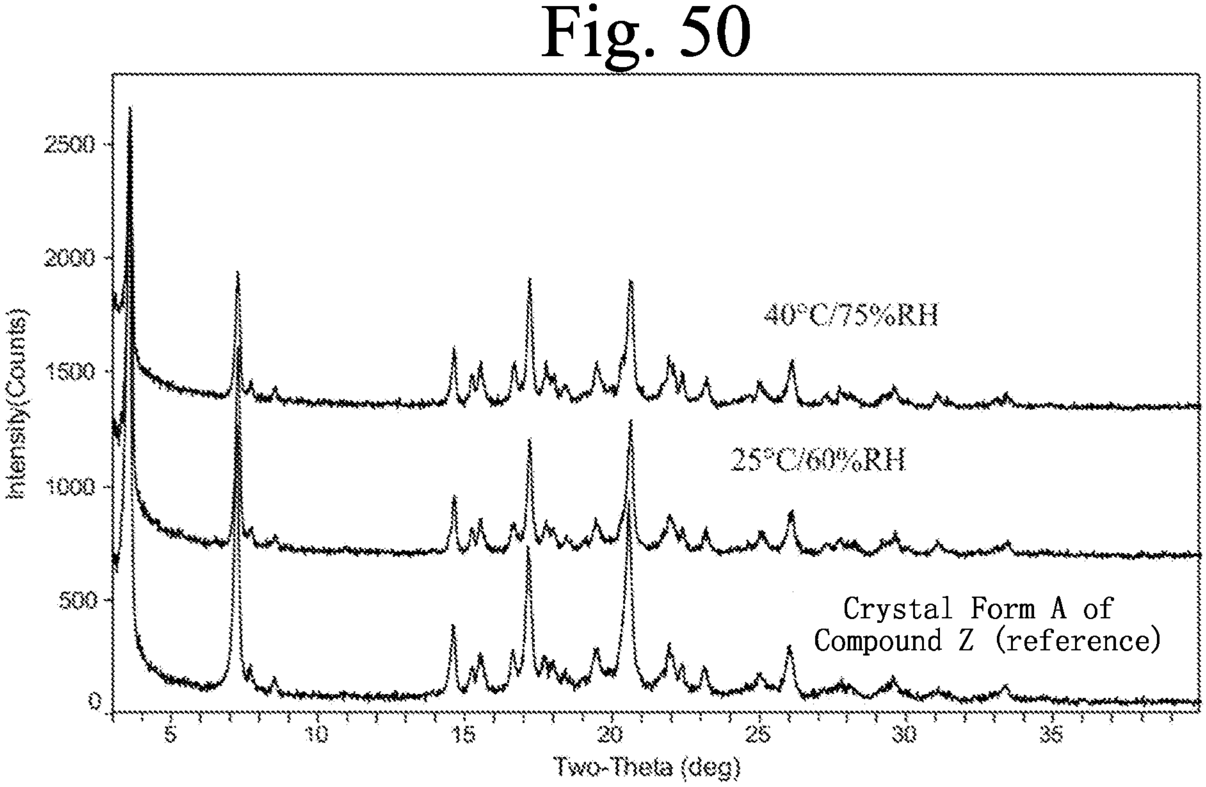
Intensity(Counts)
Two-Theta (deg)
40°C/75%RH
25°C/60%RH
Crystal Form A of
Compound Z (reference)

Fig. 51

## INTERNATIONAL SEARCH REPORT

International application No.
**PCT/CN2023/137851**

**A. CLASSIFICATION OF SUBJECT MATTER**

C07D405/14(2006.01)i； A61K31/4545(2006.01)i； A61P3/10(2006.01)i； A61P3/06(2006.01)i； A61P3/04(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

DWPI, WOTXT, EPTXT, CNTXT, CNKI, STN(REGISTRY, CAPLUS); 杭州中美华东, 胰高血糖素样肽, 受体, 激动剂, 胰岛素, 糖尿病, 苯并, 咪唑, 羧酸, 哌啶, 吡啶, GLP-1, glucagon-like peptide, receptor, agonist, insulin, diabet+, benzo, imidazol, carboxylic, piperidin, pyridin, STN中结构式检索, structural formula search in STN

**C. DOCUMENTS CONSIDERED TO BE RELEVANT**

| Category* | Citation of document, with indication, where appropriate, of the relevant passages | Relevant to claim No. |
|---|---|---|
| PX | WO 2022268152 A1 (HANGZHOU ZHONGMEI HUADONG PHARMACEUTICAL CO., LTD.) 29 December 2022 (2022-12-29)<br>claims 1-34, and description, page 21, paragraphs 3-4, and embodiment 1 | 1-2, 8-13 |
| Y | CN 110325530 A (PFIZER INC.) 11 October 2019 (2019-10-11)<br>claims 1-24, and description, pages 13, 16-17 and 77-78 | 1-2, 8-13 |
| A | CN 110325530 A (PFIZER INC.) 11 October 2019 (2019-10-11)<br>claims 1-24, and description, pages 13, 16-17 and 77-78 | 3-7 |
| Y | WO 2022109182 A1 (GILEAD SCIENCES, INC.) 27 May 2022 (2022-05-27)<br>claims 1-40, and description, pages 16 and 124-140, and table 2 | 1-2, 8-13 |
| A | WO 2022109182 A1 (GILEAD SCIENCES, INC.) 27 May 2022 (2022-05-27)<br>claims 1-40, and description, pages 16 and 124-140, and table 2 | 3-7 |
| A | CN 113227068 A (QILU REGOR THERAPEUTICS INC.) 06 August 2021 (2021-08-06)<br>claims 1-40, and description, pages 53-54, and table 4 | 1-13 |

☑ Further documents are listed in the continuation of Box C. ☑ See patent family annex.

* Special categories of cited documents:
- “A” document defining the general state of the art which is not considered to be of particular relevance
- “D” document cited by the applicant in the international application
- “E” earlier application or patent but published on or after the international filing date
- “L” document which may throw doubts on priority claim(s) or which is cited to establish the publication date of another citation or other special reason (as specified)
- “O” document referring to an oral disclosure, use, exhibition or other means
- “P” document published prior to the international filing date but later than the priority date claimed
- “T” later document published after the international filing date or priority date and not in conflict with the application but cited to understand the principle or theory underlying the invention
- “X” document of particular relevance; the claimed invention cannot be considered novel or cannot be considered to involve an inventive step when the document is taken alone
- “Y” document of particular relevance; the claimed invention cannot be considered to involve an inventive step when the document is combined with one or more other such documents, such combination being obvious to a person skilled in the art
- “&” document member of the same patent family

| Date of the actual completion of the international search | Date of mailing of the international search report |
|---|---|
| **26 February 2024** | **04 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
**PCT/CN2023/137851**

**C. DOCUMENTS CONSIDERED TO BE RELEVANT**

| Category* | Citation of document, with indication, where appropriate, of the relevant passages | Relevant to claim No. |
|---|---|---|
| A | WO 2022078152 A1 (HANGZHOU ZHONGMEI HUADONG PHARMACEUTICAL CO., LTD.) 21 April 2022 (2022-04-21) claims 1-12, and description, pages 17-27 | 1-13 |
| A | WO 2021018023 A1 (THE TITAN LEADING MED. TECH INC.) 04 February 2021 (2021-02-04) claims 1-15, and description, pages 16-51 | 1-13 |
| A | CN 114478497 A (HANGZHOU ZHONGMEI HUADONG PHARMACEUTICAL CO., LTD.) 13 May 2022 (2022-05-13) claims 1-11, and description, pages 11-12 | 1-13 |
| A | CN 112566637 A (PFIZER INC.) 26 March 2021 (2021-03-26) claims 1-20, and description, pages 18-19 and 69-71 | 1-13 |

Form PCT/ISA/210 (second sheet) (July 2022)

**INTERNATIONAL SEARCH REPORT**

International application No.
**PCT/CN2023/137851**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12-13**
because they relate to subject matter not required to be searched by this Authority, namely:

The subject matter of claims 12 and 13 (when referring to claim 12) is a method for treatment of a human disease (PCT Rule 39.1(iv)). The search is carried out on the basis of the use of the compound in the preparation of a drug for treating a corresponding disease.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/137851**

| Patent document cited in search report | Publication date (day/month/year) | Patent family member(s) | Publication date (day/month/year) |
|---|---|---|---|
| WO 2022268152 A1 | 29 December 2022 | AU 2022300055 A1 | 18 January 2024 |
| | | TW 202317553 A | 01 May 2023 |
| | | CA 3219984 A1 | 29 December 2022 |
| CN 110325530 A | 11 October 2019 | ECSP 19041071 A | 30 June 2019 |
| | | UA 122035 C2 | 25 August 2020 |
| | | US 2019119255 A1 | 25 April 2019 |
| | | US 10669259 B2 | 02 June 2020 |
| | | UY 37514 A | 31 July 2018 |
| | | MX 2019007077 A | 01 August 2019 |
| | | IL 267288 A | 29 August 2019 |
| | | IL 267288 B | 30 June 2021 |
| | | PH 12019501360 A1 | 10 February 2020 |
| | | AR 110387 A1 | 27 March 2019 |
| | | LT 3555064 T | 10 January 2023 |
| | | TW 201835066 A | 01 October 2018 |
| | | TWI 713809 B | 21 December 2020 |
| | | CL 2019001651 A1 | 04 October 2019 |
| | | US 2020255406 A1 | 13 August 2020 |
| | | US 10851081 B2 | 01 December 2020 |
| | | MA 56480 A | 27 April 2022 |
| | | MA 56480 B1 | 30 December 2022 |
| | | MY 195385 A | 18 January 2023 |
| | | US 2018170908 A1 | 21 June 2018 |
| | | US 10208019 B2 | 19 February 2019 |
| | | AU 2017374860 A1 | 20 June 2019 |
| | | AU 2017374860 B2 | 02 September 2021 |
| | | EP 3555064 A1 | 23 October 2019 |
| | | EP 3555064 B1 | 23 November 2022 |
| | | EP 3555064 B9 | 01 March 2023 |
| | | KR 20190094433 A | 13 August 2019 |
| | | KR 102314286 B1 | 21 October 2021 |
| | | JP 6637641 B1 | 29 January 2020 |
| | | JP 2020511411 A | 16 April 2020 |
| | | FI 3555064 T3 | 31 January 2023 |
| | | JP 2020063287 A | 23 April 2020 |
| | | JP 6982054 B2 | 17 December 2021 |
| | | US 2021047298 A1 | 18 February 2021 |
| | | US 11512070 B2 | 29 November 2022 |
| | | CO 2019006046 A2 | 19 June 2019 |
| | | PE 20191501 A1 | 22 October 2019 |
| | | HUE 060533 T2 | 28 March 2023 |
| | | ZA 201904616 B | 26 May 2021 |
| | | BR 112019012211 A2 | 12 November 2019 |
| | | ES 2934789 T3 | 27 February 2023 |
| | | ES 2934789 T9 | 26 April 2023 |
| | | KR 20210127782 A | 22 October 2021 |
| | | KR 102466418 B1 | 14 November 2022 |
| | | US 2023124938 A1 | 20 April 2023 |
| | | US 11802121 B2 | 31 October 2023 |
| | | DOP 2019000166 A | 31 July 2019 |

Form PCT/ISA/210 (patent family annex) (July 2022)

**INTERNATIONAL SEARCH REPORT**
**Information on patent family members**

International application No.
**PCT/CN2023/137851**

| Patent document cited in search report | | | Publication date (day/month/year) | Patent family member(s) | | | Publication date (day/month/year) |
|---|---|---|---|---|---|---|---|
| | | | | MD | 3555064 | T2 | 31 August 2023 |
| | | | | RU | 2740135 | C1 | 11 January 2021 |
| | | | | PT | 3555064 | T | 20 January 2023 |
| | | | | CR | 20190289 | A | 21 August 2019 |
| | | | | CA | 2988721 | A1 | 16 June 2018 |
| | | | | CA | 2988721 | C | 05 September 2023 |
| | | | | RS | 63849 | B1 | 31 January 2023 |
| | | | | RS | 63849 | B9 | 30 June 2023 |
| | | | | WO | 2018109607 | A1 | 21 June 2018 |
| | | | | PL | 3555064 | T3 | 06 March 2023 |
| | | | | EA | 201991193 | A1 | 15 January 2020 |
| | | | | EA | 037318 | B1 | 11 March 2021 |
| | | | | DK | 3555064 | T3 | 12 December 2022 |
| | | | | DK | 3555064 | T5 | 01 May 2023 |
| | | | | SI | 3555064 | T1 | 28 February 2023 |
| | | | | CU | 20190056 | A7 | 03 January 2020 |
| | | | | CU | 24573 | B1 | 13 January 2022 |
| | | | | HRP | 20221437 | T1 | 03 February 2023 |
| | | | | GEP | 20217265 | B | 25 June 2021 |
| WO | 2022109182 | A1 | 27 May 2022 | TW | 202237582 | A | 01 October 2022 |
| | | | | US | 2022177449 | A1 | 09 June 2022 |
| | | | | US | 11851419 | B2 | 26 December 2023 |
| | | | | WO | 2022109182 | A9 | 08 December 2022 |
| | | | | EP | 4247804 | A1 | 27 September 2023 |
| CN | 113227068 | A | 06 August 2021 | SG | 11202105241 | YA | 29 June 2021 |
| | | | | CU | 20210042 | A7 | 13 January 2022 |
| | | | | TW | 202039458 | A | 01 November 2020 |
| | | | | AU | 2019382642 | A1 | 10 June 2021 |
| | | | | BR | 112021009877 | A2 | 17 August 2021 |
| | | | | JP | 2022508203 | A | 19 January 2022 |
| | | | | WO | 2020103815 | A1 | 28 May 2020 |
| | | | | CR | 20210341 | A | 25 November 2021 |
| | | | | MX | 2021006034 | A | 21 September 2021 |
| | | | | US | 2022024901 | A1 | 27 January 2022 |
| | | | | KR | 20210106447 | A | 30 August 2021 |
| | | | | US | 2020283424 | A1 | 10 September 2020 |
| | | | | US | 10844049 | B2 | 24 November 2020 |
| | | | | PH | 12021551165 | A1 | 25 October 2021 |
| | | | | MA | 54555 | A | 29 September 2021 |
| | | | | IL | 283322 | A | 29 July 2021 |
| | | | | EP | 3883928 | A1 | 29 September 2021 |
| | | | | EP | 3883928 | A4 | 29 June 2022 |
| | | | | CA | 3120499 | A1 | 28 May 2020 |
| | | | | PE | 20211871 | A1 | 21 September 2021 |
| | | | | EA | 202191432 | A1 | 14 October 2021 |
| | | | | CO | 2021008089 | A2 | 09 September 2021 |
| | | | | CL | 2021001332 | A1 | 05 November 2021 |
| WO | 2022078152 | A1 | 21 April 2022 | EP | 4227299 | A1 | 16 August 2023 |
| WO | 2021018023 | A1 | 04 February 2021 | | None | | |

Form PCT/ISA/210 (patent family annex) (July 2022)

**INTERNATIONAL SEARCH REPORT**
**Information on patent family members**

International application No.
**PCT/CN2023/137851**

| Patent document cited in search report | Publication date (day/month/year) | Patent family member(s) | Publication date (day/month/year) |
|---|---|---|---|
| CN 114478497 A | 13 May 2022 | None | |
| CN 112566637 A | 26 March 2021 | FI 3806855 T3 | 04 May 2023 |
| | | US 2021332034 A1 | 28 October 2021 |
| | | US 11858916 B2 | 02 January 2024 |
| | | HUE 061888 T2 | 28 August 2023 |
| | | JP 2021528394 A | 21 October 2021 |
| | | JP 7053900 B2 | 12 April 2022 |
| | | IL 279224 A | 31 January 2021 |
| | | IL 279224 B1 | 01 March 2023 |
| | | IL 279224 B2 | 01 July 2023 |
| | | ES 2943510 T3 | 13 June 2023 |
| | | RU 2765721 C1 | 02 February 2022 |
| | | EP 3806855 A1 | 21 April 2021 |
| | | EP 3806855 B1 | 01 March 2023 |
| | | BR 112020024956 A2 | 09 March 2021 |
| | | CA 3103051 A1 | 19 December 2019 |
| | | CA 3103051 C | 17 January 2023 |
| | | WO 2019239371 A1 | 19 December 2019 |
| | | AU 2019285570 A1 | 17 December 2020 |
| | | AU 2019285570 B2 | 24 March 2022 |
| | | ZA 202007304 B | 29 November 2023 |
| | | SG 11202011704 XA | 30 December 2020 |
| | | KR 20210020095 A | 23 February 2021 |
| | | KR 102538572 B1 | 01 June 2023 |
| | | MX 2020013624 A | 11 August 2022 |
| | | DK 3806855 T3 | 24 April 2023 |
| | | DK 3806855 T5 | 22 May 2023 |
| | | PL 3806855 T3 | 24 July 2023 |
| | | SI 3806855 T1 | 30 June 2023 |
| | | PT 3806855 T | 03 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Remington's Pharmaceutical Sciences. 1990